# EUROPEAN PATENT APPLICATION

(11) **EP 4 737 592 A1**
(43) Date of publication of application: **06.05.2026**
(21) Application number: 25212759.2
(22) Date of filing: 31.10.2025
(51) Int. Cl.: C12Q 1/6886

(54) **METHOD FOR PREDICTING A RESPONSE TO SYSTEMIC TREATMENT IN A HORMONE RECEPTOR-POSITIVE (HR+) AND HUMAN EPIDERMAL GROWTH FACTOR RECEPTOR 2-NEGATIVE (HER2-) BREAST CANCER PATIENT**

(30) Priority: 31.10.2024 EP 24210258
(71) Applicant: GBG Forschungs GmbH, 63263 Neu-Isenburg (DE); Johann-Wolfgang-Goethe-Universität Frankfurt am Main, 60323 Frankfurt am Main (DE); PHILIPPS-UNIVERSITÄT MARBURG, 35037 Marburg (DE)
(72) Inventor: LOIBL, Sibylle, 63263 Neu-Isenburg (DE); RACHAKONDA, Panduranga Sivaramakrishna, 63263 Neu-Isenburg (DE); WEBER, Karsten, 63263 Neu-Isenburg (DE); DENKERT, Carsten, 35043 Marburg (DE); KARN, Thomas, 60590 Frankfurt am Main (DE)
(74) Representative: Vossius & Partner Patentanwälte Rechtsanwälte mbB

(57) **Abstract**

The present invention is in the field of molecular subtyping of tumor samples and therapy guidance. The present invention relates to methods, arrays, prognostic assays, systems and uses thereof for prediction of the response or resistance to and/or benefit from a systemic treatment, in particular adjuvant or neoadjuvant chemotherapy, of a subject suffering from a HR+ and HER2- breast cancer, based on the measurement(s) of expression level(s) of three or more markers in tumor samples of said subject. Equally, the present invention relates to methods, arrays, prognostic assays, systems and uses thereof for prediction of the outcome benefit from a systemic treatment, in particular adjuvant or neoadjuvant chemotherapy, of a subject suffering from a HR+ and HER2- breast cancer, based on the measurement(s) of expression level(s) of three or more markers in tumor samples of said subject.

## Description

### FIELD OF THE INVENTION

The present invention is in the field of molecular subtyping of tumor samples and therapy guidance. The present invention relates to methods, arrays, prognostic assays, systems and uses thereof for prediction of the response or resistance to and/or benefit from a systemic treatment, in particular adjuvant or neoadjuvant chemotherapy, of a subject suffering from a HR+ and HER2- breast cancer, based on the measurement(s) of expression level(s) of three or more markers in tumor samples of said subject. Equally, the present invention relates to methods, arrays, prognostic assays, systems and uses thereof for prediction of the outcome benefit from a systemic treatment, in particular adjuvant or neoadjuvant chemotherapy, of a subject suffering from a HR+ and HER2- breast cancer, based on the measurement(s) of expression level(s) of three or more markers in tumor samples of said subject.

### BACKGROUND OF THE INVENTION

Molecular subtyping of tumor biopsies (Perou CM. et al. Molecular portraits of human breast tumours. Nature. 2000 Aug 17;406(6797):747-52; Sørlie T. et al. Gene expression patterns of breast carcinomas distinguish tumor subclasses with clinical implications. Proc Natl Acad Sci U S A. 2001 Sep 11;98(19):10869-74) revealed that breast cancer constitutes a heterogeneous set of diseases with different biological features, requiring tailored therapeutic strategies. Breast cancer cells express receptors, e.g., on the surface, in the cytoplasm and the nucleus. Breast cancers can be classified according to their expression of three important receptors: estrogen receptor (ER), progesterone receptor (PR), and HER2. ER and PR are hormone receptors (HR). ER+ cancer cells (i.e., cancer cells expressing estrogen receptors) depend on estrogen for their growth, allowing for treatment with drugs inhibiting estrogen effects (e.g., tamoxifen), and ER+ cancers generally have a better prognosis. Untreated, HER2+ breast cancers tend to be more aggressive than HER2- breast cancers, however treatment of HER2+ cancer cells with certain drugs, such as the monoclonal antibody trastuzumab (in combination with conventional chemotherapy), improves the prognosis significantly. Cells that lack expression of estrogen receptors, progesterone receptors and HER2 are called triple-negative (TN), although they frequently do express receptors for other hormones, such as androgen receptor and prolactin receptor.

The definition of luminal A (LumA), luminal B (LumB), basal-like (BasalL), HER2-enriched (HER2E), and normal-like (NormL) tumors is based on gene expression profiling, including the PAM50 approach (WO2009158143A1) as well as absolute assignment of breast cancer intrinsic molecular subtype (AIMS)-based subtyping (Paquet ER, Hallett MT. Absolute assignment of breast cancer intrinsic molecular subtype. J Natl Cancer Inst. 2014 Dec 4;107(1):357). However, the subtypes identified using the PAM50- and the AIMS-based approach are not adapted to therapy-induced molecular plasticity.

In addition, other molecular assays as well as proliferation markers such as Antigen Kiel 67(Ki-67) are used. Adaptive changes of Ki-67 can be used to decide whether patients should receive endocrine therapy or chemotherapy followed by endocrine therapy (Smith I. et al. Long-term outcome and prognostic value of Ki67 after perioperative endocrine therapy in postmenopausal women with hormone-sensitive early breast cancer (POETIC): an open-label, multicentre, parallel-group, randomised, phase 3 trial. Lancet Oncol. 2020 Nov;21(11):1443-1454; Nitz UA. et al. Endocrine Therapy Response and 21-Gene Expression Assay for Therapy Guidance in HR+/HER2- Early Breast Cancer. J Clin Oncol. 2022 Aug 10;40(23):2557-2567). Due to the limited availability of paired samples, little is known about the changes of molecular subtypes during and after therapy. Therefore, an improved understanding of molecular adaptation to neoadjuvant chemotherapy could generate new advanced options for classification of tumors and tailored therapeutic strategies.

Luminal tumors consist of two different subtypes: LumA tumors have a low proliferation rate and are typically treated with endocrine therapy alone. In contrast, LumB tumors are characterized by increased proliferation and tumor aggressiveness and therapeutic strategies for these tumors often include chemotherapy, followed by endocrine therapy.

BasalL tumors tend to have a high metastasis rate and are often triple-negative. Of the AIMS subtypes, BasaL breast cancers show the highest pathological complete response (pCR) rate to neoadjuvant chemotherapy. HER2E breast cancers are usually responsive to (neo)adjuvant trastuzumab in combination with chemotherapy and are sensitive to adjuvant anthracyclines and taxanes. NormL breast cancers are often enriched for ER (Hoadley et al. Breast cancer intrinsic subtypes. Nat Rev Clin Oncol. Poster. 2014 Dec).

Genomic signatures have been developed to predict the response of breast cancer to neoadjuvant chemotherapy (NACT), with a strong contribution of immune gene expression (Denkert C et al. Tumor-infiltrating lymphocytes and response to neoadjuvant chemotherapy with or without carboplatin in human epidermal growth factor receptor 2-positive and triple-negative primary breast cancers. J Clin Oncol. 2015 Mar 20;33(9):983-91). For luminal breast cancer, clinical strategies are based on prognostic assessment of tumor samples before therapy (Cardoso F. et al. 70-Gene Signature as an Aid to Treatment Decisions in Early-Stage Breast Cancer. N Engl J Med. 2016 Aug 25;375(8):717-29; Sparano JA. et al. Adjuvant Chemotherapy Guided by a 21-Gene Expression Assay in Breast Cancer. N Engl J Med. 2018 Jul 12;379(2):111-121; Filipits M. et al. A new molecular predictor of distant recurrence in ER-positive, HER2-negative breast cancer adds independent information to conventional clinical risk factors. Clin Cancer Res. 2011 Sep 15;17(18):6012-20) to identify those patients who can be treated with endocrine therapy alone, without additional chemotherapy. These approaches have led to considerable advances in the clinical management of breast cancer with therapeutic algorithms in place for the different subtypes. However, molecular changes in the tumor after neoadjuvant therapy as well as tumor heterogeneity (Parker JS, Perou CM. Tumor Heterogeneity: Focus on the Leaves, the Trees, or the Forest? Cancer Cell. 2015 Aug 10;28(2):149-50) constitute a major clinical problem (Zardavas D. et al. Clinical management of breast cancer heterogeneity. Nat Rev Clin Oncol. 2015 Jul;12(7):381-94), and the subsequent treatment of therapy-resistant recurrent and metastatic disease remains challenging.

Post-neoadjuvant adaptive treatment has become the standard for therapy-resistant HER2-positive (von Minckwitz G. et al. Trastuzumab Emtansine for Residual Invasive HER2-Positive Breast Cancer. N Engl J Med. 2019 Feb 14;380(7):617-628) and triple-negative breast cancer (Masuda N et al. Adjuvant Capecitabine for Breast Cancer after Preoperative Chemotherapy. N Engl J Med. 2017 Jun 1;376(22):2147-2159), leading to improvement of overall survival. Response-guided therapy based on early response to NACT leads to improved outcome in hormone-receptor (HR) positive breast cancer (von Minckwitz Get al. Response-guided neoadjuvant chemotherapy for breast cancer. J Clin Oncol. 2013 Oct 10;31(29):3623-30).

The pathological complete response rate (pCR) of luminal tumors to neoadjuvant chemotherapy is generally lower compared to other subtypes (Cortazar P et al. Pathological complete response and long-term clinical benefit in breast cancer: the CTNeoBC pooled analysis. Lancet. 2014 Jul 12;384(9938):164-72; Yau Cet al. Residual cancer burden after neoadjuvant chemotherapy and long-term survival outcomes in breast cancer: a multicentre pooled analysis of 5161 patients. Lancet Oncol. 2022 Jan;23(1):149-160). It has recently been shown that in high-risk luminal tumors, addition of immunotherapy to NACT increases the pCR rate (Cardoso F etal. KEYNOTE-756: Phase III study of neoadjuvant pembrolizumab (pembro) or placebo (pbo) + chemotherapy (chemo), followed by adjuvant pembro or pbo + endocrine therapy (ET) for early-stage high-risk ER+/HER2- breast cancer. LBA 21, ESMO congress 2023, Ann. Oncol, Volume 34 - Issue S2 - 2023; Loi Set al. A randomized, double-blind trial of nivolumab (NIVO) vs placebo (PBO) with neoadjuvant chemotherapy (NACT) followed by adjuvant endocrine therapy (ET) ± NIVO in patients (pts) with high-risk, ER+ HER2L primary breast cancer (BC) LBA 20, ESMO congress 2023, Ann. Oncol, Volume 34 - Issue S2 - 2023), and it will be important to define the subgroup of patients who will derive most benefit from this neoadjuvant approach.

Hence, in view of the above, there is a continuing need of means and methods useful in making clinical decisions on the systemic treatment of HR+ and HER2- breast cancers and thus for advanced means and methods for the prediction of the response or resistance and/or benefit to and/or outcome from a systemic cancer treatment of a subject suffering from or being at risk of developing a HR+ and HER2- breast cancer.

The present disclosure fulfills the continuing need for means and methods useful in making clinical decisions on the systemic treatment and thus for advanced means and methods for the prediction of the response or resistance and/or benefit to and/or outcome from systemic treatment of a subject suffering from or being at risk of developing a HR+ and HER2- breast cancer on the basis of readily accessible clinical and experimental data.

### BRIEF DESCRIPTION OF THE INVENTION

The present invention relates to a method for predicting a response or resistance to and/or a benefit from a neoadjuvant or adjuvant systemic treatment, preferably a neoadjuvant or adjuvant treatment, preferably a chemotherapy, more preferably a neoadjuvant treatment, most preferably a neoadjuvant chemotherapy, optionally comprising a taxane-containing agent for at least six weeks, in a subject suffering from a hormone receptor-positive (HR+) and human epidermal growth factor receptor 2-negative (HER2-) breast cancer, optionally an early-stage breast cancer, comprising the steps of: determining in a sample obtained from said subject the expression levels of three or more markers selected from Table 1.1; optionally normalizing the determined expression levels of said three or more markers; mathematically combining the determined expression levels of the three or more markers selected from Table 1.1 to generate a prediction, preferably a predictive score; and predicting the response or resistance to and/or benefit from said systemic treatment in said subject based on the prediction, preferably based on the predictive score; wherein the expression levels of the three or more markers are indicative for predicting the response or resistance to and/or benefit from the systemic treatment in said subject.

Equally, the invention relates to a method predicting the outcome of a systemic treatment, preferably a neoadjuvant or adjuvant treatment, preferably a chemotherapy, more preferably a neoadjuvant treatment, most preferably a neoadjuvant chemotherapy, optionally comprising a taxane-containing agent for at least six weeks, in a subject suffering from a hormone receptor-positive (HR+) and human epidermal growth factor receptor 2-negative (HER2-) breast cancer, optionally an early-stage breast cancer, comprising the steps of: determining in a sample obtained from said subject the expression levels of three or more markers selected from Table 1.1; mathematically combining the determined expression levels of the three or more markers selected from Table 1.1 to generate a prediction, preferably a predictive score; and predicting the outcome of said systemic treatment in said subject based on the prediction, preferably based on the predictive score; wherein the expression levels of the three or more markers are indicative for predicting the outcome the systemic treatment in said subject.

In one aspect of the present invention, one or more clinical parameters selected from the group consisting of pathological grading of the tumor, clinical tumor size (cT), clinical nodal status (cN), proliferation (in particular Ki-67), immune system markers (in particular lymphocytes, preferably tumor infiltrating lymphocytes (TILs)), estrogen receptor status, and variables derived from them (clinical and pathological stage, (CPS-EG) are determined.

In one aspect of the present invention, the response or resistance to the systemic treatment is measured by the long-term development of the disease, in particular development of invasive relapses, distant metastases, disease-related survival, and/or overall survival.

In one aspect of the present invention, the expression levels of three or more markers selected from any one of Tables 3-8 are determined.

In one aspect of the present invention, the expression levels of three or more markers selected from Table 4 are determined, preferably Table 5, more preferably Table 6 or Table 8, most preferably Table 7.1.

In one aspect of the present invention, the expression levels of three or more markers selected from Table 6 or 7.1 are determined and the high expression levels of three or more markers selected from Table 6 or 7.1 in a tumor sample are indicative for a good response of the subject to the neoadjuvant chemotherapy and/or the patient is predicted to benefit from the neoadjuvant treatment, optionally wherein the three or more markers are DNA repair genes.

In one aspect of the present invention, the expression levels of three or more markers selected from Table 8 are determined and the high expression levels of a plurality of markers selected from Table 8 in a tumor sample are indicative for a bad response of the subject to the treatment and/or the patient is predicted to not benefit from the treatment.

In one aspect of the present invention, the subject has a low chance to reach a pathological complete remission (pCR) after neoadjuvant treatment.

In one aspect of the present invention, at least five markers are selected from Table 1.1, preferably at least ten markers from Table 1.1, more preferably at least twenty markers from Table 1.1, more preferably at least fifty markers from Table 1.1, most preferably all markers from Table 1.1 are selected.

In one aspect of the present invention, the expression levels are determined at gene level, preferably mRNA level, in a hybridization-based method, a PCR based method, a microarray-based method, a quantitative transcriptomic analysis, a sequencing and/or next generation sequencing (NGS) method.

In one aspect of the present invention, the predictive score is compared to a reference score to determine the response or resistance to and/or benefit from said systemic treatment in said subject, optionally wherein the subject is predicted to respond to and/or benefit from the systemic treatment if the predictive score is equal to or below the reference score or the subject is predicted not to respond to, to be resistant to and/or not to benefit from the systemic treatment if the predictive score is above the reference score.

In one aspect of the present invention, patient is treated with said systemic treatment, preferably wherein said systemic treatment is neoadjuvant chemotherapy, if the subject is predicted to respond to and/or benefit from the systemic treatment; or the subject is to be treated with a different systemic treatment or with said systemic treatment and an additional systemic treatment, if the subject is predicted not to respond to, to be resistant to and/or not to benefit from the systemic treatment.

In one aspect of the present invention, an adaptive breast cancer subtype is classified in a tumor sample.

In one aspect of the present invention, the response or resistance to and/or benefit from a systemic treatment, preferably neoadjuvant chemotherapy, in a subject suffering from a HR+ and HER2- breast cancer is predicted based on the classification of the adaptive breast cancer subtype.

Further the present invention relates to a neoadjuvant chemotherapy for use in the treatment of a HR+ and HER2- breast cancer, wherein the neoadjuvant chemotherapy is to be administered to a subject who has been identified to respond to and/or benefit from said treatment or for whom said treatment has been determined to have a positive outcome.

In one aspect the present invention relates to a neoadjuvant chemotherapy comprising a taxane-containing agent for at least six weeks for use in the treatment of a HR+ and HER2-breast cancer, wherein the neoadjuvant chemotherapy is to be administered to a subject who has been identified to respond to and/or benefit from said treatment or for whom said treatment has been determined to have a positive outcome, optionally wherein the taxane-containing agent is any one of paclitaxel, docetaxel, abraxane, cabazitaxel, and albumin-bound paclitaxel (nab-paclitaxel).

Further the present invention relates to a method for therapy guidance, wherein a decision about systemic treatment, preferably neoadjuvant chemotherapy, is based on a prediction of response or resistance to and/or benefit from said systemic treatment, preferably neoadjuvant chemotherapy, wherein if the subject is predicted to respond to and/or benefit from said systemic treatment, preferably neoadjuvant chemotherapy, the patient is treated with said systemic treatment; or wherein if the subject is predicted not to respond to, to be resistant to and/or not to benefit from said systemic treatment, preferably neoadjuvant chemotherapy, the subject is treated with a different systemic treatment or with said systemic treatment and an additional systemic treatment.

Further the present invention relates to a method for stratifying the risk of subjects suffering from HER+ and HER2- breast cancer, wherein the method comprises predicting the response or resistance to and/or benefit from systemic treatment, preferably neoadjuvant chemotherapy.

Further the present invention relates to a method of treating a subject suffering from HR+ and HER2- breast cancer with systemic treatment, preferably neoadjuvant chemotherapy, wherein the subject has been predicted to respond to and/or benefit from said systemic treatment, preferably neoadjuvant chemotherapy, optionally wherein said neoadjuvant chemotherapy comprises a taxane-containing agent for at least six weeks

Further the present invention relates to a method of treating a subject suffering from HR+ and HER2- breast cancer with a combination of two or more systemic treatments, wherein the subject has been predicted not to respond to, to be resistant to and/or not to benefit from only one systemic treatment, preferably neoadjuvant chemotherapy.

Further the present invention relates to the use of a prognostic assay for predicting the response or resistance to and/or benefit from a systemic treatment, preferably neoadjuvant chemotherapy, in a subject suffering from a HR+ and HER2- breast cancer, wherein the prognostic assay comprises a RNA-based assay, optionally wherein the prognostic assay is one or more of NGS-based RNA analysis, PCR-based RNA analysis, sequencing-based RNA analysis, quantitative transcriptome analysis, quantitative RNA expression analysis, RNA-hybridization-based technologies including spatial RNA analysis or microarrays including Affymetrix.

Further the present invention relates to a computer-implemented method for predicting the response or resistance to and/or benefit from a systemic treatment in a subject suffering from a hormone receptor-positive (HR+) and human epidermal growth factor receptor 2-negative (HER2-) breast cancer comprising the steps of: obtaining expression level data of three or more markers selected from Table 1.1 in a tumor tissue sample; processing the expression level data of each of the three or more markers using a statistical model, wherein the processing comprises mathematically combining the determined expression levels of the three or more markers selected from Table 1.1 to generate a predictive score and optionally comprises normalizing the determined expression levels of said three or more markers prior to mathematically combining them; and predicting the response or resistance to and/or benefit from said systemic treatment in said subject based on the predictive score.

In one aspect, the present invention relates to a data processing system comprising means for carrying out the steps of the computer-implemented method.

In one aspect, the present invention relates to a computer program product comprising instructions which, when the program is executed by a computer, cause the computer and/or the data processing system to carry out the steps of the computer-implemented method.

In one aspect, the present invention relates to a computer-readable storage medium comprising instructions which, when executed by a computer, cause the computer to carry out the steps of the computer-implemented method of and having stored thereon the computer program.

Further, the present invention relates to the use of the method according to the method of the present invention for therapy control, therapy guidance, monitoring, risk assessment, and/or risk stratification in a subject suffering from a HR+ and HER2- breast cancer.

### DESCRIPTION OF THE FIGURES

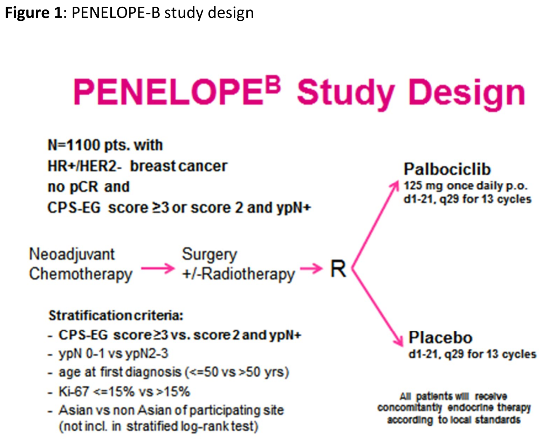

Figure 1 illustrates the study design of the Penelope^{B} study. R = Randomization (1:1).

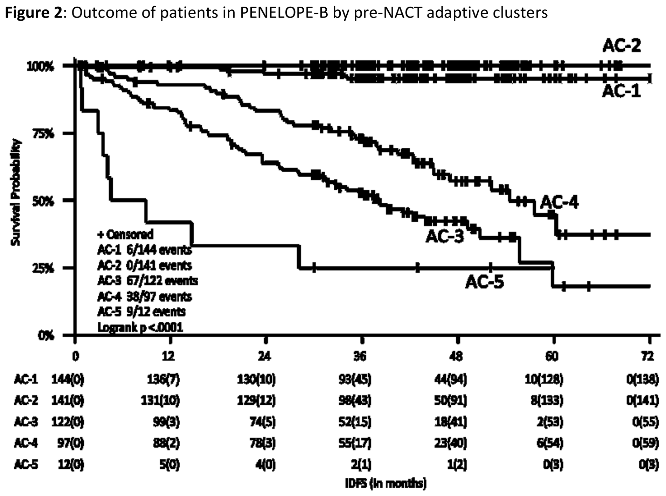

Figure 2 shows a Kaplan-Meier plot for the outcome of patients in PENELOPE^{B} study by pre-neoadjuvant chemotherapy adaptive clusters.

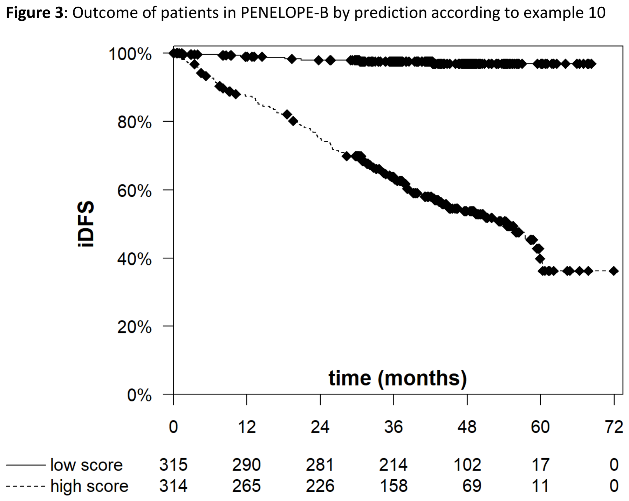

Figure 3 shows a Kaplan-Meier plot for the outcome of patients in PENELOPE^{B} study by prediction according to example 10.

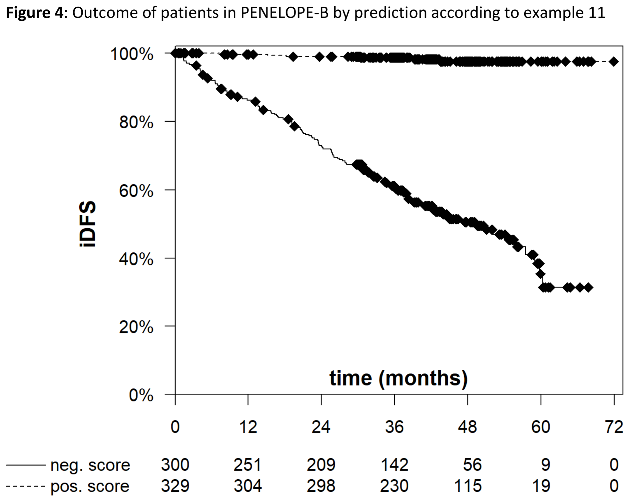

Figure 4 shows a Kaplan-Meier plot for the outcome of patients in PENELOPE^{B} study by prediction according to example 11.

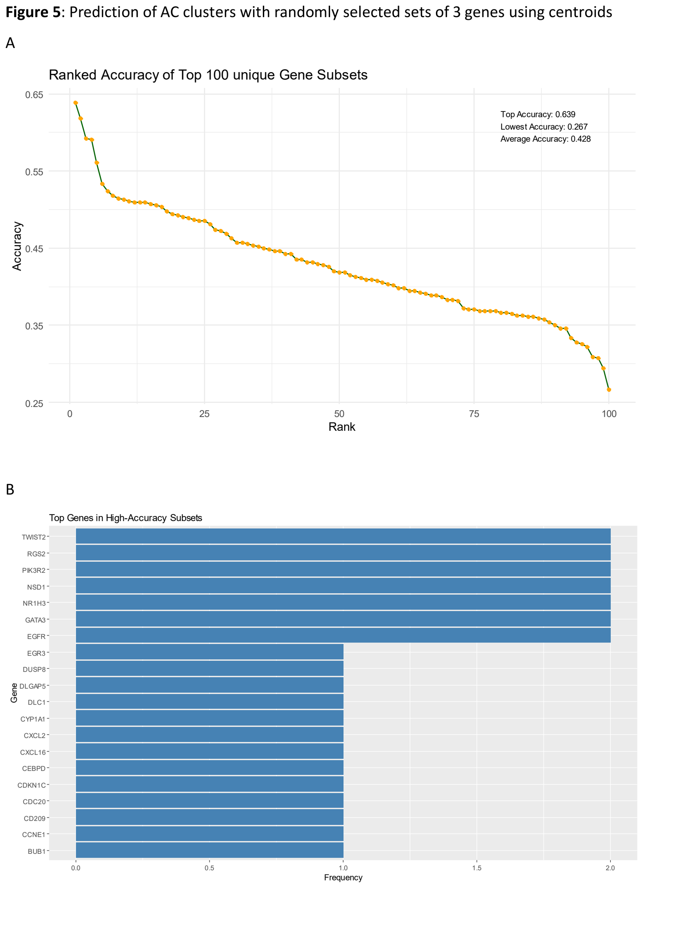

Figure 5 shows that randomly selected sets of 3 genes, wherein the genes are selected from Table 1.1, can predict the AC clusters using centroids. (A) For each gene set, 100 iterations were performed, and the accuracy results are presented in a line plot. (B) List of the 20 genes that are present in 1 or 2 gene sets.

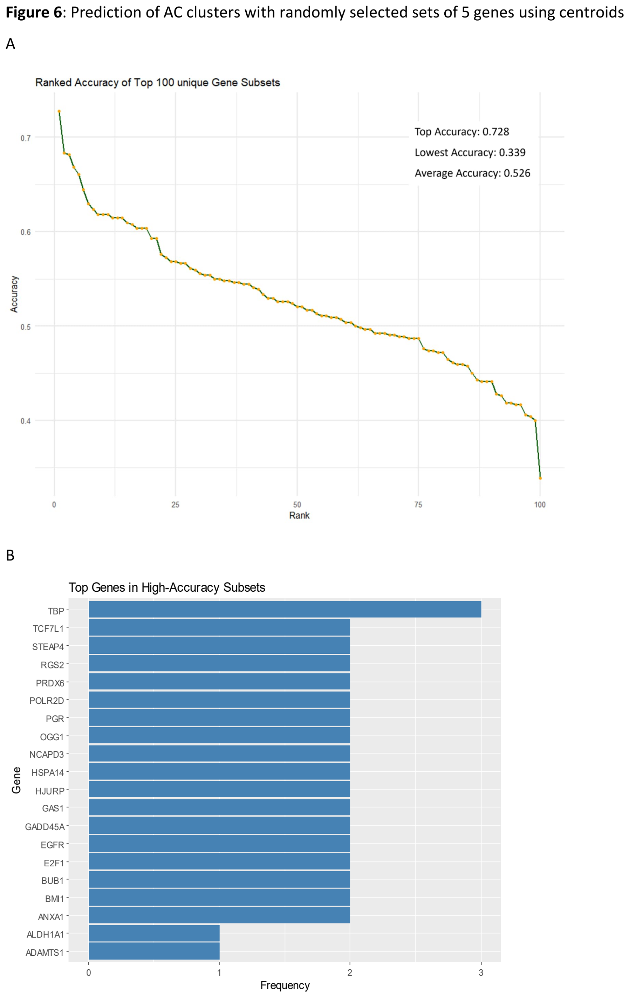

Figure 6 shows that randomly selected sets of 5 genes, wherein the genes are selected from Table 1.1, can predict the AC clusters using centroids. (A) For each gene set, 100 iterations were performed, and the accuracy results are presented in a line plot. (B) List of the 20 genes that are present in 1, 2 or 3 gene sets.

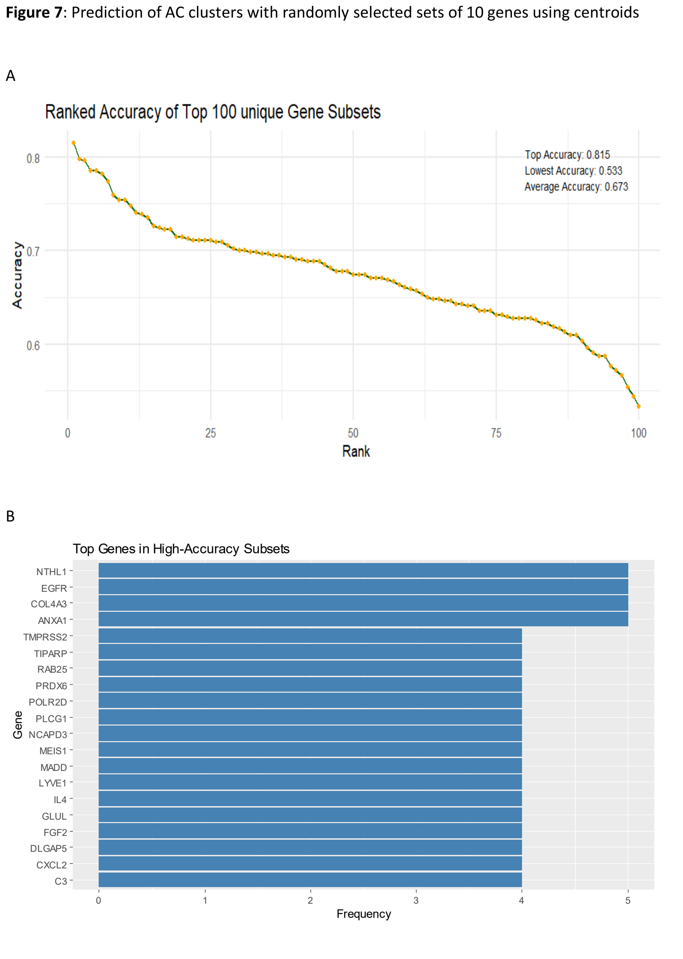

Figure 7 shows that randomly selected sets of 10 genes, wherein the genes are selected from Table 1.1, can predict the AC clusters using centroids. (A) For each gene set, 100 iterations were performed, and the accuracy results are presented in a line plot. (B) List of the 20 genes that are present in 4 or 5 gene sets.

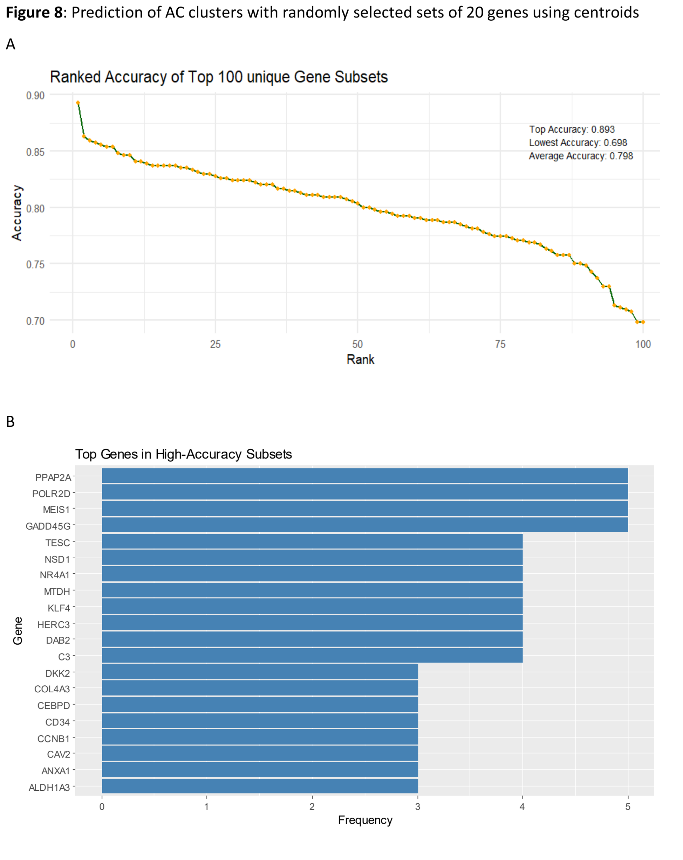

Figure 8 shows that randomly selected sets of 20 genes, wherein the genes are selected from Table 1.1, can predict the AC clusters using centroids. (A) For each gene set, 100 iterations were performed, and the accuracy results are presented in a line plot. (B) List of the 20 genes that are present in 3, 4 or 5 gene sets.

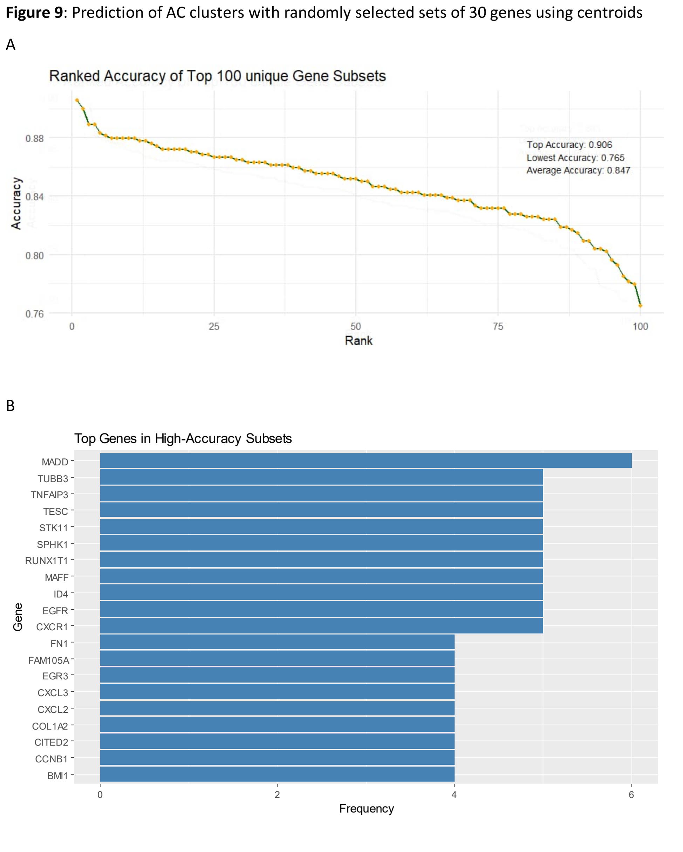

Figure 9 shows that randomly selected sets of 30 genes, wherein the genes are selected from Table 1.1, can predict the AC clusters using centroids. (A) For each gene set, 100 iterations were performed, and the accuracy results are presented in a line plot. (B) List of the 20 genes that are present in 4, 5 or 6 gene sets.

### DETAILED DESCRIPTION OF THE INVENTION

Unless defined otherwise, technical and scientific terms used herein have the same meaning as commonly understood by one of ordinary skill in the art to which this invention belongs.

The invention is based on the inventors' surprising finding that the expression level of biomarkers, which are differentially expressed before and after chemotherapy, can be used in a pretherapeutic setting to predict the response or resistance to and /or benefit from a systemic treatment in a subject and to identify different prognostic groups and adaptive cancer subtypes. Hence, the expression levels of certain biomarkers (i.e., markers listed in Table 1.1), such as DNA repair genes, or of certain combinations of biomarkers are indicative for a response or resistance to and/or benefit from a systemic treatment in a patient suffering from a hormone receptor-positive (HR+) and human epidermal growth factor receptor 2-negative (HER2-) breast cancer.

Hence, the present invention can be used in predicting a benefit from a systemic treatment and thus allows identification of therapy-resistant tumors of patients who require other forms of therapy. Further provided herein are new adaptive breast cancer subtypes which predict the response or resistance to and/or benefit from systemic treatment in a subject suffering from HR+ and HER2- breast cancer with high accuracy. The present invention is also relevant for improved therapeutic strategies.

In particular, the present invention relates, in one aspect, to a method for predicting a response or resistance to and/or benefit from a systemic treatment in a subject suffering from a hormone receptor positive (HR+) and human epidermal growth factor receptor 2-negative (HER2-) breast cancer, comprising the step of: determining in a sample obtained from said subject the expression level of three or more markers selected from Table 1.1, wherein the expression levels of the three or more markers are indicative for predicting the response or resistance to and/or benefit from the systemic treatment in said subject. Further provided herein is a method for predicting the outcome of a systemic treatment in a subject suffering from a hormone receptor positive (HR+) and human epidermal growth factor receptor 2-negative (HER2-) breast cancer, comprising the step of: determining in a sample obtained from said subject the expression level of three or more markers selected from Table 1.1, wherein the expression levels of the three or more markers are indicative for predicting the outcome of the systemic treatment in said subject.

**Table 1.1**

| | | | | | | | |
|---|---|---|---|---|---|---|---|
| ABCA3 | ABCD1 | ACKR1 | ADAMTS1 | ADIPOQ | ADIPOR1 | ADM | AKR1C3 |
| AKT3 | ALDH1A1 | ALDH1A3 | ANGPTL1 | ANLN | ANXA1 | ANXA3 | APOC2 |
| AQP1 | ASPM | BAG2 | BMI1 | BOC | BRCA1 | BTG2 | BUB1 |
| C3 | CACNG4 | CAV1 | CAV2 | CCL14 | CCL2 | CCNA2 | CCNB1 |
| CCNE1 | CCT5 | CD209 | CD34 | CDC14B | CDC20 | CDC6 | CDK5R1 |
| CDK9 | CDKN1A | CDKN1C | CDKN2C | CDKN3 | CEBPD | CELSR2 | CENPF |
| CITED2 | CLCA2 | CNTFR | COL11A1 | COL1A2 | COL4A3 | COL6A6 | CREB5 |
| CSF2 | CCN2 | CXCL10 | CXCL16 | CXCL2 | CXCL3 | CXCL9 | CXCR1 |
| CXCR4 | CYP1A1 | CYP1B1 | CCN1 | DAB2 | DES | DESI1 | DKK2 |
| DLC1 | DLGAP5 | DNAJC14 | DNAJC22 | DUSP1 | DUSP8 | DVL1 | E2F1 |
| EDN1 | EGFR | EGR1 | EGR3 | EMP1 | ETS2 | EXO1 | EZH2 |
| F3 | FABP4 | OTULINL | FANCA | FAS | FAT4 | FBXW7 | FCGR1A |
| FGF14 | FGF2 | FGF7 | FGFBP1 | VEGFD | TENM3 | FLNC | FLRT2 |
| FN1 | FOS | FOXA1 | FOXC1 | FOXO1 | GADD45A | GADD45B | GADD45G |
| GAPDH | GAS1 | GATA3 | GFRA1 | GGH | GLUL | GNGT2 | GPI |
| ADGRG6 | GPR160 | GPSM2 | GRIN2A | GSN | GSTP1 | GTSE1 | GZMB |
| H2AX | HBEGF | HERC3 | HES1 | HIF3A | H3C10 | HJURP | HMGB3 |
| HMGCS2 | HSPA14 | HSPB6 | ID4 | IGF1 | IGF2 | IGFBP6 | IKBKG |
| IL18 | IL20 | IL33 | IL4 | IL6 | IRF7 | ISG15 | JMJD1C |
| JUN | JUNB | JUND | KDM4B | KIF2C | KIT | KLF4 | KRT14 |
| KRT17 | KRT19 | KRT5 | LEPR | LIF | LIFR | LIG4 | LPL |
| LRP2 | LTB | LYVE1 | MADD | MAFF | MAGEL2 | MAML2 | MAOB |
| MAP1B | MAPK10 | MAZ | MEIS1 | MIF | MKI67 | MMP11 | MMP2 |
| MMP9 | MST1 | MT1A | MT1X | MTDH | MYB | MYBL1 | MYBL2 |
| MYCN | NAT1 | NCAPD3 | NF2 | NME1 | NOL3 | NOLC1 | CCN3 |
| NPC1 | NR1H3 | NR4A1 | NR4A3 | NRAS | NRIP1 | NSD1 | NTH L1 |
| NUF2 | NUP62 | NUSAP1 | OAS1 | OASL | OCLN | OGG1 | ORC6 |
| PABPC1 | PCNA | PCOLCE | PCSK6 | PDIA4 | PDPK1 | PDZK1 | PER1 |
| PFKL | PGR | PIAS4 | PIK3R2 | PIK3R3 | PIM2 | PITRM1 | PKM |
| PKMYT1 | PLAU | PLCG1 | PLK1 | PLK4 | PMAIP1 | PMS2P3 | POLD1 |
| POLD2 | POLR2D | PLPP1 | PLPP3 | PPID | PPP2R2C | PTPA | PRC1 |
| PRDX6 | PRKCZ | PRL | PTGS2 | PTN | RAB25 | RAD21 | RASD1 |
| RASGRF2 | RASSF7 | RBL1 | RELN | RET | RGS2 | RHOU | RIPK2 |
| RPS4X | RRM2 | RUNX1T1 | SAP30 | SELE | SERPINA3 | SERPINE1 | SERTAD1 |
| SFN | SFRP1 | SFRP4 | SGK1 | SLC16A3 | SLC19A3 | SLC27A4 | SLC2A3 |
| SLIT2 | SMAD9 | SOCS3 | SOX17 | SPC25 | SPHK1 | SPP1 | SPRY1 |
| SPRY2 | SRC | SREBF1 | STAT1 | STEAP4 | STK11 | STK36 | STMN1 |
| SUSD3 | SYCP2 | TAP1 | TBP | TCF4 | TCF7L1 | TEK | TESC |
| TFF1 | TGFB3 | TGFBR2 | THBS2 | THBS4 | TIPARP | TK1 | TMEM132A |
| TMPRSS2 | TNC | TNFAIP3 | TNFRSF1B | TNN | TNXB | TOP2A | TPO |
| TPX2 | TRAF2 | TRIB1 | TSC22D3 | TSPAN7 | TUBB3 | TWIST1 | TWIST2 |
| TXNIP | TYMS | UBE2T | VCAM1 | WNT2 | XBP1 | ZFP36L1 | |

Some of the markers listed in Table 1.1 are known to be highly inducible by preanalytical factors in tissue samples. To avoid preparation bias, these samples may not be selected from Table 1.1. Markers known to be highly inducible by preanalytical factors in tissue samples include but are not limited to BTG2, CCN1, DUSP1, EGR1, FOS, GADD45B, JUN, JUNB, PER1, RASD1, RGS2, SERPINE1, SLC2A3, SPRY1. Table 1.2 comprises a preferred subset of markers of Table 1.1 without, e.g., the markers which are known to be highly inducible by preanalytical factors in tissue samples. Hence, in a preferred aspect, provided herein is, a method for predicting a response or resistance to and/or benefit from a systemic treatment in a subject suffering from a hormone receptor positive (HR+) and human epidermal growth factor receptor 2-negative (HER2-) breast cancer, comprising the step of: determining in a sample obtained from said subject the expression level of three or more markers selected from Table 1.2, wherein the expression levels of the three or more markers are indicative for predicting the response or resistance to and/or benefit from the systemic treatment in said subject. Further provided herein is a method for predicting the outcome of a systemic treatment in a subject suffering from a hormone receptor positive (HR+) and human epidermal growth factor receptor 2-negative (HER2-) breast cancer, comprising the step of: determining in a sample obtained from said subject the expression level of three or more markers selected from Table 1.2, wherein the expression levels of the three or more markers are indicative for predicting outcome of the systemic treatment in said subject.

**Table 1.2**

| | | | | | | | |
|---|---|---|---|---|---|---|---|
| ABCA3 | ABCD1 | ACKR1 | ADAMTS1 | ADIPOQ | ADIPOR1 | ADM | AKR1C3 |
| AKT3 | ALDH1A1 | ALDH1A3 | ANGPTL1 | ANLN | ANXA1 | ANXA3 | APOC2 |
| AQP1 | ASPM | BAG2 | BMI1 | BOC | BRCA1 | BUB1 | C3 |
| CACNG4 | CAV1 | CAV2 | CCL14 | CCL2 | CCNA2 | CCNB1 | CCNE1 |
| CCT5 | CD209 | CD34 | CDC14B | CDC20 | CDC6 | CDK5R1 | CDK9 |
| CDKN1A | CDKN1C | CDKN2C | CDKN3 | CEBPD | CELSR2 | CENPF | CITED2 |
| CLCA2 | CNTFR | COL11A1 | COL1A2 | COL4A3 | COL6A6 | CREB5 | CSF2 |
| CCN2 | CXCL10 | CXCL16 | CXCL2 | CXCL3 | CXCL9 | CXCR1 | CXCR4 |
| CYP1A1 | CYP1B1 | DAB2 | DES | DESI1 | DKK2 | DLC1 | DLGAP5 |
| DNAJC14 | DNAJC22 | DUSP8 | DVL1 | E2F1 | EDN1 | EGFR | EGR3 |
| EMP1 | ETS2 | EXO1 | EZH2 | F3 | FABP4 | OTULINL | FANCA |
| FAS | FAT4 | FBXW7 | FCGR1A | FGF14 | FGF2 | FGF7 | FGFBP1 |
| VEGFD | TENM3 | FLNC | FLRT2 | FN1 | FOXA1 | FOXC1 | FOXO1 |
| GADD45A | GADD45G | GAPDH | GAS1 | GATA3 | GFRA1 | GGH | GLUL |
| GNGT2 | GPI | ADGRG6 | GPR160 | GPSM2 | GRIN2A | GSN | GSTP1 |
| GTSE1 | GZMB | H2AX | HBEGF | HERC3 | HES1 | HIF3A | H3C10 |
| HJURP | HMGB3 | HMGCS2 | HSPA14 | HSPB6 | ID4 | IGF1 | IGF2 |
| IGFBP6 | IKBKG | IL18 | IL20 | IL33 | IL4 | IL6 | IRF7 |
| ISG15 | JMJD1C | JUND | KDM4B | KIF2C | KIT | KLF4 | KRT14 |
| KRT17 | KRT19 | KRT5 | LEPR | LIF | LIFR | LIG4 | LPL |
| LRP2 | LTB | LYVE1 | MADD | MAFF | MAGEL2 | MAML2 | MAOB |
| MAP1B | MAPK10 | MAZ | MEIS1 | MIF | MMP11 | MMP2 | MMP9 |
| MST1 | MT1A | MT1X | MTDH | MYB | MYBL1 | MYBL2 | MYCN |
| NAT1 | NCAPD3 | NF2 | NME1 | NOL3 | NOLC1 | CCN3 | NPC1 |
| NR1H3 | NR4A1 | NR4A3 | NRAS | NRIP1 | NSD1 | NTH L1 | NUF2 |
| NUP62 | NUSAP1 | OAS1 | OASL | OCLN | OGG1 | ORC6 | PABPC1 |
| PCNA | PCOLCE | PCSK6 | PDIA4 | PDPK1 | PDZK1 | PFKL | PGR |
| PIAS4 | PIK3R2 | PIK3R3 | PIM2 | PITRM1 | PKM | PKMYT1 | PLAU |
| PLCG1 | PLK1 | PLK4 | PMAIP1 | PMS2P3 | POLD1 | POLD2 | POLR2D |
| PLPP1 | PLPP3 | PPID | PPP2R2C | PTPA | PRC1 | PRDX6 | PRKCZ |
| PRL | PTGS2 | PTN | RAB25 | RAD21 | RASGRF2 | RASSF7 | RBL1 |
| RELN | RET | RHOU | RIPK2 | RPS4X | RRM2 | RUNX1T1 | SAP30 |
| SELE | SERPINA3 | SERTAD1 | SFN | SFRP1 | SFRP4 | SGK1 | SLC16A3 |
| SLC19A3 | SLC27A4 | SLIT2 | SMAD9 | SOCS3 | SOX17 | SPC25 | SPHK1 |
| SPP1 | SPRY2 | SRC | SREBF1 | STAT1 | STEAP4 | STK11 | STK36 |
| STMN1 | SUSD3 | SYCP2 | TAP1 | TBP | TCF4 | TCF7L1 | TEK |
| TESC | TFF1 | TGFB3 | TGFBR2 | THBS2 | THBS4 | TIPARP | TK1 |
| TMEM132A | TMPRSS2 | TNC | TNFAIP3 | TNFRSF1B | TNN | TNXB | TOP2A |
| TPO | TPX2 | TRAF2 | TRIB1 | TSC22D3 | TSPAN7 | TUBB3 | TWIST1 |
| TWIST2 | TXNIP | TYMS | UBE2T | VCAM1 | WNT2 | XBP1 | ZFP36L1 |

Hence, the method of the invention can be used for predicting a response to a systemic treatment in a subject suffering from HR+ and HER2- breast cancer. It can also be used for predicting a resistance to a systemic treatment in a subject suffering from HR+ and HER2-breast cancer. It can further be used for predicting a benefit from a systemic treatment in a subject suffering from HR+ and HER2- breast cancer. It can further be used for predicting the outcome of a systemic treatment of a subject suffering from a HR+ and HER2- breast cancer.

In particular, the method for predicting the response or resistance to and/or the benefit from systemic treatment in a subject suffering from HR+ and HER2- breast cancer further comprises the steps of mathematically combining the determined expression levels of the three or more markers selected from Table 1.1 or Table 1.2, preferably Table 1.2, to generate a prediction, preferably a predictive score and predicting the response or resistance to and/or benefit from said systemic treatment in said subject based on the prediction, preferably on the predictive score. For example, the method can comprise the steps of (1) combining the expression levels of the three or more markers into one single value called "score" for each subject, (2) applying cutoffs to the score to classify the subject into a risk group and (3) deciding on systemic treatment for the subject based on the individual risk group. The expression levels of good prognosis markers, of bad prognosis markers, and/or of good and bad prognosis markers may be combined into one numerical score, wherein the score is indicative of the response or resistance to and/or benefit from a systemic treatment in a subject suffering from HR+ and HER2- breast cancer. Provided herein is a method for predicting a response or resistance to and/or benefit from a systemic treatment in a subject suffering from a hormone receptor-positive (HR+) and human epidermal growth factor receptor 2-negative (HER2-) breast cancer, comprising the steps of: determining in a sample obtained from said subject the expression levels of three or more markers selected from Table 1.1 or 1.2, preferably Table 1.2; mathematically combining the determined expression levels of the three or more markers selected from Table 1.1 or 1.2, preferably Table 1.2, to generate a prediction; and predicting the response or resistance to and/or benefit from said subject to said systemic treatment based on the prediction; wherein the expression levels of the three or more markers are indicative for predicting the response or resistance to and/or benefit from the systemic treatment in said subject. Further provided is a method for predicting a response or resistance to and/or benefit from a systemic treatment in a subject suffering from a hormone receptor-positive (HR+) and human epidermal growth factor receptor 2-negative (HER2-) breast cancer, comprising the steps of: determining in a sample obtained from said subject the expression levels of three or more markers selected from Table 1.1 or 1.2, preferably Table 1.2; mathematically combining the determined expression levels of the three or more markers selected from Table 1.1 or 1.2, preferably Table 1.2, to generate a predictive score; and predicting the response or resistance to and/or benefit from said subject to said systemic treatment based on the predictive score; wherein the expression levels of the three or more markers are indicative for predicting the response or resistance to and/or benefit from the systemic treatment in said subject.

Hence, the method of the invention can be used for predicting a response to a systemic treatment in a subject suffering from HR+ and HER2- breast cancer wherein the method further comprises the steps of mathematically combining the determined expression levels of the three or more markers selected from Table 1.1 or 1.2, preferably Table 1.2, to generate a prediction, preferably a predictive score and predicting the response to the systemic treatment in said subject based on the prediction, preferably the predictive score. It can also be used for predicting a resistance to a systemic treatment in a subject suffering from HR+ and HER2- breast cancer wherein the method further comprises the steps of mathematically combining the determined expression levels of the three or more markers selected from Table 1.1 or 1.2, preferably Table 1.2, to generate a prediction, preferably a predictive score and predicting the resistance to the systemic treatment in said subject based on the prediction, preferably the predictive score. It can further be used for predicting a benefit from a systemic treatment in a subject suffering from HR+ and HER2- breast cancer, wherein the method further comprises the steps of mathematically combining the determined expression levels of the three or more markers selected from Table 1.1 or 1.2, preferably Table 1.2, to generate a prediction, preferably a predictive score and predicting the benefit from the systemic treatment in said subject based on the prediction, preferably the predictive score.

In particular, the systemic treatment to which the subject suffering from a HR+ and HER2-breast cancer is predicted to respond and/or benefit from or to be resistant to according to the methods of the present invention may be any one of chemotherapy, endocrine therapy, immunotherapy, targeted therapy, optionally wherein the targeted therapy comprises the use of small molecules and/or of antibodies, anti-angiogenic therapy, biological therapy, or gene therapy. The systemic treatment may also comprise any combination of two or more of chemotherapy, endocrine therapy, immunotherapy, targeted therapy, optionally wherein the targeted therapy comprises the use of small molecules and/or of antibodies, anti-angiogenic therapy, biological therapy, or gene therapy, wherein the systemic treatment regimens can be received simultaneously and/or sequentially. The systemic treatment may be neoadjuvant or adjuvant treatment, preferably the systemic treatment is neoadjuvant treatment. In particular, the systemic treatment may be chemotherapy. Preferably, the systemic treatment may be neoadjuvant chemotherapy. For example, the (neoadjuvant) chemotherapy may comprise one or more of a taxane-containing agent, anthracycline, and/or a platinum-based chemotherapeutic drug. If the neoadjuvant chemotherapy comprises administering of a taxane-containing agent, the taxane-containing agent should be administered for at least six weeks. The taxane-containing agent is not particularly limited and can be any one of paclitaxel, docetaxel, abraxane, cabazitaxel, and albumin-bound paclitaxel (nab-paclitaxel). The platinum-based chemotherapeutic drug agent is not particularly limited and can be any one of cisplatin, carboplatin, and oxaliplatin. A subject receiving neoadjuvant chemotherapy, as used herein, may receive the neoadjuvant chemotherapy for at least 16 weeks, preferably wherein the subject receives a taxane-containing agent for at least six weeks of the neoadjuvant chemotherapy. A subject receiving neoadjuvant chemotherapy according to the present invention may also receive neoadjuvant chemotherapy for less than 16 weeks, if the subject has a progressive disease that occurred after at least six weeks of taxane-containing neoadjuvant chemotherapy. Hence provided herein, is a method for predicting a response or resistance to and/or benefit from a neoadjuvant chemotherapy, optionally comprising a taxane-containing agent for at least six weeks, in a subject suffering from a hormone receptor-positive (HR+) and human epidermal growth factor receptor 2-negative (HER2-) breast cancer, comprising the steps of: determining in a sample obtained from said subject the expression levels of three or more markers selected from Table 1.1 or 1.2, preferably Table 1.2; mathematically combining the determined expression levels of the three or more markers selected from Table 1.1 or 1.2, preferably Table 1.2, to generate a prediction, preferably a predictive score; and predicting the response or resistance to and/or benefit from said subject to said neoadjuvant chemotherapy, optionally comprising a taxane-containing agent for at least six weeks, based on the prediction, preferably the predictive score; wherein the expression levels of the three or more markers are indicative for predicting the response or resistance to and/or benefit from the neoadjuvant chemotherapy comprising a taxane-containing agent in said subject.

The HR+ and HER2+ breast cancer may be an early-stage breast cancer. As defined by the national cancer institute (NCI) an early-stage breast cancer is a breast cancer that has not spread beyond the breast or the axillary lymph nodes. This includes ductal carcinoma in situ and stage I, stage IIA, stage IIB, and stage IIIA breast cancers. Thus, an early-stage breast cancer may, for example, be stage I, stage IIA, stage IIB or stage IIIA breast cancer. The term "early-stage" and its definition is known by the skilled person. Thus, provided herein are methods to predict the response or resistance to and/or benefit from a systemic treatment, preferably a systemic neoadjuvant or systemic adjuvant chemotherapy or a systemic neoadjuvant treatment, more preferably a systemic neoadjuvant chemotherapy, optionally comprising a taxane-containing agent, in a subject suffering from an early-stage, HR+ and HER2- breast cancer. The method may comprise further steps, as disclosed herein.

In addition, the expression levels of said markers can also be mathematically combined with one or more additional markers, one or more clinical parameters and/or one or more non-clinical parameters of the respective subject to predict the response or resistance to and/or benefit from a systemic treatment, preferably a neoadjuvant or adjuvant treatment, preferably a chemotherapy, more preferably a neoadjuvant treatment, most preferably a neoadjuvant chemotherapy, optionally comprising a taxane-containing agent for at least six weeks, in said subject. In some embodiments, further biomarkers include housekeeping genes, known tumor markers and/or proliferation markers. Clinical parameters may for example be selected the group consisting of pathological grading of the tumor, clinical tumor size (cT), clinical nodal status (cN), proliferation markers (in particular Ki-67), immune system markers (in particular lymphocytes, preferably tumor infiltrating lymphocytes (TILs)), estrogen receptor status, progesterone receptor status, and variables derived from them (clinical and pathological stage, CPS-EG). Non-clinical parameters comprise age, sex, body weight and/or body-mass index (BMI). The biomarkers and/or other clinical or non-clinical parameters are well known to the person skilled in the art (see German Guideline Programm in Oncology (GGPO), Evidence-based Guideline for the Early Detection, Diagnosis, Treatment and Follow-up of Breast Cancer, Version 4.4 - May 2021 AWMF-Registernummer: 032/045OL; European Society for Medical Oncology (ESMO) Clinical Practice Guidelines: Breast Cancer https://www.esmo.org/guidelines/guidelines-by-topic/esmo-clinical-practice-guidelines-breast-cancer/early-breast-cancer; and American Society of Clinical Oncology (ASCO) guidelines on breast cancer, https://society.asco.org/practice-patients/guidelines/breast-cancer) and can be determined according to routine clinical practice. Thus, in combination with the determined expression levels these one or more additional markers, one or more clinical parameters and/or one or more non-clinical parameters are also indicative for the prediction of the response or resistance to and/or benefit from a systemic treatment, preferably a neoadjuvant or adjuvant treatment, preferably a chemotherapy, more preferably a neoadjuvant treatment, most preferably a neoadjuvant chemotherapy, optionally comprising a taxane-containing agent for at least six weeks, in a subject suffering from HR+ and HER2- breast cancer. The method provided herein may further comprise normalizing the determined expression levels of said three or more markers and said one or more additional markers, one or more clinical parameters and/or one or more non-clinical parameters, and mathematically combining the normalized expression levels of three or more markers selected from Table 1.1 or 1.2, preferably Table 1.2, with the normalized levels of the one or more additional markers, one or more clinical parameters and/or one or more non-clinical parameters to generate a prediction, preferably a predictive score and predicting the response or resistance to and/or benefit from said systemic treatment in said subject based on the prediction, preferably based on the predictive score.

In some aspects of the invention, the methods further comprise normalizing the determined expression levels of said three or more markers prior to mathematically combining them. The normalization of the determined expression levels of the three or more markers may be based on the expression levels of further markers, e.g., on the expression levels of housekeeping genes or on overall the expression level of all determined markers.

In some aspects, the mathematical combination of the determined expression levels of said three or more markers selected from Table 1.1 or 1.2, preferably Table 1.2, to generate a prediction, preferably a predictive score, is performed using a statistical and/or mathematical model. Or the mathematical combination of the determined expression levels of said three or more markers selected from Table 1.1 or 1.2, preferably Table 1.2, and the determined one or more additional markers, the one or more clinical parameters and/or the one or more non-clinical parameters to generate a prediction, preferably a predictive score, is performed using a statistical and/or mathematical model. The statistical and/or mathematical model is not particularly limited and may, for example, be one or more of weighted or non-weighted average, linear regression, logistic regression, Cox regression, non-linear regression, regression tress, any other regression, principal component analysis, ANOVA, committee voting, supervised clustering, unsupervised clustering, nearest neighbor clustering, hierarchical clustering, any other clustering, and other machine learning algorithms.

According to the methods of the present invention, the response or resistance to systemic treatment is measured by the long-term development of the disease, in particular development of invasive relapses, distant metastases, disease-related survival, and/or overall survival.

In addition to Table 1.1 or 1.2, preferably Table 1.2, the three or more markers may also be selected from any one of Tables 3-8, preferably from Table 4, more preferably from Table 5, more preferably from Table 6 or Table 8, most preferably from Table 7.1 for the prediction of the response or resistance to and/or benefit from a systemic treatment in a subject suffering from HR+ and HER2- breast cancer. Each one of Tables 3-8 comprises a subset of the markers of Table 1.1. Statistical and/or mathematical models, as mentioned above, may be used to generate such Tables comprising a subset of the markers of Table 1.1. The number of marker subsets and combination of markers is not particularly limited. The following marker subsets are exemplary subsets, and the invention is not limited to the exemplary subsets.

**Table 2: Overview of exemplary marker subsets**

| **Table Number** | **Corresponding to Example** | **Keywords** |
|---|---|---|
| 1.1 | 1 | Differentially expressed markers before and after NACT |
| 1.2 | N/A | Exclusion of markers known to be highly inducible by preanalytical factors in tissue samples |
| 3 | 2 | Highly correlating markers; subset of Table 1 |
| 4 | 3 | Prognostic markers; subset of Table 1 |
| 5 | 4 | Prognostic markers distinguishing between different adaptive cancer subtypes; subset of Table 4 |
| 6 | 5 | Good prognosis markers; subset of Table 5 |
| 7.1 | 6 | Highly correlating DNA repair markers; mutual markers of Tables 3 and 6 |
| 7.2 | 7,9 | Subset of highly correlating DNA repair markers; subset of Table 7.1 |
| 7.3 | N/A | Subset of highly correlating DNA repair markers; subset of Table 7.1 |
| 8 | 8 | Bad prognosis markers; subset of Table 5 |

For Example, the markers of Table 3 are markers highly correlating to each other. By these correlations common biological mechanisms, such as DNA repair, stress response, IFN response, estrogen signaling, proliferation, cell cycle, immune response, including inflammatory response and immune-oncology, endocrine pathways, epidermal growth factor/platelet derived growth factor (EGF/PDGF) signaling, stromal and/or endothelial cells, or IL6-Jak-Stat signaling, are revealed. In some cases, the expression levels of three or more markers selected from Table 3 are indicative for the expression level of another marker selected from Table 3. The markers of Table 5 can distinguish different adaptive breast cancer subtypes. The markers of Table 4 are prognostic for invasive disease-free survival (iDFS), wherein the subset of markers of Table 6 are indicative of a good prognosis and the markers of Table 8 are indicative of a bad prognosis. The higher the determined expression level of a marker selected from Table 6 in a tumor sample obtained from a subject suffering from a HR+ and HER2- breast cancer before neoadjuvant chemotherapy, the better is the predicted outcome of said subject. In contrast, the higher the determined expression level of a marker selected from Table 86 in a tumor sample obtained from a subject suffering from a HR+ and HER2- breast cancer before neoadjuvant chemotherapy, the worse is the predicted outcome. Table 7.1 contains a subset of markers which is contained in both Table 3 and Table 6. Thus, the markers of Table 7.1 are highly correlating and indicative of a good prognosis. Furthermore, the markers of Table 7.1 are involved in a common biological mechanism, i.e., DNA-repair. In some preferred aspects, the three or more markers whose expression levels are indicative for predicting the response or resistance to and/or benefit from a systemic treatment, preferably a neoadjuvant or adjuvant treatment, preferably a chemotherapy, more preferably a neoadjuvant treatment, most preferably a neoadjuvant chemotherapy, optionally comprising a taxane-containing agent for at least six weeks, in a subject suffering from HR+ and HER2- breast cancer are selected from Table 7.1. The markers of Table 7.2 and 7.3 represent a further subset of the markers of Table 7.1. In some embodiments, the expression levels of the markers selected from Table any one of Tables 3-8, preferably Table 7.1, may be mathematically combined with one or more additional markers, one or more clinical parameters and/or one or more non-clinical parameters to predict the likelihood of an iDFS event of a subject and/or the outcome of a subject. Provided herein in some aspects is method predicting a response or resistance to and/or benefit from a systemic treatment, preferably a neoadjuvant or adjuvant treatment, preferably a chemotherapy, more preferably a neoadjuvant treatment, most preferably a neoadjuvant chemotherapy, optionally comprising a taxane-containing agent for at least six weeks, in a subject suffering from a HR+ and HER2- breast cancer, optionally an early-stage breast cancer, comprising the steps of: determining in a sample obtained from said subject the expression levels of three or more markers selected from any one of Tables 3-8, preferably from Table 4, more preferably from Table 5, more preferably from Table 6, Table 7.2., Table 7.3, or Table 8, most preferably from Table 7.1; mathematically combining the determined expression levels of said three or more markers to generate a prediction, preferably a predictive score, optionally wherein the determined expression levels of said three or more markers are further mathematically combined with one or more additional markers, one or more clinical and/or one or more non-clinical parameters to generate a predictive score; and predicting the response or resistance to and/or benefit from said systemic treatment in said subject based on the prediction, preferably based on the predictive score. According to the methods provided herein, the expression level of three or more markers selected from Tables 3-8, preferably from Table 4, more preferably from Table 5, more preferably from Table 6, Table 7.2., Table 7.3, or Table 8, most preferably from Table 7.1 may be determined and used for predicting the outcome of a systemic treatment, by mathematically combining the determined expression levels of the three or more markers to generate a prediction, preferably a predictive score, and the outcome of said systemic treatment in said subject is predicted based on said prediction, preferably based on the predictive score. In addition, the methods provided herein may comprise mathematically combining the expression levels of said three or more markers selected from any one of Table 3-8, preferably from Table 4, more preferably from Table 5, more preferably from Table 6, most preferably from Table 7.1, with one or more additional markers, one or more clinical parameters and/or one or more non-clinical parameters of the respective subject to generate a prediction, preferably a predictive score and predicting the outcome of said systemic treatment in said subject based on the prediction, preferably based on the predictive score. The mathematical combination may be performed using a statistical and/or mathematical model known in the art.

As indicated above, the enrichment of the three or more markers selected from any one of Tables 6, 7.1, 7.2 or 7.3 in a tumor sample is indicative for a good response of the subject to the systemic treatment, preferably a neoadjuvant or adjuvant treatment, preferably a chemotherapy, more preferably a neoadjuvant treatment, most preferably a neoadjuvant chemotherapy, optionally comprising a taxane-containing agent for at least six weeks, and/or the patient is predicted to benefit from the systemic treatment, preferably a neoadjuvant or adjuvant treatment, preferably a chemotherapy, more preferably a neoadjuvant treatment, most preferably a neoadjuvant chemotherapy, optionally comprising a taxane-containing agent for at least six weeks. An enrichment of the three or more markers selected from any one of Tables 7.1, 7.2 or 7.3 in a tumor sample obtained from a subject suffering from a HR+ and HER2- breast cancer, optionally early-stage breast cancer, before initiation of a neoadjuvant chemotherapy may be indicative for a good response of the subject to the neoadjuvant chemotherapy and/or the patient is predicted to benefit from the neoadjuvant chemotherapy, wherein the three or more markers are markers associated with DNA repair. In contrast, an enrichment of the three or more markers selected from any one of Table 7.1, 7.2 or 7.3 in a tumor sample obtained from a subject suffering from a HR+ and HER2- breast cancer after neoadjuvant chemotherapy may be indicative for a bad prognosis. An enrichment of a plurality of markers selected from Table 8 in a tumor sample obtained from a subject suffering from a HR+ and HER2- breast cancer is indicative for a bad response of the subject to the systemic treatment, preferably a neoadjuvant or adjuvant treatment, preferably a chemotherapy, more preferably a neoadjuvant treatment, most preferably a neoadjuvant chemotherapy, optionally comprising a taxane-containing agent for at least six weeks, and/or the patient is predicted to not benefit from the systemic treatment, preferably a neoadjuvant or adjuvant treatment, preferably a chemotherapy, more preferably a neoadjuvant treatment, most preferably a neoadjuvant chemotherapy, optionally comprising a taxane-containing agent for at least six weeks. In particular, an enrichment of a plurality of markers selected from Table 8 in a tumor sample obtained from a subject suffering from an early-stage, HR+ and HER2- breast cancer before neoadjuvant chemotherapy may be indicative for a bad response of the subject to the neoadjuvant chemotherapy and/or the patient is predicted to not benefit from the neoadjuvant chemotherapy, optionally wherein the neoadjuvant chemotherapy comprises a taxane-containing agent for at least six weeks.

**Table 3: 184 highly correlating markers**

| | | | | | | | |
|---|---|---|---|---|---|---|---|
| ACKR1 | ADIPOQ | ALDH1A1 | ANGPTL1 | ANLN | ANXA1 | AQP1 | ASPM |
| BOC | BRCA1 | BUB1 | CAV1 | CAV2 | CCL14 | CCL2 | CCNA2 |
| CCNB1 | CCNE1 | CD209 | CDC14B | CDC20 | CDC6 | CDK5R1 | CDKN1C |
| CDKN2C | CDKN3 | CENPF | CLCA2 | CNTFR | COL11A1 | COL1A2 | COL4A3 |
| CREB5 | CSF2 | CCN2 | CXCL10 | CXCL16 | CXCL2 | CXCL3 | CXCL9 |
| CXCR1 | CYP1A1 | CCN1 | DAB2 | DKK2 | DLC1 | DLGAP5 | DNAJC14 |
| DUSP8 | E2F1 | EDN1 | EGFR | ETS2 | EXO1 | EZH2 | FANCA |
| FAS | FAT4 | FBXW7 | FGF14 | FGF2 | FGF7 | FGFBP1 | TENM3 |
| FLNC | FLRT2 | FN1 | FOXO1 | GAS1 | GNGT2 | GPSM2 | GRIN2A |
| GSN | GTSE1 | GZMB | H2AX | HERC3 | HES1 | H3C10 | HJURP |
| HSPB6 | ID4 | IGF1 | IGF2 | IGFBP6 | IKBKG | IL18 | IL33 |
| IL4 | IL6 | IRF7 | ISG15 | KIF2C | LEPR | LIF | LIG4 |
| LPL | LTB | LYVE1 | MADD | MAGEL2 | MEIS1 | MKI67 | MMP11 |
| MMP2 | MTDH | MYBL1 | MYBL2 | MYCN | CCN3 | NR1H3 | NR4A3 |
| NRAS | NSD1 | NUF2 | NUP62 | NUSAP1 | OAS1 | OASL | OGG1 |
| ORC6 | PCNA | PCOLCE | PFKL | PIM2 | PITRM1 | PKMYT1 | PLAU |
| PLK1 | PLK4 | POLD1 | POLR2D | PLPP1 | PLPP3 | PRC1 | PRDX6 |
| PRL | PTGS2 | PTN | RASD1 | RASGRF2 | RBL1 | RELN | RIPK2 |
| RPS4X | RRM2 | RUNX1T1 | SAP30 | SELE | SFRP1 | SFRP4 | SLC19A3 |
| SLIT2 | SMAD9 | SOCS3 | SOX17 | SPC25 | SPHK1 | SPRY1 | SPRY2 |
| STAT1 | STK36 | STMN1 | TAP1 | TBP | TCF4 | TEK | TESC |
| TGFB3 | TGFBR2 | THBS2 | TK1 | TNFAIP3 | TNFRSF1B | TNN | TNXB |
| TOP2A | TPX2 | TSPAN7 | TWIST2 | TYMS | UBE2T | VCAM1 | WNT2 |

**Table 4: 206 prognostic markers**

| | | | | | | | |
|---|---|---|---|---|---|---|---|
| ABCA3 | ACKR1 | ADAMTS1 | ADIPOQ | ADIPOR1 | ANLN | ANXA1 | APOC2 |
| AQP1 | ASPM | BMI1 | BRCA1 | BTG2 | BUB1 | C3 | CACNG4 |
| CCL14 | CCNA2 | CCNB1 | CCNE1 | CCT5 | CD209 | CDC6 | CDK5R1 |
| CDK9 | CDKN1A | CDKN1C | CDKN2C | CEBPD | CENPF | CITED2 | CCN2 |
| CXCL16 | CXCR1 | CXCR4 | CYP1B1 | DAB2 | DESI1 | DKK2 | DLC1 |
| DLGAP5 | DNAJC14 | DUSP1 | DUSP8 | DVL1 | EGR1 | EGR3 | EXO1 |
| OTULINL | FANCA | FCGR1A | FGF7 | FLNC | FOXA1 | FOXC1 | GAS1 |
| GATA3 | GFRA1 | GGH | GLUL | GNGT2 | GPI | GPR160 | GPSM2 |
| GSN | GSTP1 | GTSE1 | H2AX | HBEGF | HERC3 | HES1 | H3C10 |
| HJURP | HMGB3 | HMGCS2 | HSPA14 | IGF1 | IGF2 | IGFBP6 | IKBKG |
| IL18 | JMJD1C | JUNB | KDM4B | KIF2C | LIF | LIG4 | LTB |
| LYVE1 | MADD | MAFF | MAML2 | MAP1B | MAPK10 | MAZ | MKI67 |
| MMP2 | MST1 | MTDH | MYB | MYCN | NAT1 | NCAPD3 | NF2 |
| NME1 | NOL3 | NOLC1 | CCN3 | NR1H3 | NR4A1 | NR4A3 | NRAS |
| NRIP1 | NSD1 | NTH L1 | NUF2 | NUP62 | OAS1 | OASL | OCLN |
| OGG1 | ORC6 | PCSK6 | PDPK1 | PDZK1 | PER1 | PFKL | PGR |
| PIAS4 | PIK3R2 | PIK3R3 | PIM2 | PITRM1 | PKM | PKMYT1 | PLAU |
| PLCG1 | PLK4 | PMAIP1 | PMS2P3 | POLD1 | POLR2D | PLPP1 | PLPP3 |
| PPID | PPP2R2C | PTPA | PRC1 | PRDX6 | PRKCZ | PTGS2 | RASSF7 |
| RGS2 | RIPK2 | RPS4X | RRM2 | RUNX1T1 | SAP30 | SELE | SERPINA3 |
| SERTAD1 | SFN | SFRP1 | SFRP4 | SGK1 | SLC27A4 | SLC2A3 | SMAD9 |
| SOCS3 | SPHK1 | SPP1 | SPRY1 | SRC | SREBF1 | STAT1 | STEAP4 |
| STK11 | STK36 | STMN1 | SUSD3 | TAP1 | TBP | TCF4 | TCF7L1 |
| TEK | TESC | TGFB3 | TGFBR2 | THBS2 | TIPARP | TK1 | TMEM132A |
| TMPRSS2 | TNFAIP3 | TNFRSF1B | TNN | TNXB | TOP2A | TPX2 | TRAF2 |
| TUBB3 | TXNIP | TYMS | VCAM1 | WNT2 | XBP1 | | |

**Table 5: 91 markers from Table 4 distinguishing AC-1 and AC-2 vs AC-3, AC-4, and AC-5**

| | | | | | | | |
|---|---|---|---|---|---|---|---|
| ADIPOR1 | APOC2 | BMI1 | CDK5R1 | CDKN2C | CXCL16 | CXCR4 | DESI1 |
| DNAJC14 | DUSP8 | DVL1 | OTULINL | GGH | GLUL | GNGT2 | GPI |
| GPR160 | GPSM2 | GSN | GSTP1 | H2AX | HERC3 | HES1 | H3C10 |
| HMGB3 | HMGCS2 | IKBKG | IL18 | JMJD1C | LIG4 | MADD | MAFF |
| MKI67 | MST1 | MTDH | MYB | MYCN | NCAPD3 | NF2 | NME1 |
| NOL3 | NR1H3 | NR4A1 | NRAS | NRIP1 | NSD1 | NUF2 | NUP62 |
| OCLN | OGG1 | PFKL | PIAS4 | PIK3R2 | PIM2 | PITRM1 | PKM |
| PKMYT1 | PLAU | PLCG1 | PLK4 | PMAIP1 | POLR2D | PLPP1 | PLPP3 |
| PPID | PRC1 | PRDX6 | PRKCZ | RGS2 | RIPK2 | RPS4X | SAP30 |
| SFN | SOCS3 | SPHK1 | SRC | STK11 | STK36 | TAP1 | TBP |
| TCF4 | TCF7L1 | TEK | TESC | TGFB3 | TGFBR2 | TIPARP | TMEM132A |
| TMPRSS2 | TPX2 | TXNIP | | | | | |

**Table 6: 66 good prognosis markers from Table 5**

| | | | | | | | |
|---|---|---|---|---|---|---|---|
| ADIPOR1 | BMI1 | CDK5R1 | CDKN2C | DESI1 | DNAJC14 | DUSP8 | DVL1 |
| GGH | GLUL | GNGT2 | GPI | GPR160 | GPSM2 | HMGB3 | IKBKG |
| IL18 | LIG4 | MADD | MAFF | MST1 | MTDH | MYB | MYCN |
| NCAPD3 | NF2 | NME1 | NOL3 | NR1H3 | NR4A1 | NRAS | NRIP1 |
| NSD1 | NUF2 | NUP62 | OCLN | OGG1 | PFKL | PIAS4 | PIK3R2 |
| PIM2 | PITRM1 | PKM | PKMYT1 | PLAU | PLCG1 | PLK4 | PMAIP1 |
| POLR2D | PLPP1 | PLPP3 | PPID | PRC1 | PRDX6 | PRKCZ | RGS2 |
| RIPK2 | RPS4X | SAP30 | SFN | SPHK1 | SRC | STK11 | STK36 |
| TAP1 | TBP | | | | | | |

**Table 7.1: 24 correlating DNA repair genes**

| | | | | | | | |
|---|---|---|---|---|---|---|---|
| CDK5R1 | DNAJC14 | DUSP8 | GNGT2 | IL18 | LIG4 | MADD | MTDH |
| NR1H3 | NRAS | NSD1 | NUP62 | OGG1 | PFKL | PIM2 | PITRM1 |
| PLK4 | POLR2D | PRDX6 | RIPK2 | RPS4X | SAP30 | STK36 | TBP |

**Table 7.2: Subset of correlating DNA repair genes**

| | | | | | | | |
|---|---|---|---|---|---|---|---|
| PIM2 | STK36 | OGG1 | MTDH | PITRM1 | TBP | NUP62 | PLK4 |

**Table 7.3: Subset of correlating DNA repair genes**

| | | | | | | | |
|---|---|---|---|---|---|---|---|
| CDK5R1 | DNAJC14 | DUSP8 | GNGT2 | IL18 | LIG4 | MADD | NR1H3 |
| NRAS | NSD1 | NUP62 | PFKL | PIM2 | PITRM1 | PLK4 | POLR2D |
| PRDX6 | RIPK2 | RPS4X | SAP30 | STK36 | TBP | | |

**Table 8: 25 bad prognosis markers from Table 5**

| | | | | | | | |
|---|---|---|---|---|---|---|---|
| APOC2 | CXCL16 | CXCR4 | OTULINL | GSN | GSTP1 | H2AX | HERC3 |
| HES1 | H3C10 | HMGCS2 | JMJD1C | MKI67 | SOCS3 | TCF4 | TCF7L1 |
| TEK | TESC | TGFB3 | TGFBR2 | TIPARP | TMEM132A | TMPRSS2 | TPX2 |
| TXNIP | | | | | | | |

In particular, the subject may have a low chance to reach a pathological complete remission (pCR) after the systemic treatment, preferably said subject does not reach a pCR after the systemic treatment, preferably a neoadjuvant or adjuvant treatment, preferably a chemotherapy, more preferably a neoadjuvant treatment, most preferably a neoadjuvant chemotherapy, optionally comprising a taxane-containing agent for at least six weeks.

As indicated above, the determination of the expression level of three or more markers is sufficient to carry out the methods of the present invention. However, in some aspects, at least five markers are selected from Table 1.1 or Table 1.2, preferably at least ten markers from Table 1.1 or Table 1.2, more preferably at least twenty markers from Table 1.1 or Table 1.2, more preferably at least fifty markers from Table 1.1 or Table 1.2, more preferably all markers from Table 1.1 or Table 1.2, most preferably all markers from Table 1.2 are selected. In further aspects, at least five markers are selected from any one of Tables 3-8, preferably at least ten markers are selected from any one of Tables 3-6, 7.1, 7.3 or 8, more preferably at least twenty are selected from any one of Tables 3-6, 7.1, 7.3 or 8, most preferably all markers from any one of Tables 3-8 are selected. In a preferred aspect, at least five markers are selected from Table 7.1, preferably at least ten markers, more preferably at least twenty, most preferably all markers from Table 7.1 are selected.

In some aspects, three or more of CDK5R1, DNAJC14, DUSP8, GNGT2, IL18, LIG4, MADD, NR1H3, NRAS, NSD1, NUP62, PFKL, PIM2, PITRM1, PLK4, POLR2D, PRDX6, RIPK2, RPS4X, SAP30, STK36, and TBP are selected from Table 7.1 (i.e, Table 7.3). Preferably five or more, more preferably ten or more, more preferably twenty or more, most preferably all of CDK5R1, DNAJC14, DUSP8, GNGT2, IL18, LIG4, MADD, NR1H3, NRAS, NSD1, NUP62, PFKL, PIM2, PITRM1, PLK4, POLR2D, PRDX6, RIPK2, RPS4X, SAP30, STK36, and TBP are selected from Table 7.1. In general, any combination of the three or more markers selected from Table 1.1, Table 1.2 or any one of Tables 3-8, preferably Table 7.1, is indicative for predicting the response or resistance to and/or benefit from the systemic treatment in said subject. Thus, any combination of three or more markers may be selected, wherein the combination of the three or more markers may depend on the statistical and/or mathematical model that is applied to the expression level data.

Typically, the sample in the context of the present invention is a tumor tissue sample such as a primary tumor tissue sample, particularly a core biopsy sample, more particularly a core biopsy sample from a primary tumor before any systemic treatment, more particularly a core biopsy sample from a primary tumor before neoadjuvant treatment. It may, however, also be a post-surgical residual tumor tissue sample or a post-surgical lymph node sample. In one typical application, the sample is a formalin-fixed, paraffin-embedded (FFPE) tumor tissue sample. The sample may be obtained before the systemic treatment from which the response or resistance and/or benefit of the subject is to be predicted, e.g., before neoadjuvant treatment or before adjuvant treatment. Preferably, the sample is obtained before neoadjuvant treatment, most preferably the sample is obtained before neoadjuvant chemotherapy.

The expression level of the three or more markers can in principle be determined at gene level and/or at protein level. In some embodiments, the expression level of the three or more markers is determined at gene level, i.e., on DNA level and/or on RNA level, in a hybridization-based method, a PCR based method, a microarray-based method, a quantitative transcriptomic analysis, a sequencing and/or next generation sequencing (NGS) method. In a preferred embodiment, the expression level is determined at mRNA level in a hybridization-based method, a PCR based method, a microarray-based method, a quantitative transcriptomic analysis, a sequencing and/or NGS method. When determined at protein level, immunoassay methods are typically used, e.g., an immunohistochemistry (IHC) assay. Alternatively CISH, ELISA (enzyme linked immunoassay), RIA (radioimmunoassay) or the use of protein microarrays, two- hybrid screening, blotting methods including western blot, one- and two-dimensional gel electrophoresis, isoelectric focusing as well as methods being based on mass spectrometry like MALDI-TOF and the like may be used to determine protein expression level of the markers.

In some aspects, the methods of the present invention comprise comparing the predictive score, to a reference score to determine the response or resistance to and/or benefit from a systemic treatment preferably a neoadjuvant or adjuvant treatment or a chemotherapy, more preferably a neoadjuvant treatment, most preferably a neoadjuvant chemotherapy, optionally comprising a taxane-containing agent, in a subject suffering from a HR+ and HER2-breast cancer, optionally wherein the subject is predicted to respond to and/or benefit from said systemic treatment if the predictive score is equal to or below the reference score or the subject is predicted not to respond to and/or not to benefit from the systemic treatment if the predictive score is above the reference score. In some embodiments, a low predictive score is indicative for a good response of the subject to the systemic treatment, and the subject is predicted to benefit from the systemic treatment, wherein a good response is characterized by a low risk of occurrence of an invasive disease-free survival (iDFS) event. In contrast, a high predictive score may be indicative for a bad response of the subject to the systemic treatment, and the subject is predicted to not benefit from the systemic treatment, wherein a bad response is characterized by a high risk of occurrence of an invasive disease-free survival (iDFS) event. The predictive score may be compared to a reference score to determine the outcome from a systemic treatment in a subject suffering from HR+ and HER2-breast cancer, wherein the subject is predicted to have a good outcome if the predictive score is equal to or below the reference score or the subject is predicted have a bad outcome if the predictive score is above the reference score.

The systemic treatment of the subject depends on the prediction of the outcome. Hence, if the subject is predicted to respond to and/or benefit from the systemic treatment, the patient may receive said systemic treatment according to the methods of the invention. As indicated above, the systemic treatment may be any one of chemotherapy, endocrine therapy, immunotherapy, targeted therapy, optionally wherein the targeted therapy comprises the use of small molecules and/or of antibodies, anti-angiogenic therapy, biological therapy, or gene therapy. The treatment is neoadjuvant or adjuvant treatment, preferably chemotherapy, more preferably neoadjuvant chemotherapy, most preferably the systemic treatment is neoadjuvant chemotherapy comprising a taxane-containing agent for a least six weeks. Hence, if the subject is predicted to respond to and/or benefit from the systemic treatment, the subject is to be treated with said systemic treatment. Hence, provided herein is a method for predicting a response or resistance to and/or benefit from a neoadjuvant chemotherapy, optionally comprising a taxane-containing agent for at least six weeks, in a subject suffering from a HR+ and HER2- breast cancer, comprising the steps of: determining in a tumor sample obtained from said subject the expression levels of three or more markers, preferably all markers, selected from any one of Tables 1.1, 1.2, or 3-8, preferably from Table 1.2 or 7.1, most preferably from Table 7.1; mathematically combining the determined expression levels of said markers, optionally combining the determined expression levels of said markers with one or more additional markers, one or more clinical parameters and/or one or more non-clinical parameters, to generate a prediction, preferably a predictive score; optionally comparing the predictive score to a reference score; predicting a response or resistance to and/or benefit from said neoadjuvant chemotherapy in said subject; and if the subject is predicted to respond to and/or benefit from said neoadjuvant chemotherapy, deciding that the subject is to be treated with said neoadjuvant chemotherapy, optionally comprising a taxane-containing agent for at least six weeks. A subject suffering from HR+ and HER2- breast cancer, who is predicted to respond to and/or benefit from a systemic treatment according to the methods of the present invention, may further benefit from additional non-systemic treatments, such as radiation therapy or surgery.

It might also be the case that the subject is predicted to respond to and/or benefit from a systemic treatment, however the overall prognosis of the subject is not good. Or the subject is predicted to only partially respond to and/or only partially benefit from a systemic treatment. Or the subject is predicted to have better response and/or an additional benefit from a systemic treatment which comprises the combination of two or more systemic treatment regimens, i.e., any combination of two or more of chemotherapy, endocrine therapy, immunotherapy, targeted therapy, optionally wherein the targeted therapy comprises the use of small molecules and/or of antibodies, anti-angiogenic therapy, biological therapy, or gene therapy wherein the two or more systemic treatment regimens can be received simultaneously and/or sequentially, i.e., one systemic treatment may be neoadjuvant systemic treatment and a second systemic treatment may be an adjuvant or post-neoadjuvant systemic treatment or both systemic treatments may be neoadjuvant systemic treatments. Thus, the combination of two systemic treatment regimens, e.g., the combination of a neoadjuvant chemotherapy and a post-neoadjuvant or adjuvant systemic treatment, may improve the outcome of a patient. In particular, a subject who is predicted to respond to and/or benefit from a neoadjuvant chemotherapy, optionally comprising a taxane-containing agent for at least six weeks, wherein the prediction is made according to the methods of the present disclosure, may additionally receive post-neoadjuvant or adjuvant treatment, optionally one of targeted therapy, endocrine therapy, immunotherapy, or anti-angiogenic therapy. For example, a subject who is predicted to respond to and/or benefit from a neoadjuvant chemotherapy, optionally comprising a taxane-containing agent for at least six weeks, wherein the prediction is made according to the methods of the present disclosure, may additionally receive a post-neoadjuvant or adjuvant cyclin-dependent kinases 4 and 6 (CDK4/6) inhibitor therapy. A subject suffering from HR+ and HER2- breast cancer, who is predicted to partially respond to and/or benefit from a systemic treatment according to the methods of the present invention, may further benefit from additional non-systemic treatments, such as radiation therapy or surgery.

In contrast, if the subject is predicted not to respond to, be resistant to and/or not to benefit from the systemic treatment, wherein the prediction is made according to the methods disclosed herein, the subject may be treated with a the systemic treatment in an intensified form, i.e., higher dosage of the systemic treatment, the systemic treatment with more cycles of drug administration, the systemic treatment with a longer duration (e.g., more than 16 weeks), the systemic treatment intensified after care (e.g., higher frequency of checks for metastases), the systemic treatment with increased supervision, the systemic treatment with additional neoadjuvant treatment and/or the systemic treatment in combination with one or more additional systemic post-neoadjuvant or adjuvant treatments. In some embodiments, if the subject is predicted not to respond to, to be resistant to and/or not to benefit from the systemic treatment, the subject may be treated with a different systemic treatment. Examples of such "different treatments" include, but are not limited to, a treatment with different chemotherapeutics, a treatment with additional drugs (e.g., with a CDK4/6 inhibitor), a treatment comprising endocrine therapy, a treatment comprising immunotherapy, a treatment comprising targeted therapy, such as a targeted therapy comprising small molecules or antibodies, a treatment comprising anti-angiogenic therapy, a treatment comprising the combination of two or more treatment regimens, and/or additional alterations compared to the systemic treatment to which the subject is predicted not to respond to, to be resistant to and/or not to benefit from.

In some embodiments, a subject predicted not to respond to, to be resistant to and/or not to benefit from a neoadjuvant chemotherapy, optionally comprising a taxane-containing agent for at least six weeks, wherein the prediction is made according to the methods of the present disclosure, may respond to and/or benefit from a different systemic treatment and the subject is to be treated with a different and/or intensified systemic treatment, wherein the different and/or intensified systemic treatment is any one of a systemic treatment comprising a higher dosage, a systemic treatment with more cycles of drug administration, a systemic treatment with a longer duration (e.g., more than 16 weeks), a systemic treatment intensified after care (e.g., higher frequency of checks for metastases), a systemic treatment with increased supervision, a systemic treatment with different chemotherapeutics, a systemic treatment with additional drugs (e.g., with a CDK4/6 inhibitor), a systemic treatment comprising endocrine therapy, a systemic treatment comprising immunotherapy, a systemic treatment comprising targeted therapy, such as a targeted therapy comprising small molecules or antibodies, a treatment comprising anti-angiogenic therapy, and/or a more aggressive systemic treatment.

In some embodiments, if a subject predicted patient not to respond to, to be resistant to and/or not to benefit from a neoadjuvant chemotherapy alone, optionally comprising a taxane-containing agent, wherein the prediction is made according to the methods of the present disclosure, the subject may respond to and/or benefit from said neoadjuvant chemotherapy in combination with an additional systemic treatment regimen, optionally an additional post-neoadjuvant or adjuvant treatment, and the subject is treated with said neoadjuvant chemotherapy and said post-neoadjuvant or adjuvant treatment, wherein said post-neoadjuvant or adjuvant treatment is any one of a systemic treatment with different chemotherapeutics, a systemic treatment with additional drugs (e.g., with a CDK4/6 inhibitor), a systemic treatment comprising endocrine therapy, a systemic treatment comprising immunotherapy, a systemic treatment comprising targeted therapy, such as a targeted therapy comprising small molecules or antibodies, and/or a systemic treatment comprising anti-angiogenic therapy. For example, a subject predicted patient not to respond to, to be resistant to and/or not to benefit from a neoadjuvant chemotherapy, may receive immunotherapy in addition to said neoadjuvant chemotherapy. Or subject predicted to be resistant to, not respond to and/or not benefit from a neoadjuvant chemotherapy, may receive post-neoadjuvant or adjuvant systemic treatment in addition to said neoadjuvant chemotherapy.

The molecular profiling of the subject might reveal that the subject is predicted to have a good outcome but to not benefit from neoadjuvant chemotherapy. Thus, in some embodiments, a subject predicted not to respond to, to be resistant to and/or not to benefit from a systemic treatment, preferably a neoadjuvant or adjuvant treatment, preferably a chemotherapy, more preferably a neoadjuvant treatment, most preferably a neoadjuvant chemotherapy, optionally comprising a taxane-containing agent for at least six weeks, is predicted to have a good outcome, optionally the subject is predicted to have a good outcome, if the subject is treated with a different systemic treatment, wherein said different systemic treatment is any one of a treatment with more cycles of drug administration, a systemic treatment with a longer duration (e.g., more than 16 weeks), a systemic treatment with different chemotherapeutics, a systemic treatment with additional drugs (e.g., with a CDK4/6 inhibitor), a systemic treatment comprising endocrine therapy, a systemic treatment comprising immunotherapy, a systemic treatment comprising targeted therapy, such as a targeted therapy comprising small molecules or antibodies, and/or a treatment comprising anti-angiogenic therapy. A subject suffering from HR+ and HER2- breast cancer, who is predicted not to respond to, to be resistant to and/or not to benefit from a systemic treatment according to the methods of the present invention, may further benefit from additional non-systemic treatments, such as radiation therapy or surgery.

In some aspects, the subject receives non-systemic treatment, such as radiation therapy and/or surgery, in addition to the systemic treatment, wherein the decision for non-systemic treatment is made independently of the prediction of the response or resistance to and/or benefit from the systemic treatment.

The method provided herein may further comprise classifying an adaptive breast cancer subtype in a tumor sample obtained from a subject suffering from a HR+ and HER2- breast cancer, wherein the classification may be performed based on the determined expression levels of three or more markers selected from Table 1.1 or Table 1.2. The response or resistance to and/or benefit from a systemic treatment, preferably neoadjuvant chemotherapy, in a subject suffering from a HR+ and HER2- breast cancer may be predicted based on the classification of the adaptive breast cancer subtype.

In one aspect, the invention relates to neoadjuvant chemotherapy for use in the treatment of a HR+ and HER2- breast cancer, optionally an early-stage breast cancer, wherein the neoadjuvant chemotherapy is to be administered to a subject, who has been identified to respond to and/or benefit from said systemic neoadjuvant chemotherapy and/or for whom said neoadjuvant chemotherapy has been determined to have a positive outcome according to the methods of the present invention, optionally wherein the chemotherapy comprises one or more of a taxane-containing agent, anthracycline, and/or a platinum-based chemotherapeutic drug. Further provided herein a neoadjuvant chemotherapy comprising a taxane-containing agent for at least six weeks for use in the treatment of a HR+ and HER2-breast cancer, wherein the neoadjuvant chemotherapy is to be administered to a subject who has been identified to respond to and/or benefit from said systemic neoadjuvant chemotherapy and/or for whom said systemic neoadjuvant chemotherapy has been determined to have a positive outcome according to the methods of the present invention, optionally wherein the taxane-containing agent is any one of paclitaxel, docetaxel, abraxane, cabazitaxel, and albumin-bound paclitaxel (nab-paclitaxel). Further provided is anthracycline or a platinum-based chemotherapeutic drug and a taxane-containing agent for use in the treatment of a HR+ and HER2- breast cancer, optionally an early-stage breast cancer, wherein the anthracycline or the platinum-based chemotherapeutic drug and the taxane-containing agent are to be administered to a subject, who has been identified to respond to and/or benefit from said chemotherapeutic drugs and/or for whom said chemotherapeutic drugs have been determined to have a positive outcome according to the methods of the present invention, optionally wherein the chemotherapeutic drugs are to be administered in a neoadjuvant setting and the treatment is a neoadjuvant treatment. Also provided herein is a combination of a neoadjuvant chemotherapy, optionally comprising a taxane-containing agent for at least six weeks, and an additional systemic therapy for use in the treatment of a HR+ and HER2- breast cancer, optionally in an early stage, wherein the neoadjuvant chemotherapy and the additional systemic therapy are to be administered to a subject who has been identified to respond to and/or benefit from said combination of a neoadjuvant chemotherapy and the additional systemic therapy and/or for whom said combination of a neoadjuvant chemotherapy and the additional systemic therapy has been determined to have a positive outcome according to the methods of the present invention. The additional systemic therapy is not particularly limited and may be neoadjuvant, post-neoadjuvant or adjuvant therapy. The second systemic therapy may be any one of an endocrine therapy, an immunotherapy, a chemotherapy, a targeted therapy, or anti-angiogenic therapy. For example, the second systemic therapy may comprise a CDK4/6 inhibitor.

Provided herein is, in another aspect, a method for therapy guidance, wherein a decision about systemic treatment, preferably a neoadjuvant or adjuvant treatment, preferably a chemotherapy, more preferably a neoadjuvant treatment, most preferably a neoadjuvant chemotherapy, optionally comprising a taxane-containing agent for at least six weeks, is based on a prediction of response or resistance to and/or benefit from said systemic treatment, wherein the prediction is made according to the methods of the present invention, wherein, if the subject is predicted to respond to and/or benefit from said systemic treatment, the patient is treated with said systemic treatment; or wherein, if the subject is predicted not to respond to, to be resistant to and/or not to benefit from said systemic treatment, the subject is to be treated with a different systemic treatment, optionally with a different neoadjuvant chemotherapy, or the subject is to be treated with a combination of said systemic treatment, preferably neoadjuvant chemotherapy, and an additional neoadjuvant, post-neoadjuvant or adjuvant treatment, wherein the additional neoadjuvant, post-neoadjuvant or adjuvant treatment is any one of a systemic therapy comprising endocrine therapy, a systemic therapy comprising immunotherapy, a systemic therapy comprising targeted therapy or a systemic therapy comprising anti-angiogenic therapy, or the subject is to be treated with any one of endocrine therapy alone, immunotherapy alone, targeted therapy alone or anti-angiogenic therapy alone. A subject suffering from HR+ and HER2- breast cancer may further benefit from additional non-systemic treatments, such as radiation therapy or surgery.

Further provided herein is, a method for stratifying the risk of subjects suffering from HER+ and HER2- breast cancer, wherein the method comprises predicting the response or resistance to and/or benefit from systemic treatment, preferably a neoadjuvant or adjuvant treatment, preferably a chemotherapy, more preferably a neoadjuvant treatment, most preferably a neoadjuvant chemotherapy, optionally comprising a taxane-containing agent for at least six weeks, according to the methods of the present invention.

In another aspect, the present invention relates to a method of treating a subject suffering from HR+ and HER2- breast cancer, optionally an early-stage breast cancer, with a systemic treatment, wherein the systemic treatment is neoadjuvant or adjuvant treatment, and wherein the subject has been predicted to respond to and/or benefit from said systemic treatment. Further provided is a method of treating a subject suffering from HR+ and HER2-breast cancer, optionally an early-stage breast cancer, with neoadjuvant or adjuvant chemotherapy, wherein the subject has been predicted to respond to and/or benefit from said neoadjuvant or adjuvant chemotherapy. Further provided is a method of treating a subject suffering from HR+ and HER2- breast cancer, optionally an early-stage breast cancer, with neoadjuvant chemotherapy, wherein the subject has been predicted to respond to and/or benefit from said neoadjuvant chemotherapy. Also provided is a method of treating a subject suffering from HR+ and HER2- breast cancer, optionally an early-stage breast cancer, with neoadjuvant chemotherapy comprising a taxane-containing agent for at least six weeks, wherein the subject has been predicted to respond to and/or benefit from said neoadjuvant chemotherapy comprising a taxane-containing agent.

In another aspect, the present invention relates to a method of treating a subject suffering from a HR+ and HER2- breast cancer, optionally an early-stage breast cancer, with a combination of two or more systemic treatments, wherein the subject has been predicted not to respond to, to be resistant to and/or not to benefit from only one systemic treatment. Further provided is a method of treating a subject suffering from a HR+ and HER2- breast cancer, optionally an early-stage breast cancer, with a combination of a neoadjuvant chemotherapy and immunotherapy, wherein the subject has been predicted not to respond to, to be resistant to and/or not to benefit from the neoadjuvant chemotherapy alone. Also provided is a method of treating a subject suffering from a HR+ and HER2- breast cancer, optionally an early-stage breast cancer, with a combination of a neoadjuvant chemotherapy comprising a taxane-containing agent for at least six weeks and immunotherapy, wherein the subject has been predicted not to respond to, to be resistant to and/or not to benefit from the neoadjuvant chemotherapy comprising a taxane-containing agent alone. Further provided is a method of treating a subject suffering from a HR+ and HER2- breast cancer, optionally an early-stage breast cancer, with a combination of a neoadjuvant chemotherapy and endocrine therapy, wherein the subject has been predicted not to respond to, to be resistant to and/or not to benefit from the neoadjuvant chemotherapy alone. Also provided is a method of treating a subject suffering from a HR+ and HER2- breast cancer, optionally an early-stage breast cancer, with a combination of a neoadjuvant chemotherapy comprising a taxane-containing agent for at least six weeks and endocrine therapy, wherein the subject has been predicted not to respond to, to be resistant to and/or not to benefit from the neoadjuvant chemotherapy comprising a taxane-containing agent alone. Further provided is a method of treating a subject suffering from a HR+ and HER2- breast cancer, optionally an early-stage breast cancer, with a combination of a neoadjuvant chemotherapy and targeted therapy, wherein the subject has been predicted not to respond to, to be resistant to and/or not to benefit from the neoadjuvant chemotherapy alone. Also provided is a method of treating a subject suffering from a HR+ and HER2- breast cancer, optionally an early-stage breast cancer, with a combination of a neoadjuvant chemotherapy comprising a taxane-containing agent for at least six weeks and targeted therapy, wherein the subject has been predicted not to respond to, to be resistant to and/or not to benefit from the neoadjuvant chemotherapy comprising a taxane-containing agent alone. Further provided is a method of treating a subject suffering from a HR+ and HER2- breast cancer, optionally an early-stage breast cancer, with a combination of a neoadjuvant chemotherapy and anti-angiogenic therapy, wherein the subject has been predicted not to respond to, to be resistant to and/or not to benefit from the neoadjuvant chemotherapy alone. Also provided is a method of treating a subject suffering from a HR+ and HER2- breast cancer, optionally an early-stage breast cancer, with a combination of a neoadjuvant chemotherapy comprising a taxane-containing agent for at least six weeks and anti-angiogenic therapy, wherein the subject has been predicted not to respond to, to be resistant to and/or not to benefit from the neoadjuvant chemotherapy comprising a taxane-containing agent alone. A method of treating a subject suffering from a HR+ and HER2- breast cancer, optionally an early-stage breast cancer, with a combination of a neoadjuvant chemotherapy and a post-neoadjuvant systemic treatment, optionally a post-neoadjuvant treatment comprising a CDK4/6 inhibitor, wherein the subject has been predicted not to respond to, to be resistant to and/or not to benefit from the neoadjuvant chemotherapy alone is also provided. Further provided is a method of treating a subject suffering from a HR+ and HER2- breast cancer, optionally an early-stage breast cancer, with a combination of a neoadjuvant chemotherapy comprising a taxane-containing agent for at least six weeks and a post-neoadjuvant systemic treatment, optionally a post-neoadjuvant treatment comprising a CDK4/6 inhibitor, wherein the subject has been predicted not to respond to, to be resistant to and/or not to benefit from the neoadjuvant chemotherapy comprising a taxane-containing agent alone.

Further provided herein is the use of a prognostic assay for predicting the response to and/or benefit from a systemic treatment, preferably a neoadjuvant or adjuvant treatment, preferably a chemotherapy, more preferably a neoadjuvant treatment, most preferably a neoadjuvant chemotherapy, optionally comprising a taxane-containing agent for at least six weeks, in a subject suffering from a HR+ and HER2- breast cancer, wherein the prognostic assay is a RNA-based test, optionally wherein the prognostic assay comprises one or more of NGS-based RNA analysis, PCR-based RNA analysis, sequencing-based RNA analysis, quantitative transcriptome analysis, quantitative RNA expression analysis, RNA-hybridization-based technologies including spatial RNA analysis or microarrays including Affymetrix. The RNA-based prognostic assay, optionally the one or more of NGS-based RNA analysis, PCR-based RNA analysis, sequencing-based RNA analysis, quantitative transcriptome analysis, quantitative RNA expression analysis, RNA-hybridization-based technologies including spatial RNA analysis or microarrays including Affymetrix may be performed using RNA isolated from a tumor sample obtained from said subject suffering from a HR+ and HER2- breast cancer. The prognostic assay according to the present invention may be used for determining the expression level of three or more markers selected from any one of Tables 1.1., 1.2, or 3-8, preferably from Table 1.2 or 7.1, most preferably from Table 7.1, in a tumor sample of a subject suffering from HR+ and HER2- breast cancer, wherein the expression level of said three or more markers is indicative of the response or resistance to and/or benefit from a systemic treatment, preferably a neoadjuvant chemotherapy, in said subject. The prognostic assay may further comprise the determination of one or more additional markers (i.e., markers not comprised in any one of Tables 1.1, 1.2, or 3-8; such as housekeeping genes, known tumor markers and/or proliferation markers), of one or more clinical parameters and/or one or more of non-clinical parameters.

The present invention further relates to a computer-implemented method for predicting the response or resistance to and/or benefit from a systemic treatment, preferably a neoadjuvant or adjuvant treatment, preferably a chemotherapy, more preferably a neoadjuvant treatment, most preferably a neoadjuvant chemotherapy, optionally comprising a taxane-containing agent for at least six weeks, in a subject suffering from a HR+ and HER2- breast cancer comprising the steps of: obtaining expression level data of three or more markers selected from any one of Tables 1.1, 1.2, or 3-8, preferably of Table 1.2 or 7.1, most preferably of Table 7.1, in a tumor tissue sample obtained from said subject, processing the expression level data of each of the three or more markers using a statistical model, wherein the processing comprises mathematically combining the determined expression levels of the three or more markers selected from any one of Tables 1.1, 1.2, or 3-8, preferably of Table 1.2 or 7.1, most preferably of Table 7.1, to generate a predictive score and optionally comprises normalizing the determined expression levels of said three or more markers prior to mathematically combining them, and predicting the response or resistance to and/or benefit from said subject to said systemic treatment, based on the predictive score. Also envisaged herein is a computer-implemented method for predicting the response or resistance to and/or benefit from a systemic treatment, preferably a neoadjuvant or adjuvant treatment or a chemotherapy, more preferably a neoadjuvant treatment, most preferably a neoadjuvant chemotherapy, optionally comprising a taxane-containing agent for at least six weeks, in a subject suffering from a HR+ and HER2- breast cancer comprising the steps of: obtaining expression level data of three or more markers selected from any one of Tables 1.1, 1.2, or 3-8, preferably of Table 1.2 or 7.1, most preferably of Table 7.1, in a tumor tissue sample obtained from subject and expression level data of one or more additional biomarkers in said sample, one or more clinical parameters of said subject and/or one or more non-clinical parameters of said subject, processing the expression level data of each of the three or more markers and the expression level data of the one or more additional biomarkers, one or more the clinical parameters and/or the one or more non-clinical parameters using a statistical model, wherein the processing comprises mathematically combining the determined expression level data of each of the three or more markers and the expression level data of the additional biomarkers, the clinical parameters and/or non-clinical parameters to generate a predictive score and optionally comprises normalizing the expression level data of each of the three or more markers and the expression level data of the additional biomarkers, the clinical parameters and/or non-clinical parameters prior to mathematically combining them, and predicting the response or resistance to and/or benefit from said systemic treatment, in said subject based on the predictive score. Further provided herein is a data processing system comprising means for carrying out the steps of the computer-implemented method, a computer program product comprising instructions which, when the program is executed by a computer, cause the computer and/or the data processing system carry out the steps of the computer-implemented method and a computer-readable storage medium comprising instructions which, when executed by a computer, cause the computer to carry out of the computer-implemented method and having stored thereon said computer program. The processing may be performed by statistical and/or mathematical model, wherein the statistical and/or mathematical model is one or more of weighted or non-weighted average, linear regression, logistic regression, Cox regression, non-linear regression, regression trees, any other regression, principal component analysis, ANOVA, committee voting, supervised clustering, unsupervised clustering, nearest neighbor clustering, hierarchical clustering, any other clustering, and other machine learning algorithms.

### DETAILED DEFINITIONS

For the purposes of the present invention, the "subject" (or "patient") may be a mammal. In the context of the present invention, the term "subject" includes both humans and other mammals. Thus, the herein provided methods are applicable to both human and animal subjects, i.e., the method can be used for medical and veterinary purposes. Accordingly, said subject may be an animal such as a mouse, rat, hamster, rabbit, guinea pig, ferret, cat, dog, sheep, bovine species, horse, camel, or primate. Most preferably herein the subject is human. However, the diagnosis and treatment of canine, feline and equine mammals is of further particular interest. The subject may in principle be female and male, female may be pre- or perimenopausal. In the context of breast cancer, female subjects are more often diseased than male subjects. Hence, in the most prevalent aspect, the subject is a human female. In one embodiment, the subject may be a subject fulfilling the inclusion criteria of the PENELOPE^{B} study (see PENELOPE-B: Clinical study, NCT01864746, https://www.clinicaltrials.gov/study/NCT01864746 for details). The subject may have an estimated life expectancy of at least 5 years irrespective of the diagnosis of breast cancer.

In the context of the present invention the response, resistance and/or benefit is typically determined in terms of the invasive disease-free survival (iDFS), distant-disease-free survival (DDFS), pathological complete response (pCR), loco-regional recurrence free interval (LRRFI), loco-regional invasive recurrence free interval (LRIRFI), disease free survival (DFS), event free survival (EFS) and/or overall survival (OS). The most typical endpoint in the context of the present invention is iDFS.

As used herein, the terms "predicting an outcome" and "prediction of an outcome" of a disease are used interchangeably and refer to a prediction of an outcome of a subject undergoing a given systemic treatment, e.g., a neoadjuvant or adjuvant treatment, preferably a neoadjuvant treatment, preferably a chemotherapy, most preferably a neoadjuvant chemotherapy, optionally comprising a taxane-containing agent for at least six weeks. The terms "predicting an outcome" and "prediction of an outcome" may, in particular, relate to an individual assessment of the malignancy of a tumor, or to the expected survival rate (OS, overall survival; DFS, disease-free survival or iDFS, invasive disease-free survival) of a subject, if the tumor is treated with a given systemic therapy, e.g., the neoadjuvant chemotherapy.

As used herein, the term "predicting the response to a systemic treatment" refers to the act of determining a likely response or resistance and/or benefit of the systemic treatment in a subject suffering from or being at risk of developing HR+ and HER2- breast cancer. The treatment may be neoadjuvant, systemic treatment or adjuvant, systemic treatment. In a preferred embodiment, the treatment is neoadjuvant treatment, more preferably neoadjuvant chemotherapy, most preferably neoadjuvant chemotherapy comprising a taxane-containing agent. The predictive methods of the invention are not particularly limited and any suitable statistical model and/or reference may be used. For example, the prediction of a response or resistance to and/or benefit from a systemic treatment can be made in comparison with subjects suffering from HR+ and HER2- breast cancer for whom the outcome is known. The prediction of a response or resistance to and/or benefit from a systemic treatment can also be made by mathematically combining the determined expression levels of three or more markers. Or the prediction of a response or resistance to and/or benefit from a systemic treatment can be made in comparison with healthy subjects. The predictive methods of the present invention can be used clinically to make treatment decisions by choosing the most appropriate treatment modalities for the subject.

As used herein, the term "predicting a resistance to a systemic treatment" "predicting a resistance to a neoadjuvant chemotherapy" relate to a prediction of a resistance of a patient undergoing a given systemic therapy, e.g., the neoadjuvant chemotherapy. The terms "predicting a resistance to the systemic treatment" and "predicting a resistance to the neoadjuvant chemotherapy" may, in particular, relate to a non-response and/or a non-benefit in said subject by individual assessment of the malignancy of a tumor, or to the expected survival rate (OS, overall survival or iDFS, invasive disease-free survival) of a patient, if the tumor is treated with a given therapy, e.g., the neoadjuvant chemotherapy.

The term "prognosis" or "prognosticate" relates to an individual assessment of the malignancy of a tumor or disease outcome of a patient treated with a given systemic therapy. A good prognosis indicates that the subject is likely to respond to and/or benefit from the systemic treatment. Thus, the subject has a high expected survival rate (OS, overall survival or iDFS, invasive disease-free survival). A bad prognosis indicates that the subject is likely not responding to, resistant to and/or not benefiting from the systemic treatment. Thus, the subject has a short, expected survival rate (OS, overall survival or iDFS, invasive disease-free survival).

As used herein, the term "response" refers to any response to the systemic treatment, i.e., to the neoadjuvant or adjuvant treatment, preferably to the chemotherapy, more preferably to the neoadjuvant treatment, most preferably to the neoadjuvant chemotherapy, optionally comprising a taxane-containing agent for at least six weeks. Non-limiting examples commonly used in oncology to evaluate the response of the subject to a therapy may be a change in tumor mass and/or volume and/or prolongation of time to distant metastasis or time to death following treatment. As used herein, "benefit" from a given systemic therapy is an improvement in health or wellbeing that can be observed in patients under said therapy, but it is not observed in patients not receiving this therapy. Non-limiting examples commonly used in oncology to gauge a benefit from therapy are survival, overall survival, disease free survival, invasive disease-free survival, metastasis free survival, disappearance of metastasis, tumor regression, and tumor remission. Vice versa, the term "resistance" as used herein refers to any non-response and or non-benefit to the neoadjuvant treatment. Non-limiting examples commonly used in oncology to evaluate the resistance of the subject to a therapy may be a change in tumor mass and/or volume and/or shorter time to distant metastasis or time to death following treatment.

The benefit from and/or response or resistance to a systemic treatment may be assessed in a neoadjuvant situation where the size of a tumor after systemic intervention can be compared to the initial size and dimensions as measured by CT, **PET,** mammogram, ultrasound or palpation, usually recorded as "clinical response" of a patient. Response or resistance and/or benefit may also be assessed by caliper measurement or pathological examination of the tumor after biopsy or surgical resection. Response or resistance and/or benefit may be recorded in a quantitative fashion like percentage change in tumor volume or in a qualitative fashion like "no change" (NC), "partial remission" (PR), "complete remission" (CR) or other qualitative criteria. Assessment of tumor response or resistance and/or benefit may be done early after the onset of neoadjuvant therapy e.g., after a few hours, days, weeks or preferably after a few months. A typical endpoint for response or resistance and/or benefit assessment is upon termination of neoadjuvant chemotherapy or upon surgical removal of residual tumor cells and/or the tumor bed. Response or resistance and/or benefit may also be assessed by comparing time to distant metastasis or death of a patient following neoadjuvant or adjuvant non-chemotherapy and/or chemotherapy with time to distant metastasis or death of a patient not treated with non-chemotherapy and/or chemotherapy.

As used herein, the term "clinical response" is well understood by the person skilled in the art and may include clinical response with levels of complete response, partial response, stable disease, progressive disease.

As used herein, the terms "pCR", "pathological complete remission" and "pathological complete response" are used interchangeably and are well understood by the person skilled in the art. As used herein, the term pCR refers to a complete disappearance or absence of invasive tumor cells in the breast and/or lymph nodes as assessed by a histopathological examination. In particular, the term "pCR" may refer to ypT0 and ypN0, or ypT0, or ypTis and ypN0. In one embodiment, the response and/or benefit and/or outcome may be the pCR.

As used herein, the term "outcome" refers to a condition attained in the course of a disease. This disease outcome may e.g., be a clinical condition such as "recurrence of disease", "development of metastasis", "development of nodal metastasis", "development of distant metastasis", "survival", "death", "tumor remission rate", a disease stage or grade or the like. In one embodiment, the outcome is the pathological complete response (pCR), loco-regional recurrence free interval (LRRFI), loco-regional invasive recurrence free interval (LRIRFI), distant-disease-free survival (DDFS), invasive disease-free survival (iDFS), event free survival (EFS) and/or overall survival (OS).

As used herein, the terms "treatment", "treat", "treating" and grammatical variations thereof refer to subjecting an individual subject to a protocol, regimen, process or remedy, in which it is desired to obtain a physiologic response or outcome in that subject, e.g., a patient. In particular, the methods and compositions of the present invention may be used to slow the development of disease symptoms or delay the onset of the disease or condition, or halt the progression of disease development. However, because every treated subject may not respond to a particular treatment protocol, regimen, process or remedy, treating does not require that the desired physiologic response or outcome be achieved in each and every subject or subject population, e.g., patient population. Accordingly, a given subject or subject population, e.g., patient population may fail to respond or respond inadequately to treatment. In particular, "treatment" as used herein refers to the treatments of the present invention, i.e., neoadjuvant or adjuvant treatment, preferably neoadjuvant treatment, preferably chemotherapy, most preferably neoadjuvant chemotherapy. However, "treatment" may also refer to further other systemic therapies and/or components, such as non-chemotherapeutic agents, endocrine therapy, immunotherapy, targeted therapy, and/or anti-angiogenic therapy and/or to non-systemic treatments, including radiation therapy and surgery.

As used herein, "systemic treatment" refers to a treatment using drug substances that work throughout the whole body, i.e., that travel through the bloodstream, reaching and affecting cells all over the body. Non-limiting examples of systemic therapy include chemotherapy, endocrine therapy, targeted therapy, or immunotherapy.

As used herein, "chemotherapy" is a systemic treatment that uses drugs to stop the growth of cancer cells, either by killing the cells or by stopping them from dividing. The chemotherapy may be neoadjuvant or adjuvant chemotherapy. The chemotherapeutic drugs are not particularly limited and may be, inter alia, taxane-containing agents, anthracyclines, or platinum-based chemotherapeutic drugs.

As used herein, a "taxane-containing" agent is a chemotherapeutic drug comprising taxane. A "taxane-containing (neoadjuvant) chemotherapy" is a (neoadjuvant) chemotherapy during which a taxane or a taxane-containing drug is administered. Taxanes are widely-used and divers chemotherapeutic agents for treating various solid malignancies and work as microtubule inhibitors. The resulting cell cycle inhibition during the G2/M phase activates the cellular apoptosis pathways. They form the backbone of chemotherapeutic regimens in breast, ovarian, non-small cell lung, and head and neck cancers. Non-limiting examples of taxanes include paclitaxel, docetaxel, abraxane, cabazitaxel, and albumin-bound paclitaxel (nab-paclitaxel).

As used herein, a "platinum-based chemotherapeutic drug" is a chemotherapeutic drug comprising platinum ion compounds. Non limiting examples of platinum-based chemotherapeutic drugs include cisplatin, carboplatin, and oxaliplatin.

Neoadjuvant treatment refers to medicines that are administered before surgery. Adjuvant (meaning "in addition to") treatment refers to medicines administered after surgery. The term "adjuvant" relates to a postoperative systemic therapy regimen consisting of a panel of hormonal, chemotherapeutic, small molecules and/or antibody agents, which is aimed to eradicate micrometastasis (tumor cells spread throughout the body), thereby preventing from recurrence and improving survival. The neoadjuvant treatment in the context of the present invention may e.g., be one or more of chemotherapy, endocrine therapy, immunotherapy, targeted therapy, wherein the targeted therapy may comprise small- molecules and/or antibodies, anti-angiogenic therapy, biological therapy, and/or gene therapy. Preferably, the neoadjuvant treatment is neoadjuvant chemotherapy. The adjuvant treatment is any one of chemotherapy, endocrine therapy, immunotherapy, targeted therapy, wherein the targeted therapy may comprise small- molecules and/or antibodies, anti-angiogenic therapy, biological therapy, and/or gene therapy, preferably any one of adjuvant chemotherapy, adjuvant endocrine therapy, adjuvant immunotherapy, adjuvant targeted therapy or adjuvant anti-angiogenic therapy. As used herein, a subject receiving neoadjuvant chemotherapy may receive the treatment for at least 16 weeks, preferably the subject receives a taxane-containing agent for at least six weeks during the neoadjuvant chemotherapy. A subject receiving neoadjuvant chemotherapy according to the present invention may also receive neoadjuvant chemotherapy for less than 16 weeks if the subject has a progressive disease that that occurred after at least six weeks of taxane-containing neoadjuvant treatment.

"Targeted therapy" as used herein refers to a type of systemic treatment that uses drugs or other substances to target specific molecules that cancer cells need to survive and spread. Targeted therapies work in different ways to treat cancer. Some stop cancer cells from growing by interrupting signals that cause them to grow and divide, stopping signals that help form blood vessels, delivering cell-killing substances to cancer cells, or starving cancer cells of hormones they need to grow. Other targeted therapies help the immune system kill cancer cells or directly cause cancer cell death. Most targeted therapies are either small-molecule drugs, monoclonal antibodies or bi-specific antibodies. Targeted therapy used for treating breast cancer includes, but is not limited to, treatment using abemaciclib; ado-trastuzumab emtansine; alpelisib; anastrozole; capivasertib; elacestrant dihydrochloride; everolimus; exemestane; fam-trastuzumab deruxtecan-nxki; fulvestrant; goserelin acetate; lapatinib ditosylate; letrozole; margetuximab-cmkb; neratinib maleate; olaparib; palbociclib;; pembrolizumab; pertuzumab; pertuzumab, trastuzumab, and hyaluronidase-zzxf; ribociclib; sacituzumab govitecan-hziy; talazoparib tosylate; tamoxifen citrate; toremifene; trastuzumab; or tucatinib.

As used herein "endocrine therapy" and "hormone therapy" are used interchangeably. Endocrine therapy refers to a systemic treatment that adds, inhibits, or removes hormones. Hormones can cause certain cancers (such as prostate and breast cancer) to grow. Endocrine therapy may be administered as neoadjuvant or adjuvant treatment. To slow or stop the growth of cancer, synthetic hormones or other drugs may be given to block the body's natural hormones, or surgery is used to remove the gland that makes a certain hormone. Breast cancers sensitive to hormone therapy include HR-positive, i.e., ER+ and PR+ breast cancers.

Neoadjuvant endocrine therapy of breast cancer includes, but is not limited to, treatment with aromatase inhibitors, such as exemestane, anastrozole and letrozole. Adjuvant therapy for early-stage breast cancer includes, without limitation, one or a combination of tamoxifen or aromatase inhibitors, optionally in combination with ovarian suppression. Endocrine therapy of advanced or metastatic breast cancer includes, but is not limited to, treatment with antiestrogens, such as tamoxifen, toremifene or fulvestrant, and/or aromatase inhibitors, such as exemestane, anastrozole and letrozole. Advanced breast cancer may also be treated with a combination of hormone therapy and one of several targeted therapies, for example letrozole or fulvestrant in combination with Palbociclib, an inhibitor of the cyclin-dependent kinases 4 and 6 (CDK4 and CDK6).

"Immunotherapy" as used herein refers to a systemic treatment designed to elicit or amplify an immune response to eradicate a cancer, i.e., a breast cancer. Immunotherapies include, inter alia, cytokines, cancer treatment vaccines, antibodies, or cell-based immunotherapies, such as CAR T Cell therapy. Preferred antibodies include monoclonal antibodies and bispecific antibodies.

"Anti-angiogenic therapy" as used herein refers to treatments that inhibits the growths of blood vessels in tumors. If the drug is able to prevent a cancer from growing blood vessels, it might slow the growth of the cancer or sometimes shrink it.

Non limiting examples of CDK4/6 inhibitors include palbociclib (Ibrance^{®}), ribociclib (Kisqali^{®}), abemaciclib (Verzenios^{®}) and Trilaciclib.

A "biological therapy" as used herein is a type of treatment that uses substances made from living organisms to treat disease. These substances may occur naturally in the body or may be made in the laboratory. In cancer, some biological therapies stimulate or suppress the immune system to help the body fight cancer. Other biological therapies attack specific cancer cells, which may help keep them from growing or kill them. "Gene therapy" as used herein refers to an experimental treatment that adds a new gene or replaces or repairs a mutated (changed) gene inside the body's cells to help prevent or treat certain diseases, such as cancer. Gene therapy may also be used to train the body's immune system to recognize and attack cancer cells or to protect healthy cells from the effects of cancer treatment.

As mentioned above, a subject suffering from HR+ and HER2- breast cancer may receive non-systemic treatment in addition to a systemic treatment. Non-limiting examples of non-systemic treatment include radiation therapy and surgery. As used herein a "non-systemic treatment" is a treatment that acts locally in a subject. As used herein "radiation therapy" refers to the use of high-energy radiation from x-rays, gamma rays, neutrons, protons, and other sources to kill cancer cells and shrink tumors. Radiation may come from a machine outside the body (external-beam radiation therapy), or it may come from radioactive material placed in the body near cancer cells (internal radiation therapy or brachytherapy). Systemic radiation therapy uses a radioactive substance, such as a radiolabeled monoclonal antibody, that travels in the blood to tissues throughout the body. Also called irradiation and radiotherapy.

As used herein, the term "disease" is defined as a deviation from the normal structure or function of any part, organ or system of the body (or any combination thereof). A specific disease is manifested by characteristic symptoms and signs, including both chemical and physical changes. Certain characteristic signs, symptoms, and related factors of the disease can be quantitated through a variety of methods to yield important diagnostic information. For example, the neoplastic disease may be a tumor or cancer. As used herein, the term "tumor" refers to all neoplastic cell growth and proliferation, whether malignant or benign, and all pre-cancerous and cancerous cells and tissues. As used herein, the term "cancer" refers to uncontrolled cellular growth, and is not limited to any stage, grade, histomorphological feature, invasiveness, aggressivity, or malignancy of an affected tissue or cell aggregation. For example, stage 0 breast cancer, stage I breast cancer, stage II breast cancer, stage III breast cancer, stage IV breast cancer, grade I breast cancer, grade II breast cancer, grade III breast cancer, malignant breast cancer, primary carcinomas of the breast, and all other types of cancers, malignancies and transformations associated with the breast are included. As used herein, the term "neoplastic lesion" or "neoplastic disease" or "neoplasia" refers to a cancerous tissue this includes carcinomas, (e.g., carcinoma in situ, invasive carcinoma, metastatic carcinoma) and pre-malignant conditions, neomorphic changes independent of their histological" origin (e.g., ductal, lobular, medullary, mixed origin).

As mentioned herein above, the neoplastic disease is a HR+ and HER2- breast cancer. Along with classification of histological type and grade, breast cancers are routinely evaluated for expression of hormone receptors (estrogen receptor (ER) and progesterone receptor (PR)) and for expression of HER2 (ErbB2). ER and PR are both nuclear receptors (they are predominantly located at cell nuclei, although they can also be found at the cell membrane). HER2, or human epidermal growth factor receptor type 2, is a receptor normally located on the cell surface. As used herein, the term "HR+ and HER2- breast cancers" refers to tumors (e.g., carcinomas), in which the cancer cells/tumor cells score positive (i.e., using conventional histopathological methods or in-situ hybridization methods, in particular fluorescence in-situ hybridization (FISH)), for estrogen receptor (ER) and/or progesterone receptor (PR), i.e., ≥1% ER and/or PR positive stained cells and the tumor cells are not amplified for epidermal growth factor receptor type 2 (HER2 or ErbB2), i.e., IHC score 0-1 or FISH negative (in-situ hybridization (ISH) ratio) <2.0 status.

The hormone receptor-positive (HR+) and human epidermal growth factor receptor 2-negative (HER2-) breast cancer can be an early, non-metastatic breast cancer or a recurrent and/or metastatic breast cancer. The breast cancer may be unilateral or bilateral and should be assessable for resection or biopsy. As used herein, the term "recurrent" refers in particular to the occurrence of metastasis. Such metastasis may be distal metastasis that can appear after the initial diagnosis, even after many years, and therapy of a tumor, to local events such as infiltration of tumor cells into regional lymph nodes, or occurrence of tumor cells at the same site and organ of origin. The terms "early" or "early-stage" breast cancer as used herein primarily refer to non-metastatic diseases, in particular cancer. In one embodiment, the neoplastic disease is a non-metastatic disease. In one embodiment, the breast cancer of the present invention is a non-metastatic disease. This includes, for example, a breast cancer that has not spread beyond the breast or the axillary lymph nodes. This includes ductal carcinoma in situ and stage I, stage IIA, stage IIB, and stage IIIA breast cancers. In contrast, a late-stage breast cancer is metastasized or T4.

In some embodiments, the subject may suffer from HR+ and HER2- breast cancer with increased risk, optionally an early-stage HR+ and HER2- breast cancer. As used herein, "increased risk" refers to additional risk factors of the subject, such as age, clinical nodal status (cN) positive and/or high cN, high clinical tumor size (cT), high grading, high expression of Ki-67 or of any other proliferation marker, low tumor infiltrating lymphocytes (TILs), and/or a high clinical and pathologic stage and estrogen receptor status (CPS-EG) score. Increased risk also relates to an increased risk of recurrence. cN positive indicates that the cancer has spread to lymph nodes before start of the treatment. As used herein, "high grading" relates to a cancer grade 3 or 4. In some embodiments, the subject has cancer grade 3 or grade 4 before start of the treatment. An increased risk may also refer to a high risk of an iDFS event.

As used herein, the terms "sample" or "biological sample" as are used interchangeably and refer to a sample obtained from the subject. The sample may be of any biological tissue or fluid suitable for carrying out the method of the present invention, i.e., for assessing whether a subject suffering from a HR+ and HER2- breast cancer, optionally early-stage, HR+ and HER2-breast cancer, will respond or be resistant to and/or benefit from the systemic treatment, e.g., systemic neoadjuvant or adjuvant treatment, preferably a systemic neoadjuvant or systemic adjuvant chemotherapy or a systemic neoadjuvant treatment, more preferably a systemic neoadjuvant chemotherapy, optionally comprising a taxane-containing agent, and/or for assessing the outcome of said patient to said systemic treatment. However, typically, once the subject's is determined to have a response and/or benefit and/or good outcome with said systemic treatment according to the methods of the present invention, the subject will receive the said systemic treatment as soon as possible.

In particular, the sample may be obtained from any tissue and/or fluid of a subject suffering from a HR+ and HER2- breast cancer, optionally early-stage, HR+ and HER2- breast cancer. Preferably, the tissue and/or fluid of the sample may be taken from any material of the breast cancer and/or from any material associated with the breast cancer. Such a sample may, for example, comprise cells obtained from the subject. In one embodiment, the sample may be a tumor sample. A "tumor sample" is a biological sample containing tumor cells, whether intact or degraded. In one embodiment, the sample is a tumor sample obtained from said subject. The sample may also be a bodily fluid. Such fluids may include the lymph. In one embodiment, the sample is a lymph node sample obtained from said subject. In another embodiment, the sample is a tumor sample, or a lymph node sample obtained from said subject.

The sample may also include sections of tissues. Such sections of tissues also encompass frozen or fixed sections. These frozen or fixed sections may be used, e.g., for histological purposes. In one embodiment, the sample from said subject is a formalin-fixed paraffin embedded (FFPE) sample or a fresh-frozen sample. A sample to be analyzed may be taken by aspiration or punctuation, excision or by any other surgical method leading to biopsy or resected cellular material. The sample may be from a core-biopsy. The sample may be taken before start of the systemic treatment, preferably the sample is taken before start of the neoadjuvant treatment, more preferably the sample is taken before start of the neoadjuvant chemotherapy. In particular, the tumor sample is tumor tissue sample, particularly wherein the sample is a primary tumor tissue sample, particularly a core biopsy sample, more particularly a core biopsy sample from a primary tumor before any treatment.

As used herein, the terms "marker" and "biomarker" are used interchangeably. As used herein, the term "expression level of the three or more markers" refers to the quantity of the molecular entity of the marker in a sample that is obtained from the subject. In other words, the concentration of the marker is determined in the sample. It is also envisaged that a fragment of the marker can be detected and quantified. Thus, it is apparent to the person skilled in the art, in order to determine the expression of a marker, parts and fragments of said marker can be used instead. Suitable method to determine the expression level of the three or more markers are described herein below in detail. As used herein, the term "marker" relates to measurable and quantifiable biological markers which serve as indices for health- and physiology-related assessments, such as a disease/disorder/clinical condition risk. Furthermore, a marker is defined as a characteristic that is objectively measured and evaluated as an indicator of normal biological processes, pathogenic processes, or pharmacologic responses to a therapeutic intervention. As discussed herein above a biomarker may be measured on a biological sample (e.g., as a tissue test). A marker may be a nucleic acid, a protein, or another molecule. In principle, a single marker or a combination of two markers is sufficient for the methods of the present disclosure. Preferably, the expression level of three or more markers selected from any one of Tables 1.1, 1.2, or 3-8 is determined to predict the response or resistance to and/or benefit from a systemic treatment in a subject suffering from a hormone receptor-positive (HR+) and human epidermal growth factor receptor 2-negative (HER2-) breast cancer, wherein the expression levels of the three or more markers are indicative for predicting the response or resistance to and/or benefit from the systemic treatment in said subject. However, in principle, determination of the expression level of one marker is sufficient for carrying out the methods of the disclosure. In some embodiments, the expression levels of three or more markers selected from any one of Tables 1.1, 1.2, or 3-8, preferably of Table 1.2 or 7.1, more preferably of Table 7.1, are and one or more additional markers, such as proliferation markers, known tumor markers and/or housekeeping markers, are determined in a sample obtained in a subject suffering from a HR+ and HER2- breast cancer to predict the response or resistance to and/or benefit from a systemic treatment in said subject. In a preferred embodiment, the expression levels of all markers selected from any one of 1.1, 1.2, or 3-8, preferably of Table 1.2 or 7.1, more preferably of Table 7.1, are determined for carrying out the methods of the disclosure.

A predictive marker relates to a marker which can be used to predict the response or resistance and/or benefit of the subject towards a given systemic treatment, e.g., a neoadjuvant or adjuvant treatment, optionally a neoadjuvant chemotherapy. A predictive marker can either be predictive for a good prognosis or for a bad prognosis. Predictive markers which are indicative of a good prognosis include, *inter alia,* the markers of Table 6, i.e., ADIPOR1, BMI1, CDK5R1, CDKN2C, DESI1, DNAJC14, DUSP8, DVL1, GGH, GLUL, GNGT2, GPI, GPR160, GPSM2, HMGB3, IKBKG, IL18, LIG4, MADD, MAFF, MST1, MTDH, MYB, MYCN, NCAPD3, NF2, NME1, NOL3, NR1H3, NR4A1, NRAS, NRIP1, NSD1, NUF2, NUP62, OCLN, OGG1, PFKL, PIAS4, PIK3R2, PIM2, PITRM1, PKM, PKMYT1, PLAU, PLCG1, PLK4, PMAIP1, POLR2D, PLPP1, PLPP3, PPID, PRC1, PRDX6, PRKCZ, RGS2, RIPK2, RPS4X, SAP30, SFN, SPHK1, SRC, STK11, STK36, TAP1, and TBP. In particular, high expression of OGG1, DNAJC14, POLR2D, LIG4, NME1 or NTHL1 indicates a good prognosis and/or a good response to neoadjuvant chemotherapy. Predictive markers which are indicative of a bad prognosis include, inter alia, the markers of Table 8, i.e., APOC2, CXCL16, CXCR4, OTULINL, GSN, GSTP1, H2AX, HERC3, HES1, H3C10, HMGCS2, JMJD1C, MKI67, SOCS3, TCF4, TCF7L1, TEK, TESC, TGFB3, TGFBR2, TIPARP, TMEM132A, TMPRSS2, TPX2, and TXNIP.

The markers or predictive markers of the present invention can be grouped according to their ontology: chromosomal location, biological function, molecular pathway, involvement in a biological process, regulation of certain cellular processes, or some other common properties. Some markers can be associated with DNA repair, stress response, IFN response, estrogen signaling, proliferation, cell cycle, immune response, including inflammatory response and immune-oncology, endocrine pathways, epidermal growth factor/platelet derived growth factor (EGF/PDGF) signaling, stromal and/or endothelial cells, or IL6-Jak-Stat signaling. The person skilled in the art knows which markers, e.g., the markers of Table 1.1, are associated with which group.

For example, the group of markers which can be associated with DNA repair mechanisms comprises CDK5R1, DNAJC14, DUSP8, GNGT2, IL18, LIG4, MADD, MTDH, NR1H3, NRAS, NSD1, NUP62, OGG1, PFKL, PIM2, PITRM1, PLK4, POLR2D, PRDX6, RIPK2, RPS4X, SAP30, STK36, and TBP (*cf.* Tables 7.1-7.3). In some embodiments, enrichment of markers associated with DNA repair in a tumor sample obtained before the initiation of systemic treatment, in particular before the initiation of neoadjuvant chemotherapy, is indicative of a good response and/or a good prognosis. In contrast, in some embodiments, enrichment of markers associated with DNA repair in a tumor sample obtained after the systemic treatment, in particular after neoadjuvant chemotherapy, is indicative of a bad response and/or a poor prognosis. "DNA repair" as used herein refers to cellular processes by which a cell identifies and corrects damages in the genome encoding DNA molecules.

Some of the markers may be differentially expressed before and after systemic treatment. Hence, the markers may also be grouped according to the prognosis their expression indicated before and after systemic treatment, i.e., enrichment of the markers in pre- and post-treatment samples indicates a good prognosis, enrichment of the markers pre- and post-treatment samples indicates a bad prognosis, enrichment of the markers in pre-treatment samples indicates good prognosis and enrichment of the markers in post-treatment samples indicates bad prognosis, or enrichment of the markers in pre-treatment samples indicates bad prognosis and enrichment of the markers in post-treatment samples indicates good prognosis.

As mentioned above, additionally to the expression level of three or more markers selected from any one of Tables 1.1, 1.2, or 3-8, the expression level of additional markers may be determined. These additional markers include, but are not limited to, known tumor markers, proliferation markers or housekeeping genes. Proliferation makers are, for example, genes or proteins which are known to be involved in proliferation. "Housekeeping genes" or "housekeeping markers" as used herein, are genes that are consistently expressed across tissues, essential, carrying out cellular maintenance, and conserved across species.

As used herein, a marker or a gene that is enriched may be identified by gene set enrichment analysis (GSEA), which is a computational method that determines whether an a priori defined set of genes shows statistically significant, concordant differences between two phenotypes. Enrichment of a marker means that this marker is over-represented in a large set of genes or proteins and may have an association with different phenotypes.

The higher the expression of a good prognosis marker, the lower is the risk of an iDFS event. The lower the expression of a good prognosis marker the higher is the risk of an iDFS event. Vice versa, for bad prognosis markers a higher expression of a bad prognosis marker indicates a higher risk of an iDFS event, while a lower expression of a bad prognosis marker indicates a lower risk of an iDFS event. The risk of an iDFS event may further depend on the combination of markers (i.e., statistical and/or mathematical model used for the combination, number of markers and/or compilation of markers) for which the expression levels are determined and/or on additional clinical and/or non-clinical parameters. A subject with relatively high expression of one bad prognosis marker may still have an overall good prognosis if certain other markers, e.g., at least two good prognosis markers, are also expressed on a relatively high level. A subject with a higher expression level of a bad prognosis marker compared to the expression level of said marker in another subject may have a higher risk of an iDFS event. However, the risk of an iDFS event may still be low overall. Or a subject with a higher expression level of a good prognosis marker compared to the expression level of said marker in another subject may have a lower risk of an iDFS event. The risk for an iDFS event may also depend on the time which has passed since the systemic treatment. Hence, the risk of an iDFS event may increase over time.

As used herein, "high expression of three or more markers" or "increased expression of three or more markers" may be a relative term indicating that the expression of the three or more markers is increased in a sample compared to the expression of said three or more markers in another sample, wherein the expression level of each of the three or more markers is assessed and compared individually. An increased expression of a marker may be a marker whose gene expression is upregulated and/or enriched. The expression level of a marker in a tumor sample obtained from a subject suffering from HR+ and HER2- breast cancer (i.e., a breast cancer sample) can be compared to a reference value, i.e., the expression level of said marker in a sample obtained from a healthy individual, to the mean expression level of said marker in a plurality of samples obtained from a population of healthy subjects, or compared to the expression level of said marker in a sample obtained from a non-cancerous section of said subject in order to determine whether the marker has a high expression in the sample of interest. It is also possible to compare a pre- and a post-treatment sample of the same subject in order to determine whether a marker has an increased expression before or after systemic treatment. A low expression or a decreased expression of a marker may be determined accordingly. The reference value may be lower or higher than the expression level of the three or more markers. For example, the reference value of a marker may be 2-fold lower or 2-fold higher than the expression level of the marker. The difference between the expression level of the three or more markers compared to their respective reference values may alternatively be determined by absolute values, e.g., by the difference of the expression level of the three or more markers and their respective reference values, or by relative values, e.g., by the percentage increase or decrease of the expression level of the three or more markers compared to their respective reference values. The expression level of the at least one marker which deviates from the reference value may be indicative for a particular response and/or benefit and/or outcome of the systemic treatment in a subject suffering from a HR+ and HER2-breast cancer. In other words, an upregulation or a downregulation of the expression level of the three or more markers compared to their respective reference values may be indicative for a response and/or benefit and/or good outcome from a systemic treatment in said subject. In particular, the extent of upregulation of the expression level of the three or more good prognosis markers compared to their respective reference values may be indicative for a particular response to and/or benefit from and/or outcome of the systemic treatment in a subject suffering from HR+ and HER2- breast cancer. For example, the expression level of the three or more good prognosis markers above by 3-fold rather than above 2-fold compared to their respective reference values may be indicative with a higher likelihood for a response and/or benefit from the systemic treatment in said subject. Or the expression level of the three or more bad prognosis markers above by 3-fold rather than above 2-fold compared to their respective reference values may be indicative with a higher likelihood for a resistance to and/or no benefit from the systemic treatment in said subject.

The determination of expression levels in a sample may also be referred to as "molecular profiling". Molecular profiling is laboratory method that uses a sample of tissue, blood, or other body fluid to check for certain genes, proteins, or other molecules that may be a sign of a disease or condition, such as cancer. Molecular profiling can also be used to check for certain changes in a gene or chromosome that may increase a person's risk of developing cancer or other diseases. Molecular profiling may be performed in combination with other procedures, such as biopsies, to help diagnose some types of cancer. It may also be used to help plan treatment, find out how well systemic treatment is working, make a prognosis, or predict whether cancer will come back or spread to other parts of the body. Also called biomarker testing and molecular testing. Hence, in one embodiment, the present invention provides new markers, i.e., the markers in Table 1.1, which may be used for molecular profiling of samples obtained from subjects suffering from HR+ and HER2- breast cancer.

The gene names as used in the context of the present invention refer to gene names according to the official gene symbols provided by the HGNC (HUGO Gene Nomenclature Committee) and as used by the NCBI (National Center for Biotechnology Information).

When referring to markers of the present invention as identified by the gene names mentioned herein above, the person skilled in the art how to derive the respective RNA, in particular the mRNA, or the protein of the marker identified by its gene name.

In one embodiment, the expression level of the three or more markers is the protein expression level or the RNA expression level, preferably mRNA expression level. For example, the expression level refers to a determined level of gene expression. A "gene" is a set of segments of nucleic acid that contains the information necessary to produce a functional RNA product. A "gene product" is a biological molecule produced through transcription or expression of a gene, e.g., an mRNA, cDNA or the translated protein. An "mRNA" is the transcribed product of a gene and shall have the ordinary meaning understood by a person skilled in the art. A "molecule derived from an mRNA" is a molecule which is chemically or enzymatically obtained from an mRNA template, such as cDNA.

The expression level of a marker may be a determined level of protein, RNA, or mRNA expression as an absolute value or compared to a reference gene, to the average of two or more reference value, or to a computed average expression value or to another informative protein, RNA or mRNA without the use of a reference sample.

In some embodiments, the expression level is the RNA expression level, preferably mRNA expression level, and is determined by at least one of a hybridization-based method, a PCR based method, a microarray-based method, a quantitative transcriptomic analysis, a sequencing and/or next generation sequencing method hybridization-based method, a PCR based method, a microarray-based method, a quantitative transcriptomic analysis, a sequencing and/or next generation sequencing method.

As used herein, the term "hybridization-based method" refers to a method, wherein complementary, single-stranded nucleic acids or nucleotide analogues may be combined into a single double stranded molecule. Nucleotides or nucleotide analogues will bind to their complement under normal conditions, so two complementary strands will bind to each other. In bioanalytics, very often labelled, single stranded probes are in order to find complementary target sequences. If such sequences exist in the sample, the probes will hybridize to said sequences which can then be detected due to the label. Other hybridization-based methods comprise microarray and/or biochip methods. For example, probes may be immobilized on a solid phase, which is then exposed to a sample. If complementary nucleic acids exist in the sample, these will hybridize to the probes and can thus be detected. These approaches are also known as "array-based methods". Examples of microarray-based methods include without limitation the Affymetrix GeneChip technology, Agilent or Illumina microarray technology. Yet another hybridization-based method is PCR, which is described below. When it comes to the determination of expression levels, hybridization-based methods may for example be used to determine the amount of mRNA for a given gene. An oligonucleotide capable of specifically binding sequences a gene or fragments thereof relates to an oligonucleotide which specifically hybridizes to a gene or gene product, such as the gene's mRNA or cDNA or to a fragment thereof. To specifically detect the gene or gene product, it is not necessary to detect the entire gene sequence. A fragment of about 20-150 bases will contain enough sequence specific information to allow specific hybridization. In general, the hybridization-based methods are preferably RNA-hybridization-based technologies including spatial RNA analysis, for example.

The term "a PCR based method" as used herein refers to methods comprising a polymerase chain reaction (PCR). This is a method of exponentially amplifying nucleic acids, e.g., DNA by enzymatic replication in vitro. As PCR is an in vitro technique, it can be performed without restrictions on the form of DNA, and it can be extensively modified to perform a wide array of genetic manipulations. When it comes to the determination of expression levels, a PCR based method may for example be used to detect the presence of a given mRNA by (1) reverse transcription of the complete mRNA pool (the so called transcriptome) into cDNA with help of a reverse transcriptase enzyme, and (2) detecting the presence of a given cDNA with help of respective primers. This approach is commonly known as reverse transcriptase PCR (rtPCR). Moreover, PCR-based methods comprise e.g., real time PCR, and, particularly suited for the analysis of expression levels, kinetic or quantitative PCR (qPCR). As used herein, the term "primer" refers to the ordinary meaning of this term which is well known to the person skilled in the art of molecular biology. Primers shall be understood as being polynucleotide molecules having a sequence identical, complementary, homologous, or homologous to the complement of the regions of a target molecule, which is to be detected or quantified, e.g., the three or more markers.

The term "sequencing method" or "next generation sequencing methods" as used herein refers to high-throughput methods to provide insights in the transcriptome of a cell with high resolution and coverage. Using next generation sequencing-based techniques, such as RNA-Seq, the whole transcriptome may be analyzed. Sequencing methods as used herein include but are not limited to Sanger sequencing or next generation sequencing, such as RNA-Seq. RNA-Seq refers to method for determining the nucleotide sequences of RNA, which is based on high throughput methods, such as NGS. RNA-Seq includes reverse transcription of RNA to cDNA and subsequent DNA-sequencing. As used herein "quantitative transcriptomics" refers to a method for quantification of the transcriptome, e.g., by using RNA-Seq-based approaches. Sequencing can be applied to investigate mRNA, total RNA, pre-mRNA, and noncoding RNA, such as microRNA and long ncRNA. In a preferred embodiment, mRNA expression is determined using sequencing methods. Commonly used sequencing platforms which can be used for determining the expression levels of three or more markers selected from any one of Tables 2-8 include, but are not limited to, Massive Analysis of cDNA Ends (MACE) Sequencing, Illumina-based NGS technology, Oxford Nanopore Sequencing, ThermoFisher patho sequencing, 10X Visium Spatial Gene Expression, Twist Bioscience, Lexogen RNA-Seq, HTG Edge, Roche 454, GS FLX Titanium, Life Technologies SOLiD4, Complete Genomics, MGI, BGI. When it comes to the determination of expression levels, a sequencing- or next generation sequencing-based method may for example be used to detect the presence of a given mRNA by (1) library preparation, including cDNA synthesis, e.g., reverse transcription of the complete mRNA pool (the so-called transcriptome) into cDNA with help of a reverse transcriptase enzyme, (2) the sequencing step, and (3) data analysis.

Quantitative methods such as targeted RNA sequencing, hybridization-based assays and quantitative PCR are particularly preferred herein.

By "array" or "matrix" an arrangement of addressable locations or "addresses" on a device is meant. The locations can be arranged in two dimensional arrays, three dimensional arrays, or other matrix formats. The number of locations can range from several to at least hundreds of thousands. Most importantly, each location represents a totally independent reaction site. Arrays include but are not limited to nucleic acid arrays, protein arrays and antibody arrays. A "nucleic acid array" refers to an array containing nucleic acid probes, such as oligonucleotides, nucleotide analogues, polynucleotides, polymers of nucleotide analogues, morpholino oligomers or larger portions of genes. The nucleic acid and/or analogue on the array is preferably single stranded. Arrays wherein the probes are oligonucleotides are referred to as "oligonucleotide arrays" or "oligonucleotide chips." A "microarray," herein also refers to a "biochip" or "biological chip", an array of regions having a density of discrete regions of at least about 100/cm², and preferably at least about 1000/cm².

In one embodiment, the expression level of the three or more markers may be the protein level. It is clear to the person skilled in the art that a reference to a nucleotide sequence may comprise reference to the associated protein sequence which is coded by said nucleotide sequence. The expression level of a protein may be measured indirectly, e.g. by obtaining a signal wherein the signal strength is correlated to the amount of mRNA transcripts of that gene or it may be obtained directly at a protein level, e.g., by immunohistochemistry, CISH, ELISA (enzyme linked immunoassay), RIA (radioimmunoassay) or the use of protein microarrays, two- hybrid screening, blotting methods including western blot, one- and two dimensional gel electrophoresis, isoelectric focusing as well as methods being based on mass spectrometry like MALDI-TOF and the like. The term "immunohistochemistry" or IHC refers to the process of localizing proteins in cells of a tissue section exploiting the principle of antibodies binding specifically to antigens in biological tissues. Immunohistochemical staining is widely used in the diagnosis and treatment of cancer. Specific molecular markers are characteristic of particular cancer types. IHC is also widely used in basic research to understand the distribution and localization of biomarkers in different parts of a tissue. In a preferred embodiment, the protein expression levels of the three or more markers is determined by IHC. In some embodiments, three or more markers selected from any one of Tables 1.1, 1.2, or 3-8, preferably of Table 1.2 or 7.1, more preferably of Table 7.1, are stained with antibodies using immunohistochemical methods to determine the expression levels of said three or more markers in a tumor tissue sample obtained from a subject suffering from a HR+ and HER2- breast cancer, optionally early-stage breast cancer, to predict the response or resistance to and/or benefit from a systemic treatment in said subject, wherein the expression levels of said three or more markers are indicative for predicting the response or resistance to and/or benefit from the systemic treatment/chemotherapy in said subject.

Provided herein is a prognostic assay and/or the use of an prognostic assay for predicting the response to and/or benefit from a systemic treatment, preferably a neoadjuvant or adjuvant treatment, preferably a chemotherapy, more preferably a neoadjuvant treatment, most preferably a neoadjuvant chemotherapy, optionally comprising a taxane-containing agent for at least six weeks, in a subject suffering from a breast cancer, optionally a HR+ and HER2-breast cancer. Hence, the present invention also relates to a prognostic assay and/or the use of a prognostic assay for assessing the likelihood whether a patient suffering from a HR+ and HER2- breast cancer, optionally early-stage breast cancer, will benefit from and/or respond to a systemic treatment. In general, the prognostic assay is suitable to determine the expression levels of markers using the same methods as used for the determination of the expression levels of three or more markers as described above. In a preferred embodiment, the prognostic assay use an RNA-based method, such as NGS-based RNA analysis, PCR-based RNA analysis, sequencing-based RNA analysis, quantitative transcriptome analysis, quantitative RNA expression analysis, RNA-hybridization-based technologies including spatial RNA analysis or microarrays including Affymetrix. In one embodiment, the prognostic assay may comprise one or more detection reagents for determining the level of the expression level of the three or more markers and reference data including the reference level of the three or more markers. The prognostic assay may further comprise a software for (automatically) analyzing the determined expression levels of the three or more markers selected from any one of Tables 1.1, 1.2, or 3-8, preferably of Table 1.2 or 7.1, most preferably of Table 7.1, and optionally of the determined one or more additional markers, the one or more clinical parameters and/or the one or more non-clinical parameters. For example, the software may make a prediction of the response or resistance to and/or benefit form a systemic treatment, preferably a neoadjuvant or adjuvant treatment, preferably a chemotherapy, more preferably a neoadjuvant treatment, most preferably a neoadjuvant chemotherapy, optionally comprising a taxane-containing agent for at least six weeks, in a subject suffering from a HR+ and HER2- breast cancer based on the determined expression levels of the three or more markers selected from any one of Tables 1.1, 1.2, or 3-8, preferably of Table 1.2 or 7.1, most preferably of Table 7.1, and optionally of the determined one or more additional markers, the one or more clinical parameters and/or the one or more non-clinical parameters. This prediction of the software can be made by mathematically combining the determined expression levels of the three or more markers and/or by mathematically combining the determined expression levels of the three or more markers and the determined one or more additional markers, the one or more clinical parameters and/or the one or more non-clinical parameters by using a statistical and/or mathematical model. The statistical and/or mathematical model of the array may be one or more of weighted or non-weighted average, linear regression, logistic regression, Cox regression, non-linear regression, any other regression, principal component analysis, ANOVA, committee voting, supervised clustering, unsupervised clustering, nearest neighbor clustering, hierarchical clustering, any other clustering, and other machine learning algorithms. The software may comprise the expression levels of a training dataset to calculate a prediction.

The present invention also relates to the use of the method for predicting a response or resistance to and/or a benefit from a systemic treatment (i.e., systemic neoadjuvant or adjuvant treatment, preferably a systemic neoadjuvant or systemic adjuvant chemotherapy or a systemic neoadjuvant treatment, more preferably a systemic neoadjuvant chemotherapy, optionally comprising a taxane-containing agent) in a subject suffering from a HR+ and HER2-breast cancer, optionally early-stage breast cancer. Equally, the present invention relates to the use of the method for predicting the outcome of a systemic treatment in a subject suffering from a HR+ and HER2- breast cancer, optionally early-stage breast cancer.

As mentioned herein above, in addition to the expression level of the three or more markers, further parameters of the subject may be determined. As used herein, a parameter is a characteristic, feature, or measurable factor that can help in defining a particular system. A parameter is an important element for health- and physiology-related assessments, such as a disease/disorder/clinical condition risk. Furthermore, a parameter is defined as a characteristic that is objectively measured and evaluated as an indicator of normal biological processes, pathogenic processes, or pharmacologic responses to a therapeutic intervention. For example, such further markers include but are not limited to age, sex, menopausal status, molecular subtype, estrogen-receptor (ER) status, progesterone-receptor (PR) status, human epidermal growth factor receptor 2 (HER2) status, Ki-67, tumor infiltrating lymphocytes, PD-1 activity, PD-L1 activity, histological tumor type, nodal status, metastases status, TNM staging, smoking history, standard criteria for measuring how the disease impacts a patient's daily living abilities, such as Eastern Cooperative Oncology Group (ECOG) performance status (0: fully active to 5: dead) or Karnofsky status (100: fully active to 0: dead), tumor size at baseline and/or tumor grade at baseline. However, the method of the present invention does not need to rely on further parameters. In one embodiment, the method further comprises the determination of one more clinical parameters selected from the group consisting of pathological grading of the tumor, tumor size and nodal status. For example, the clinical parameter may be the pathological grading of the tumor at baseline and/or the tumor size at baseline and/or nodal status at baseline. The baseline refers to a value representing an initial level of a measurable quantity. The person skilled in the art knows that the baseline level may be determined before subject(s) are exposed to an environmental stimulus, receive an intervention such as a therapeutic treatment, or before a change of an environmental stimulus or a change in intervention such as a change in therapeutic treatment is induced. For example, the baseline may be determined before the start of the systemic treatment of the subject(s) or before the start of a therapeutic intervention, such as the neoadjuvant treatment, or before the start of another therapeutic intervention, such as a non-chemotherapy or chemotherapy combined with the neoadjuvant treatment. The baseline level may be used for comparison with values representing response or resistance, benefit and/or outcome to an environmental stimulus and/or intervention, for example a particular systemic treatment.

In other embodiments, a positive likelihood ratio, negative likelihood ratio, odds ratio, or hazard ratio is used as a measure of a test's ability to predict risk, response, outcome or benefit. In the case of a positive likelihood ratio, a value of 1 indicates that a positive result is equally likely among subjects in both the "good outcome" and "poor outcome" groups; a value greater than 1 indicates that a positive result is more likely in the diseased group; and a value less than 1 indicates that a positive result is more likely in the control group. In the case of a negative likelihood ratio, a value of 1 indicates that a negative result is equally likely among subjects in both the "diseased" and "control" groups; a value greater than 1 indicates that a negative result is more likely in the test group; and a value less than 1 indicates that a negative result is more likely in the control group. In certain preferred embodiments, markers and/or marker panels are preferably selected to exhibit a positive or negative likelihood ratio of at least about 1.5 or more or about 0.67 or less, more preferably at least about 2 or more or about 0.5 or less, still more preferably at least about 5 or more or about 0.2 or less, even more preferably at least about 10 or more or about 0.1 or less, and most preferably at least about 20 or more or about 0.05 or less. The term "about" in this context refers to +/- 5% of a given measurement.

In the case of an odds ratio, a value of 1 indicates that a positive result is equally likely among subjects in both the "diseased" and "control" groups; a value greater than 1 indicates that a positive result is more likely in the diseased group; and a value less than 1 indicates that a positive result is more likely in the control group. In certain preferred embodiments, markers and/or marker panels are preferably selected to exhibit an odds ratio of at least about 2 or more or about 0.5 or less, more preferably at least about 3 or more or about 0.33 or less, still more preferably at least about 4 or more or about 0.25 or less, even more preferably at least about 5 or more or about 0.2 or less, and most preferably at least about 10 or more or about 0.1 or less. The term "about" in this context refers to +/- 5% of a given measurement.

In the case of a hazard ratio, a value of 1 indicates that the relative risk of an endpoint (e.g., iDFS) is equal in both the "treatment" and "control/placebo" groups; a value greater than 1 indicates that the risk is higher in the diseased group; and a value less than 1 indicates that the risk is higher in the control group. In certain preferred embodiments, markers and/or marker panels are preferably selected to exhibit a hazard ratio of at least about 1.1 or more or about 0.91 or less, more preferably at least about 1.25 or more or about 0.8 or less, still more preferably at least about 1.5 or more or about 0.67 or less, even more preferably at least about 2 or more or about 0.5 or less, and most preferably at least about 2.5 or more or about 0.4 or less. The term "about" in this context refers to +/5% of a given measurement.

As used herein, the term "score" or "predictive score" refers to a numeric value derived from the mathematical combination of the expression levels of at least two markers and/or the combination of the expression level of the at least one marker and at least one further parameter. Preferably, the expression levels of three or more markers or the expression levels of three or more markers and one or more further parameters are combined to derive a numeric value, i.e., a score. As used herein, the term "combination" or "combining" refers to deriving a numeric value from a determined expression level of at least two markers, preferably of at least three markers, or from a determined expression level of at least one marker, preferably of at least three markers, and at least one further parameter. An algorithm may be applied to the expression levels of at least two markers or the expression level of at least one marker and at least one further parameter to obtain the numerical value or the score. An "algorithm" is a process that performs some sequence of operations to produce information. Preferably, the score is designed in such a way that high score values indicate a high risk / bad outcome, and low scores indicate a low risk / good outcome.

In some embodiments, a mathematical combination of expression levels of said three or more markers, ("score" or "predictive score") is compared to a reference score and/or a cutoff value. In some embodiments, the predictive score is compared to a reference value to determine the likelihood of a good response to and/or benefit from the systemic treatment, preferably a neoadjuvant or adjuvant treatment, preferably a chemotherapy, more preferably a neoadjuvant treatment, most preferably a neoadjuvant chemotherapy, optionally comprising a taxane-containing agent for at least six weeks. Such "reference-score" can be a numerical cutoff value, it can be derived from a reference measurement of one or more other markers in the same sample, or one or more other markers and/or the same marker in one other sample or in a plurality of other samples. The reference score can be a predetermined level that has been determined based on a population of healthy subjects or based on a population of population of subjects suffering from a HR+ and HER- breast cancer who have received a certain systemic treatment and for whom the response or resistance to and/or benefit from said systemic treatment is known. In one embodiment, the method comprises comparing the expression level of each of said three or more markers to a predetermined reference level. In another embodiment the reference value can be determined from a validation cohort, preferentially as the median or any other percentile, or preferentially by specifying one or more aims for statistical measures such as specificity, sensitivity, negative predictive value, positive predictive value, overall correctness, area under receiver operator curve, odds ratio, hazard ratio, or c-index.

The response or resistance to and/or the benefit from a systemic treatment, preferably a neoadjuvant or adjuvant treatment, preferably a chemotherapy, more preferably a neoadjuvant treatment, most preferably a neoadjuvant chemotherapy, optionally comprising a taxane-containing agent for at least six weeks, in a subject suffering from a HR+ and HER2-breast cancer may be predicted based on the comparison of the predictive score with a reference score. In some embodiments, the outcome of the treatment, optionally a neoadjuvant chemotherapy, in a subject suffering from a HR+ and HER2- breast cancer may be prognosticated based on the comparison of the predictive score with a reference score. In some embodiments, the response or resistance to and/or the benefit from the systemic treatment, preferably a neoadjuvant or adjuvant treatment, preferably a chemotherapy, more preferably a neoadjuvant treatment, most preferably a neoadjuvant chemotherapy, optionally comprising a taxane-containing agent for at least six weeks, in a subject suffering from a HR+ and HER2- breast cancer may be predicted and the outcome of the systemic treatment, preferably a neoadjuvant or adjuvant treatment, preferably a chemotherapy, more preferably a neoadjuvant treatment, most preferably a neoadjuvant chemotherapy, optionally comprising a taxane-containing agent for at least six weeks,in a subject suffering from a HR+ and HER2- breast cancer may be prognosticated based on the comparison of the predictive score with a reference score.

The skilled person will understand that associating a diagnostic or prognostic indicator, i.e., the expression level of the three or more markers, with the prediction of a response, benefit or with a prognostic risk of a future clinical outcome is a statistical analysis. For example, a marker level or a score of lower than X may signal that a subject is more likely to suffer from an adverse outcome than a subject with a level or score more than or equal to X, as determined by a level of statistical significance. Or a marker level or score of higher than X may signal that a subject is more likely to suffer from an adverse outcome than a subject with a level or score less than X, as determined by a level of statistical significance. Additionally, a change in marker concentration from baseline levels or may be reflective of subject prognosis, and the degree of change in marker level may be related to the severity of adverse events.

Statistical significance is often determined by comparing two or more populations and determining a confidence interval and/or a p value; see, e.g., Dowdy and Wearden, Statistics for Research, John Wiley & Sons, New York, 1983. Preferred confidence intervals of the invention are 90%, 95%, 97.5%, 98%, 99%, 99.5%, 99.9% and 99.99%, while preferred p values are 0.1, 0.05, 0.025, 0.02, 0.01, 0.005, 0.001, and 0.0001. For example, the expression levels of the three or more markers are indicative for the prediction and/or said prognosis and/or outcome compared to the expression level of a reference value at a p-value equal or below 0.05, preferably 0.01, more preferably 0.001 and even more preferably below 0.0001.

Combining the expression levels and/or parameters can be accomplished for example by multiplying each expression level and/or parameter with a defined and specified coefficient and summing up such products to yield a score. The score may be also derived from expression levels together with further parameter(s) like lymph node status or tumor grading as such variables can also be coded as numbers in an equation. The score may be used on a continuous scale to predict the response or resistance and/or benefit and/or the outcome of the subject to the systemic treatment, e.g., the systemic neoadjuvant or adjuvant treatment, preferably a systemic neoadjuvant or systemic adjuvant chemotherapy or a systemic neoadjuvant treatment, more preferably a systemic neoadjuvant chemotherapy, optionally comprising a taxane-containing agent. Cut-off values may be applied to distinguish clinically relevant subgroups, i.e., "responder", "non-responder", "positive outcome" and "negative outcome".

Cutoff values for such scores can be determined, for example, by constructing a receiver-operator curve (ROC curve) on the basis of all conceivable cut-off values, determining the single point on the ROC curve with the lowest proximity to the upper left corner (0/1) in the ROC plot. Typically, most of the time cut-off values will be determined by less formalized procedures by choosing the combination of sensitivity and specify determined by such cut-off value providing the most beneficial medical information to the problem investigated.

The sensitivity and specificity of a prognostic test depends on more than just the analytical "quality" of the test, they also depend on the definition of what constitutes a low risk or high risk result, and which systemic treatment decision is indicated by the test result. In practice, Receiver Operating Characteristic curves (ROC curves), are typically calculated by plotting the value of a variable versus its relative frequency in "low risk" (i.e., patients for whom standard treatment is the preferred choice) and "high risk" populations (i.e., patients who need alternative or intensified treatment). For any particular marker, a distribution of marker levels for subjects with and without a disease will likely overlap. Under such conditions, a test does not absolutely distinguish low from high risk with 100% accuracy, and the area of overlap indicates where the test cannot distinguish these risk classes.

Therefore, different markers are combined to improve the prognostic performance.

A treatment decision may be based on the mathematically combined score of the claimed markers but will consider other clinical factors in addition. These factors may include (but not limited to) the age or life expectancy of the patient, the general health status of the patient, the risks of side effects of the potential treatments for the individual patient, known prognostic biomarkers such as tumor size, nodal status, grading, proliferation, and immune markers, or the expression of genes not claimed. Cutoffs may be defined on different levels of the treatment decision process:
- solely on the mathematically combined score the of claimed markers,
- on a mathematical combination of said combined score and the expression of other genes,
- on a mathematical combination of said combined score and other clinical factors,
- on a mathematical combination of said combined score and all or any subset of clinical factors.

The cutoff may also depend on clinical factors. As an example, the risk may be expressed as the 10-year likelihood of a distant metastasis calculated from the claimed markers, the expression of some other genes, tumor size, and nodal status. For a young and healthy patient with low likelihood of severe treatment side effects the cutoff may be e.g. 5% for metastasis, while an older patient with known cardiac insufficiency and thus moderate risk of side effects may prefer to avoid intensified cancer treatment unless the risk of metastasis exceeds e.g. 40%.

The area under the ROC curve is a measure of the probability that the perceived measurement will allow correct identification of a condition. ROC curves can be used even when test results don't necessarily give an accurate number. As long as one can rank results, one can create a ROC curve. For example, results of a test might be ranked according to degree (e.g., 1=low, 2=intermediate, and 3=high, potentially results in three different treatment regimes). This ranking can be correlated to results in the "normal" population, and a ROC curve created. These methods are well known in the art (See, e.g., Hanley et al.1982. Radiology 143: 29-36). The horizontal axis of the ROC curve represents (1-specificity), which increases with the rate of false positives. The vertical axis of the curve represents sensitivity, which increases with the rate of true positives. Thus, for a particular cut-off selected, the value of (1-specificity) may be determined, and a corresponding sensitivity may be obtained.

Suitable threshold levels for the stratification of subjects into different groups (categories; e.g., subjects that are to be treated with the systemic treatment, preferably a neoadjuvant or adjuvant treatment, preferably a chemotherapy, more preferably a neoadjuvant treatment, most preferably a neoadjuvant chemotherapy, optionally comprising a taxane-containing agent for at least six weeks, and subjects which are not to be treated with the systemic treatment, preferably a neoadjuvant or adjuvant treatment, preferably a chemotherapy, more preferably a neoadjuvant treatment, most preferably a neoadjuvant chemotherapy, optionally comprising a taxane-containing agent for at least six weeks, have to be determined for each particular combination of a marker level, further markers and/or parameters, medication and disease. This can e.g., be done by grouping a reference population of patients according to their marker level into certain quantiles, e.g., quartiles, quintiles or even according to suitable percentiles. For each of the quantiles or groups above and below certain percentiles, hazard ratios can be calculated comparing the risk for a particular (adverse) outcome, i.e., an "unfavorable effect", e.g., in terms of survival rate, between those patients who have received a certain medication and those who received alternative medication. In such a scenario, a hazard ratio (HR) above 1 indicates a higher risk for an adverse outcome for the patients who have received a systemic treatment than for patients who did not. A HR below 1 indicates beneficial effects of a certain systemic treatment in the group of patients. A HR around 1 (e.g., +/- 0.1) indicates no elevated risk but also no benefit from medication for the particular group of patients. By comparison of the HR between certain quantiles of patients with each other and with the HR of the overall population of patients, it is possible to identify those quantiles of patients who have an elevated risk and those who benefit from medication and thereby stratify subjects according to the present invention.

Alternatively, a cutoff can be determined based on clinically motivated requirements on the specificity, on the sensitivity, on the likelihood for a clinical event (e.g., metastasis) in the low risk or in the high-risk group, or on the HR between the two risk groups. The definition of the specificity, sensitivity, and event likelihood may depend on a clinically motivated definition of a time frame, e.g., an event within 10 years after diagnosis. The cutoff values to compare the combined score with may depend on the treatment options available.

A "discriminant function" is a function of a set of variables used to classify an object or event. A discriminant function thus allows classification of a patient, samples or event into a category or a plurality of categories according to data or parameters available from said subject, sample or event. Such classification is a standard instrument of statistical analysis well known to the skilled person. For example, the subject may be classified to be indicative for the prediction and/or prognosis of group i) to iv):
i) an increased likelihood of the patient to respond and/or benefit from a systemic treatment, preferably a neoadjuvant or adjuvant treatment, preferably a chemotherapy, more preferably a neoadjuvant treatment, most preferably a neoadjuvant chemotherapy, optionally comprising a taxane-containing agent for at least six weeks,;
ii) an increased likelihood of the patient not to respond to, to be resistant to and/or not to benefit from systemic treatment, preferably a neoadjuvant or adjuvant treatment, preferably a chemotherapy, more preferably a neoadjuvant treatment, most preferably a neoadjuvant chemotherapy, optionally comprising a taxane-containing agent for at least six weeks,;
iii) an increased likelihood of the patient to have a good outcome after a systemic treatment, preferably a neoadjuvant or adjuvant treatment, preferably a chemotherapy, more preferably a neoadjuvant treatment, most preferably a neoadjuvant chemotherapy, optionally comprising a taxane-containing agent for at least six weeks,
iv) an increased likelihood of the patient have a poor outcome after a systemic treatment, preferably a neoadjuvant or adjuvant treatment, preferably a chemotherapy, more preferably a neoadjuvant treatment, most preferably a neoadjuvant chemotherapy, optionally comprising a taxane-containing agent for at least six weeks.

Classification is not limited to these categories, but may also be performed into a plurality of categories, such as "responder" and "good outcome" or grading or the like. A "responder" is a subject who responds and/or is predicted to response to a certain systemic treatment. A "non-responder" is a subject who does not respond and/or is predicted to not respond to a certain systemic treatment. Classification shall also be understood in a wider sense as a discriminating score, where e.g., a higher score represents a higher likelihood of distant metastasis, e.g., the (overall) risk of a distant metastasis. Examples for discriminant functions which allow a classification include, but are not limited to, functions defined by support vector machines (SVM), k-nearest neighbors (kNN), (naive) Bayes models, linear regression models or piecewise defined functions such as, for example, in subgroup discovery, in decision trees, in logical analysis of data (LAD) and the like. In a wider sense, continuous score values of mathematical methods or algorithms, such as correlation coefficients, projections, support vector machine scores, other similarity-based methods, combinations of these and the like are examples for illustrative purpose. For example, the expression level of each of said at least one marker comprises combining the expression level of each of the at least one marker with a coefficient, wherein the coefficient is indicative for the prognosis and/or prediction.

In one embodiment, the at least one marker is substituted by at least one substitute marker, wherein the expression level of the substitute marker correlates with the expression level of the at least one marker. The decision whether the at least one marker may be substitute with a substitute marker may be determined by the Pearson correlation coefficient. The application of Pearson's correlation coefficient is common to statistical sampling methods, and it may be used to determine the correlation of two variables. The Pearson coefficient may vary between -1 and +1. A coefficient of 0 indicates that neither of the two variables can be predicted from the other 'y a linear equation, while a correlation of +1 or -1 indicates that one variable may be perfectly predicted by a linear function of the other. A more detailed discussion of the Pearson coefficient may be found in McGraw-Hill Encyclopedia of Science and Technology, 6th Edition, Vol. 17. For example, the substitute marker correlates with the at least one marker by an absolute value of the Pearson correlation coefficient of at least |0.4|, preferably at least |0.6|, more preferably of at least |0.8|. For example, the expression levels of the markers of Table 3 correlate to each other. Thus, the markers of Table 3 may be substitute markers for each other. However, other markers not comprised in Table 3 may also substitute for each other if they are correlating as described above.

The present invention also relates to kits and the use of kits for assessing the likelihood whether a patient suffering from a HR+ and HER2- breast cancer, optionally early-stage breast cancer, will benefit from and/or respond to or be resistant to a systemic treatment. The kits comprise one or more detection reagents for determining the level of the expression level of the three or more markers and reference data including the reference level of the three or more markers, optionally wherein said detection reagents comprise at least a pair of oligonucleotides capable of specifically binding to the three or more markers. As used herein, the term "primer" refers to the ordinary meaning of this term which is well known to the person skilled in the art of molecular biology. Primers shall be understood as being polynucleotide molecules having a sequence identical, complementary, homologous, or homologous to the complement of the regions of a target molecule, which is to be detected or quantified, e.g., the three or more markers.

The present invention also relates to a method for classifying an adaptive breast cancer subtype in a tumor sample. The method comprises obtaining a training dataset, performing a cluster analysis on the training dataset, associating each cluster to an outcome, combining the clusters to prediction clusters according to outcome risk, wherein the prediction clusters correspond to the adaptive cancer subtypes, determining the expression levels of three or more markers selected from Table 1.1 in a tumor sample obtained from a subject suffering from a HR+ and HER2- breast cancer, and assigning said subject to an adaptive cancer subtype, wherein the assigning is based on combined clusters of training patients. In one embodiment, the method for classifying an adaptive breast cancer subtype in a tumor sample obtained from a subject suffering from HR+ and HER2- breast cancer comprises the steps of: obtaining a training dataset, by determining the expression levels of all markers listed in Table 1.1 in a plurality of pre-NACT and post-NACT training samples obtained from a training cohort, wherein each pre-NACT sample can be matched to a post-NACT sample; performing a first cluster analysis on the training dataset to obtain a clustered dataset, the methods of clustering are not particularly limited and may be any one of supervised clustering, unsupervised clustering, nearest neighbor clustering, hierarchical clustering, any other clustering, preferably the method of clustering is hierarchical clustering; associating each cluster to an outcome, wherein an outcome may be associated if the cluster comprises at least one pre-NACT sample, preferably the cluster comprises more pre-NACT samples than post-NACT samples; combining of the clusters to prediction clusters according to outcome risk, wherein the prediction clusters correspond to the adaptive cancer subtypes; determining the expression levels of three or more markers selected from Table 1.1 in a tumor sample obtained from a subject suffering from a HR+ and HER2- breast cancer; and assigning said subject to an adaptive cancer subtype, wherein the assigning is based on combined clusters of training patients. In one embodiment, the method may further comprise adding the expression level of three or more marker from a tumor sample obtained from a said subject to the training dataset, performing a second clustering of the training dataset additionally comprising the expression level data of said subject, identifying the cluster of the tumor sample of said subject, identifying the training samples in said cluster, determining into which cluster said training samples had clustered in the first cluster analysis, assigning the subject to the cluster to the cluster of the first cluster analysis comprising the highest percentage of training samples which clustered with the tumor sample of the subject in the second clustering.

A "training dataset", as used herein, refers to a dataset comprising the expression level data of training samples obtained from a training cohort. The training dataset can be used to train a machine learning model or an algorithm to predict the response or resistance to and/or benefit from a systemic treatment in a subject suffering from a HR+ and HER2- breast cancer. The training dataset may be split into two parts, wherein one part serves a training dataset and the second part serves as validation dataset.

A "training cohort", as used herein, refers to a plurality of patients who are known to suffer from a particular neoplastic disease, i.e., early-stage, HR+ and HER2- breast cancer and who have received neoadjuvant treatment, in particular neoadjuvant chemotherapy, preferably for at least 16 weeks, comprising administration of a taxane-containing agent for at least six weeks. Hence, the response or resistance and/or benefit from said neoadjuvant treatment of each of the patients of the training cohort is known. Preferably after the neoadjuvant treatment, the patients of the training cohort did not reach pathological complete remission (pCR) after the neoadjuvant treatment, and residual invasive disease remained either in the breast or as residual nodal invasion after neoadjuvant treatment. After neoadjuvant treatment, the patients of the training cohort underwent surgery, including resection of all clinically evident disease and ipsilateral axillary lymph node dissection, wherein histologically complete resection (R0) of the invasive and ductal in situ tumor was required in case of breast conserving surgery as the final treatment, no evidence of gross residual disease (R2) was required after total mastectomy (R1 resection is acceptable), or axillary dissection was not required in patients with a negative sentinel-node biopsy before (pN0, pN+(mic)) or after (ypN0, ypN+(mic)) neoadjuvant chemotherapy. Optionally the patients of the training cohort received adjuvant radiotherapy. The patients of the training cohort may, for example, be the participants of the PENELOPE^{B} study and/or have the same clinical picture. The patients of the training cohort may serve as reference subjects for the prediction of the response to or resistance to and/or benefit from neoadjuvant treatment in a subject, for example based on the known expression levels of markers and the outcome in one or more patients of the training cohort, the outcome of a subject can be predicted. The training cohort may be separated into two part, the larger part serves as training cohort and the smaller part is used for validation. The validation cohort is not used for the training model.

A "training sample", as used herein, refers to a biological sample obtained from a patient of the training cohort. A training sample can be obtained from a patient of the training cohort before and/or after neoadjuvant treatment. Preferably two training samples are obtained from one patient of the training cohort, one before and one after neoadjuvant treatment. The sample obtained before the neoadjuvant treatment is referred to as "pre-NACT sample". The pre-NACT sample is a biopsy sample from a breast cancer before chemotherapy. The sample obtained after the neoadjuvant treatment is referred to as "post-NACT sample". The post-NACT sample is a resection sample from a breast cancer after neoadjuvant chemotherapy. Preferably, each training sample obtained from a patient of the training cohort before neoadjuvant treatment can be matched to a training sample obtained after neoadjuvant treatment from the same patient of the training cohort. The expression levels of all markers comprised in Table 1.1 are determined in the training samples as described above. For example, the mRNA expression levels in the training samples may be determined using HTG EdgeSeq Oncology Biomarker Panel targeting 2549 genes. In some embodiments, the markers of Table 1.1 are expressed differentially in the pre- and post-NACT training sample of one patient of the training cohort. A training sample can be a tumor sample, such as a tumor tissue sample, particularly wherein the sample is a primary tumor tissue sample, particularly a core biopsy sample, more particularly a core biopsy sample from a primary tumor before any treatment. It may, however, also be a post-surgical residual tumor tissue sample or a post-surgical lymph node sample. In one typical application, the sample is a formalin-fixed, paraffin-embedded (FFPE) tumor tissue sample.

"Adaptive breast cancer subtype", as used herein, refers to breast cancer subtypes which can be associated with a certain prediction of a response or resistance to and/or benefit from a certain treatment. The decision of treatment of a subject suffering from a HR+ and HER2-breast cancer may be based on the association of said subject with an adaptive breast cancer subtype. The adaptive breast cancer subtypes are superior compared to the classical intrinsic AIMS subtypes, as they are able to predict the response or resistance to and/or benefit from a systemic treatment, in particular a neoadjuvant chemotherapy, more accurately than the AIMS subtypes. Furthermore, the adaptive breast cancer subtypes of the present invention reflect molecular alterations pre- and post-therapy. AC-1 and AC-3 are enriched for tumors with an AIMS subtype change from LumB to LumA during NACT, with improved iDFS for AC-1. AC-2 and AC-4 are enriched for tumors with a constant LumA AIMS subtype before and after NACT, with improved iDFS for AC-2, AC-5 is enriched for tumors with a pre-NACT Basal/HER2E AIMS subtype with the worst prognosis.

The clustering or cluster analysis applied in the methods of the present invention encompasses a variety of algorithmic approaches, including but not limited to supervised clustering, unsupervised clustering, nearest neighbor clustering, hierarchical clustering, k-means clustering, density-based clustering, and any other clustering. In some embodiments, the clustering includes supervised clustering, unsupervised clustering, nearest neighbor clustering, hierarchical clustering, any other clustering. The methods address the specific challenges posed by the data, such as high dimensionality, heterogeneity, and noise. The clustering methods are applicable to a wide range of medical research areas, including but not limited to genomic and proteomic data analysis for biomarker discovery and disease classification; medical imaging analysis for tumor detection and segmentation for example for histology purposes; and patient stratification for personalized medicine and treatment optimization.

As used herein, the term "distance calculation" refers to the calculation of the distance between the sample obtained from the subject and the centroids (or representative points) of each predictive cluster, i.e., adaptive breast cancer subtype. It may also refer to the calculation of the distance between the expression level of on sample obtained from a subject and the expression levels of a plurality of training samples obtained from a training cohort. Common distance metrics include Euclidean distance, Manhattan distance, or Mahalanobis distance, depending on the nature of your data and the clustering method used. One may also use single linkage. The subject may be assigned to the cluster with the minimal distance. This may be done using a nearest centroid classifier.

### Particular items of the present invention

In particular aspects, the invention relates to the following items:
1. A method for predicting a response or resistance to and/or benefit from a systemic treatment in a subject suffering from a hormone receptor-positive (HR+) and human epidermal growth factor receptor 2-negative (HER2-) breast cancer, comprising the step of:
   a) determining in a sample obtained from said subject the expression levels of three or more markers selected from Table 1.1;
   wherein the expression levels of the three or more markers are indicative for predicting the response or resistance to and/or benefit from the systemic treatment in said subject.
2. A method for predicting the outcome of a systemic treatment in a subject suffering from a hormone receptor positive (HR+) and human epidermal growth factor receptor 2-negative (HER2-) breast cancer, comprising the step of:
   a) determining in a sample obtained from said subject the expression level of three or more markers selected from Table 1.1;
   wherein the expression level of the three or more markers is indicative for the outcome of the systemic treatment in said subject.
3. The method of item 1 further comprising the steps of:
   (b) mathematically combining the determined expression levels of the three or more markers selected from Table 1.1 to generate a prediction, preferably a predictive score; and
   (c) predicting the response or resistance to and/or benefit from said systemic treatment in said subject based on the prediction, preferably based on the predictive score.
4. The method of any one of items 1 to 3, wherein the systemic treatment is neoadjuvant or adjuvant treatment, preferably wherein the systemic treatment is neoadjuvant treatment.
5. The method of any one of items 1 to 4, wherein the systemic treatment is chemotherapy, preferably wherein the systemic treatment is neoadjuvant chemotherapy.
6. The method of item 5, wherein the chemotherapy, preferably the neoadjuvant chemotherapy, comprises a taxane-containing agent, anthracycline, and/or a platinum-based chemotherapeutic drug.
7. The method of item 6, wherein the chemotherapy, preferably the neoadjuvant chemotherapy, is to be administered for 16 weeks and/or comprises a taxane-containing agent for at least six weeks, optionally wherein the taxane-containing agent is any one of paclitaxel, docetaxel, abraxane, cabazitaxel, and albumin-bound paclitaxel (nab-paclitaxel).
8. The method of any one of items 1 to 7, wherein the HR+ and HER2- breast cancer is early-stage breast cancer.
9. The method of any one of items 1 to 8, wherein the method further comprises the determination of one or more clinical parameters selected from the group consisting of pathological grading of the tumor, clinical tumor size (cT), clinical nodal status (cN), proliferation (in particular Ki-67), immune system markers (in particular lymphocytes, preferably tumor infiltrating lymphocytes (TILs)), estrogen receptor status, progesterone receptor status, and variables derived from them (clinical and pathological stage, CPS-EG).
10. The method of any one of items 1 to 9, further comprising normalizing the determined expression levels of said three or more markers prior to mathematically combining them.
11. The method of any one of items 1 to 10, wherein the mathematical combination of the determined expression levels of said three or more markers selected from Table 1.1 to generate a prediction, optionally a mathematical combination of the determined expression levels of said three or more markers and one or more additional markers, one or more clinical parameters and/or one or more non-clinical parameters, is performed using a statistical and/or mathematical model.
12. The method of any one of items 1 to 11, wherein the response or resistance to the systemic treatment is measured by the long-term development of the disease, in particular development of invasive relapses, distant metastases, disease-related survival, and/or overall survival.
13. The method of any one of items 1 to 12, wherein the expression levels of three or more markers selected from any one of Tables 3-8 are determined.
14. The method of item 13, wherein the expression levels of three or more markers selected from Table 4 are determined, preferably Table 5, more preferably Table 6 or Table 8, most preferably Table 7.1.
15. The method of item 14, wherein the expression levels of three or more markers selected from Table 6 or 7.1 are determined and the high expression levels of three or more markers selected from Table 6 or 7.1 in a tumor sample are indicative for a good response of the subject to the neoadjuvant chemotherapy and/or the patient is predicted to benefit from the neoadjuvant treatment, optionally wherein the three or more markers are DNA repair genes.
16. The method of item 13, wherein the expression levels of three or more markers selected from Table 8 are determined and high expression levels of a plurality of markers selected from Table 8 in a tumor sample are indicative for a bad response of the subject to the treatment and/or the patient is predicted to not benefit from the treatment.
17. The method of any one of items 1 to 16, wherein said subject has a low chance to reach a pathological complete remission (pCR) after systemic treatment.
18. The method of any one of items 1 to 17, wherein at least five markers are selected from Table 1.1, preferably at least ten markers from Table 1.1, more preferably at least twenty markers from Table 1.1, more preferably at least fifty markers from Table 1.1, most preferably all markers from Table 1.1 are selected.
19. The method of any one of items 1 to 18, wherein the tumor sample is tumor tissue sample, preferably wherein the sample is a primary tumor tissue sample, more preferably a core biopsy sample, more preferably a core biopsy sample from a primary tumor before any systemic treatment, most preferably a core biopsy sample from a primary tumor before neoadjuvant treatment.
20. The method of any one of items 1 to 19, wherein the expression levels are determined at gene level, preferably mRNA level, in a hybridization-based method, a PCR based method, a microarray-based method, a quantitative transcriptomic analysis, a sequencing and/or next generation sequencing (NGS) method.
21. The method of any one of items 1 to 20, further comprising comparing the predictive score to a reference score to determine the response or resistance to and/or benefit from said systemic treatment in said subject,
   optionally wherein the subject is predicted to respond to and/or benefit from the systemic treatment if the predictive score is equal to or below the reference score or the subject is predicted not to respond to, to be resistant to and/or not to benefit from the systemic treatment if the predictive score is above the reference score.
22. Method of any one of items 1 to 21,
   a) wherein if the subject is predicted to respond to and/or benefit from the systemic treatment, the patient is to be treated with said systemic treatment, preferably wherein said systemic treatment is neoadjuvant chemotherapy; or
   b) wherein if the subject is predicted not to respond to, to be resistant to and/or not to benefit from the systemic treatment, preferably wherein said systemic treatment is neoadjuvant chemotherapy, the subject is to be treated with a different systemic treatment or with said systemic treatment and an additional systemic treatment.
23. The method according to any one of items 1 to 22, further comprising classifying an adaptive breast cancer subtype in a tumor sample.
24. The method of item 23, further comprising predicting the response or resistance to and/or benefit from a systemic treatment, preferably neoadjuvant chemotherapy, in a subject suffering from a HR+ and HER2- breast cancer based on the classification of the adaptive breast cancer subtype.
25. The method of any one of items 1 to 24, wherein the three or more markers are selected from Table 1.1.
26. The method of any one of items 1 to 24, wherein the three or more markers are selected from Table 3.
27. The method of any one of items 1 to 24, wherein the three or more markers are selected from Table 4.
28. The method of any one of items 1 to 24, wherein the three or more markers are selected from Table 5.
29. The method of any one of items 1 to 24, wherein the three or more markers are selected from Table 6.
30. The method of any one of items 1 to 24, wherein the three or more markers are selected from Table 7.1.
31. The method of any one of items 1 to 24, wherein the three or more markers are selected from Table 7.2.
32. The method of any one of items 1 to 24, wherein the three or more markers are selected from Table 7.3.
33. The method of any one of items 1 to 24, wherein the three or more markers are selected from Table 8.
34. The method of any one of items 1 to 32, wherein the subject is a human female.
35. Neoadjuvant chemotherapy for use in the treatment of a HR+ and HER2- breast cancer, wherein the neoadjuvant chemotherapy is to be administered to a subject who has been identified to respond to and/or benefit from said treatment or for whom said treatment has been determined to have a positive outcome according to the method of any one of items 1 to 34.
36. Neoadjuvant chemotherapy comprising a taxane-containing agent for at least six weeks for use in the treatment of a HR+ and HER2- breast cancer, wherein the neoadjuvant chemotherapy is to be administered to a subject who has been identified to respond to and/or benefit from said treatment or for whom said treatment has been determined to have a positive outcome according to the method of any one of items 1 to 34, optionally wherein the taxane-containing agent is any one of paclitaxel, docetaxel, abraxane, cabazitaxel, and albumin-bound paclitaxel (nab-paclitaxel).
37. A method for therapy guidance, wherein a decision about systemic treatment, preferably neoadjuvant chemotherapy, is based on a prediction of response or resistance to and/or benefit from said systemic treatment, preferably neoadjuvant chemotherapy, wherein the prediction is made according to the method of any one of items 1 to 34,
   a) wherein if the subject is predicted to respond to and/or benefit from said systemic treatment, preferably neoadjuvant chemotherapy, the patient is treated with said systemic treatment; or
   b) wherein if the subject is predicted not to respond to, to be resistant to and/or not to benefit from said systemic treatment, preferably neoadjuvant chemotherapy, the subject is treated with a different systemic treatment or with said systemic treatment and an additional systemic treatment.
38. A method for stratifying the risk of subjects suffering from HER+ and HER2- breast cancer, wherein the method comprises predicting the response or resistance to and/or benefit from systemic treatment, preferably neoadjuvant chemotherapy, according to the method of any one of items 1 to 34.
39. Method of treating a subject suffering from HR+ and HER2- breast cancer with systemic treatment, preferably neoadjuvant chemotherapy, wherein the subject has been predicted to respond to and/or benefit from said systemic treatment, preferably neoadjuvant chemotherapy, optionally wherein said neoadjuvant chemotherapy comprises a taxane-containing agent for at least six weeks.
40. Method of treating a subject suffering from HR+ and HER2- breast cancer with a combination of two or more systemic treatments, wherein the subject has been predicted not to respond to, to be resistant to and/or not to benefit from only one systemic treatment, preferably neoadjuvant chemotherapy.
41. Use of a prognostic assay for predicting the response or resistance to and/or benefit from a systemic treatment, preferably neoadjuvant chemotherapy, in a subject suffering from a HR+ and HER2- breast cancer, wherein the prognostic assay comprises a RNA-based assay, optionally wherein the prognostic assay is one or more of NGS-based RNA analysis, PCR-based RNA analysis, sequencing-based RNA analysis, quantitative transcriptome analysis, quantitative RNA expression analysis, RNA-hybridization-based technologies including spatial RNA analysis or microarrays including Affymetrix.
42. A computer-implemented method for predicting the response or resistance to and/or benefit from a systemic treatment in a subject suffering from a hormone receptor-positive (HR+) and human epidermal growth factor receptor 2-negative (HER2-) breast cancer comprising the steps of:
   (a) obtaining expression level data of three or more markers selected from Table 1.1 in a tumor tissue sample;
   (b) processing the expression level data of each of the three or more markers using a statistical model, wherein the processing comprises mathematically combining the determined expression levels of the three or more markers selected from Table 1.1 to generate a predictive score and optionally comprises normalizing the determined expression levels of said three or more markers prior to mathematically combining them; and
   (c) predicting the response or resistance to and/or benefit from said systemic treatment in said subject based on the predictive score.
43. A data processing system comprising means for carrying out the steps of the method of item 42.
44. A computer program product comprising instructions which, when the program is executed by a computer, cause the computer and/or the data processing system of item 43 to carry out the steps of the method of item 42.
45. A computer-readable storage medium comprising instructions which, when executed by a computer, cause the computer to carry out the steps of the method of item 42 and having stored thereon the computer program of item 44.
46. A method for predicting a response or resistance to and/or benefit from a neoadjuvant chemotherapy comprising a taxane-containing agent for at least six weeks in a female subject suffering from an early-stage, hormone receptor-positive (HR+) and human epidermal growth factor receptor 2-negative (HER2-) breast cancer, comprising the steps of:
   a) determining in a core biopsy sample from a primary tumor obtained from said subject the expression levels of all markers selected from Table 7.1, wherein the expression level is determined at mRNA level in a next generation sequencing-based method;
   wherein the expression level of said markers is indicative for predicting the response or resistance to and/or benefit from said neoadjuvant chemotherapy in said subject;
   (b) optionally determining of one or more additional biomarkers, one or more clinical parameters and/or one or more non-clinical parameters;
   (c) optionally normalizing the determined expression levels of said three or more markers and optionally of said one or more additional biomarkers, one or more clinical parameters and/or one or more non-clinical parameters;
   (d) using a mathematical and/or statistical model to mathematically combine the determined expression levels of said markers selected from Table 7.1 and optionally of said one or more additional biomarkers, one or more clinical parameters and/or one or more non-clinical parameters to generate a predictive score;
   (e) comparing the predictive score to a reference score to determine the response or resistance to and/or benefit from said neoadjuvant chemotherapy in said subject; and
   (f) predicting the response or resistance to and/or benefit from said subject to said treatment based on the comparison of the predictive score and the reference value;
   wherein the response or resistance to treatment is measured by the long-term development of the disease, in particular development of invasive relapses, distant metastases, disease-related survival, and/or overall survival.
47. Method for classifying an adaptive breast cancer subtype in a tumor sample obtained from a subject suffering from a HR+ and HER2- breast cancer.
48. The method of item 46 further comprising predicting the response to and/or benefit from a neoadjuvant treatment of a subject suffering from a HR+ and HER2- breast cancer based on the classification as an adaptive breast cancer subtype.
49. Chemotherapy for use in the treatment of a HR+ and HER2- breast cancer, wherein the chemotherapy is to be administered to a subject who has been predicted to respond to and/or benefit from said chemotherapy or for whom said chemotherapy has been determined to have a positive outcome according to the method of any one of items 1 to 34.
50. Endocrine therapy for use in the treatment of a HR+ and HER2- breast cancer, wherein the endocrine therapy is to be administered to a subject who has been predicted to respond to and/or benefit from said endocrine therapy or for whom said endocrine therapy has been determined to have a positive outcome according to the method of any one of 1 to 34.
51. Immunotherapy therapy for use in the treatment of a HR+ and HER2- breast cancer, wherein the immunotherapy is to be administered to a subject who has been predicted to respond to and/or benefit from said immunotherapy or for whom said immunotherapy has been determined to have a positive outcome according to the method of any one of 1 to 34.
52. Targeted therapy for use in the treatment of a HR+ and HER2- breast cancer, wherein the targeted therapy is to be administered to a subject who has been predicted to respond to and/or benefit from said targeted therapy or for whom said targeted therapy has been determined to have a positive outcome according to the method of any one of 1 to 34.
53. Anti-angiogenic therapy for use in the treatment of a HR+ and HER2- breast cancer, wherein the anti-angiogenic therapy is to be administered to a subject who has been predicted to respond to and/or benefit from said anti-angiogenic therapy or for whom said anti-angiogenic therapy has been determined to have a positive outcome according to the method of any one of 1 to 34.
54. A combination therapy comprising two or more systemic treatments for the use in the treatment of a HR+ and HER2- breast cancer, wherein the combination therapy comprising two or more systemic treatments is to be administered to a subject who has been predicted to respond to and/or benefit from said combination therapy comprising two or more systemic treatments or for whom said combination therapy comprising two or more systemic treatments have been determined to have a positive outcome according to the method of any one of 1 to 34.
55. A combination therapy comprising two or more systemic treatments for the use in the treatment of a HR+ and HER2- breast cancer, wherein the combination therapy comprising two or more systemic treatments is to be administered to a subject who has been predicted not to respond to, to be resistant to and/or not to benefit from only one systemic treatment or for whom only one systemic treatment has been determined to have a negative outcome according to the method of any one of 1 to 34.
56. A combination therapy comprising a neoadjuvant chemotherapy and an additional systemic therapy for the use in the treatment of a HR+ and HER2- breast cancer, wherein the combination therapy comprising a neoadjuvant chemotherapy and an additional systemic therapy is to be administered to a subject who has been predicted to respond to and/or benefit from said combination therapy comprising a neoadjuvant chemotherapy and an additional systemic therapy or for whom said combination therapy comprising a neoadjuvant chemotherapy and an additional systemic therapy have been determined to have a positive outcome according to the method of any one of 1 to 34.
57. A combination therapy comprising a neoadjuvant chemotherapy and an additional systemic therapy for the use in the treatment of a HR+ and HER2- breast cancer, wherein the combination therapy comprising a neoadjuvant chemotherapy and an additional systemic therapy is to be administered to a subject who has been predicted not to respond, to be resistant to and/or not to benefit from said neoadjuvant chemotherapy alone or for whom said neoadjuvant chemotherapy alone has been determined to have a negative outcome according to the method of any one of 1 to 34.
58. The combination therapy of item 56 or 57, wherein the neoadjuvant chemotherapy comprises a taxane-containing agent for at least six weeks, optionally wherein the neoadjuvant chemotherapy is to be administered for 16 weeks.
59. The combination therapy of any one of items 53 to 58, wherein the additional therapy is a neoadjuvant, post-neoadjuvant or adjuvant systemic treatment.
60. The combination therapy of item 59, wherein the additional therapy is endocrine therapy.
61. The combination therapy of item 59, wherein the additional therapy is immunotherapy.
62. The combination therapy of item 59, wherein the additional therapy is targeted therapy.
63. The combination therapy of item 59, wherein the additional therapy is anti-angiogenic therapy.
64. The combination therapy of item 59, wherein the additional therapy comprises a CDK4/6 inhibitor.
65. A method for therapy control, therapy guidance, monitoring, risk assessment, and/or risk stratification in a subject suffering from a HR+ and HER2- breast cancer.

### EXAMPLES

In the Example section several abbreviations are used, as defined in the following. If applicable, the definitions apply throughout the entire application.

AC-1, AC-2, ...: adaptive cluster(s). Found by unsupervised hierarchical clustering, see example 1 for details.
AIMS: Absolute assignment of breast cancer intrinsic molecular subtype.
cN: clinical nodal status, i.e., number of positive lymph nodes before treatment
cT: clinical tumor size, i.e., size of tumor before treatment
iDFS: invasive disease-free survival, primary endpoint of the PENELOPE-B study
NACT: neoadjuvant chemotherapy
pCR: pathological complete remission (of the tumor after NACT)
PENELOPE-B: Clinical study, NCT01864746, see
https://www.clinicaltrials.gov/study/NCT01864746 for details
post-NACT sample: Resection sample from a breast cancer after neoadjuvant chemotherapy
pre-NACT sample: Biopsy sample from a breast cancer before chemotherapy
TILs: tumor infiltrating lymphocytes
ypN: post-NACT nodal status, i.e., positive lymph nodes after NACT
ypT: post-NACT tumor size, i.e., size of tumor after NACT

### Example 1:

### Overview of clinical study

The data underlying the present invention were obtained in the context of the PENELOPE^{B} study (NCT01864746).

PENELOPE^{B} is a randomized, double-blind, placebo-controlled Phase 3 study comparing one year of palbociclib plus at least five years of standard adjuvant endocrine therapy to placebo plus at least five years of standard adjuvant endocrine therapy in 1,250 women with HR+, HER2- early breast cancer (eBC) at high risk of recurrence who have residual invasive disease after completing neoadjuvant chemotherapy. Patients in the trial scored 3 or higher (or 2 if there were lymph node metastases at the time of surgery) on the clinical-pathologic stage - estrogen/grade (CPS-EG). The CPS-EG is a validated risk assessment tool combining: clinical stage before neoadjuvant treatment, pathological stage after neoadjuvant treatment, grading and estrogen-receptor status.

More than 190 clinical sites in 12 countries around the globe participated in PENELOPE^{B}. The study opened in November 2013 and closed recruitment on December 31, 2017.

### Inclusion and Exclusion Criteria of the PENELOPE^{B} study

Gene lists were identified based on patients from the post-neoadjuvant PENELOPE-B breast cancer study (NCT01864746). These patients were selected according to the following conditions:

### Inclusion Criteria

1) Written informed consent prior to beginning specific protocol procedures, including expected cooperation of the patients for the treatment and follow-up, must be obtained and documented according to the local regulatory requirements.
2) Willingness and ability to provide archived formalin fixed paraffin embedded tissue block or a partial block from surgery after neoadjuvant chemotherapy and from core-biopsy before start of neoadjuvant chemotherapy, which will be used for centralized retrospective confirmation of hormone- and HER2-status and to evaluate correlation between genes, proteins, and mRNAs relevant to the endocrine and cell cycle pathways and sensitivity/resistance to the investigational agents. In case of bilateral breast cancer, tumor tissue of both sides needs to be assessable.
3) Histologically confirmed unilateral or bilateral primary invasive carcinoma of the breast.
4) Residual invasive disease post-neoadjuvant either in the breast or as residual nodal invasion.
5) Centrally confirmed hormone-receptor-positive (≥1% ER and/or PR positive stained cells) and HER2-normal (IHC score 0-1 or FISH negative (in-situ hybridization (ISH) ratio) <2.0 status) assessed preferably on tissue from post-neoadjuvant residual invasive disease or core biopsy of the breast, or if no other tissue is available, the residual tumor of the lymph node can be assessed. In case of bilateral breast cancer, hormone receptor positivity and HER2-normal status has to be centrally confirmed for both sides.
6) Centrally assessed Ki-67, pRB, and Cyclin D1 status assessed preferably on post-neoadjuvant residual invasive disease of the breast, or if not possible, of residual nodal invasion or core biopsy. In case of bilateral breast cancer, tumor tissue of both sides needs to be assessable.
7) Patients must have received neoadjuvant chemotherapy of at least 16 weeks. This period must include 6 weeks of a taxane-containing neoadjuvant therapy (Exception: For patients with progressive disease that occurred after at least 6 weeks of taxane-containing neoadjuvant treatment, a total treatment period of less than 16 weeks is also eligible).
8) Adequate surgical treatment including resection of all clinically evident disease and ipsilateral axillary lymph node dissection. Histologically complete resection (R0) of the invasive and ductal in situ tumor is required in case of breast conserving surgery as the final treatment. No evidence of gross residual disease (R2) is required after total mastectomy (R1 resection is acceptable). Axillary dissection is not required in patients with a negative sentinel-node biopsy before (pN0, pN+(mic)) or after (ypN0, ypN+(mic) neoadjuvant chemotherapy.
9) Less than 16 weeks interval since the date of final surgery or less than 10 weeks from completing radiotherapy (whichever occurs last) at date of randomization.
10) Completion of adjuvant radiotherapy according to standard guidelines (e.g., AGO Mamma, NCCN) is strongly recommended. If radiotherapy is not performed, the reason for this needs to be documented in the eCRF.
11) No clinical evidence for locoregional or distant relapse during or after preoperative chemotherapy. Local progression during chemotherapy is not an exclusion criterion.
12) A clinical-pathologic stage - estrogen/grade (CPS-EG) score of ≥3, or score 2 if nodal status at surgery is ypN+, calculated using local estrogen receptor status and grade assessed on either core biopsies taken before start of neoadjuvant treatment or surgical specimen (see chapter 21.1 of the study protocol).
13) Age at diagnosis at least 18 years.
14) Eastern Cooperative Oncology Group (ECOG) performance status (PS) 0 or 1.
15) Resolution of all acute toxic effects of prior anti-cancer therapy or surgical procedures to NCI Common Terminology Criteria for Adverse Events (CTCAE) version 4.0 Grade ≤1 (except alopecia or other toxicities not considered a safety risk for the patient at investigator's discretion).
16) Estimated life expectancy of at least 5 years irrespective of the diagnosis of breast cancer.
17) The patient must be accessible for scheduled visits, treatment and follow-up. Patients registered in this trial must be treated at the participating center which could be the Principal Investigator's or a Co-investigator's site.

### Exclusion Criteria

1) Known severe hypersensitivity reactions to compounds similar to palbociclib or palbociclib/placebo excipients or to endocrine treatments.
2) Inadequate organ function immediate prior to randomization including: Hemoglobin <10g/dL (100g/L); ANC < 2000/mm³ (< 2.0 x 109/L); Platelets <100,000/mm³ (< 100 x 109/L); AST or ALT >1.5 x upper limit of normal (ULN); alkaline phosphatase > 2.5 x ULN, total serum bilirubin > 1.25 x ULN; serum creatinine >1.25 x ULN or estimated creatinine clearance < 60 mL/min as calculated using the method standard for the institution; severe and relevant co-morbidity that would interact with the participation in the study
3) Evidence for infection including wound infections, human immunodeficiency virus (HIV) or any type of hepatitis
4) QTc >480 msec
5) Uncontrolled electrolyte disorders (eg, hypocalcemia, hypokalemia, hypomagnesemia).
6) Any of the following within 6 months of randomization: myocardial infarction, severe/unstable angina, ongoing cardiac dysrhythmias of NCI CTCAE version 4.0 Grade ≥2, atrial fibrillation of any grade, coronary/peripheral artery bypass graft, symptomatic congestive heart failure, cerebrovascular accident including transient ischemic attack, or symptomatic pulmonary embolism.
7) Active inflammatory bowel disease or chronic diarrhea, short bowel syndrome, or any upper gastrointestinal surgery including gastric resection.
8) Prior malignancy (including invasive or ductal in-situ breast cancer) within 5 years prior to randomization, except curatively treated basal cell carcinoma of the skin and carcinoma in situ of the cervix.
9) Current severe acute or uncontrolled chronic systemic disease (e.g., diabetes mellitus) or psychiatric condition or laboratory abnormality that may increase the risk associated with study participation or investigational product administration or may interfere with the interpretation of study results and, in the judgment of the investigator, would make the patient inappropriate for entry into this study.
10) Recent (within the past year) or active suicidal behavior.
11) Pregnancy or lactation period. Women of childbearing potential must implement adequate non-hormonal contraceptive measures (barrier methods, intrauterine contraceptive devices, sterilization) during study treatment and for 90 days after discontinuation. A serum pregnancy test must be negative in premenopausal women or women with amenorrhea of less than 12 months.
12) Major surgery within 2 weeks prior to randomization.
13) 10 weeks or more have passed since completion of radiotherapy at day of randomization and 16 weeks interval since the date of final surgery have passed.
14) Prior treatment with any CDK4/6 inhibitor.
15) Patients treated within the last 7 days prior to randomization and/or concurrent use of drugs known to be strong CYP3A4 inhibitors or inducers
16) Concurrent treatment with other experimental drugs. Participation in another clinical trial with any investigational not marketed drug within 30 days prior to randomization.
17) Male patients.

### Gene expression analysis

A total of 1250 ER+/HER2- BC patients with residual disease after neoadjuvant chemotherapy (NACT) and increased risk (CPS-EG score of ≥3 or 2 with ypN+) were randomized into the PENELOPE^{B} (NCT01864746) trial to receive palbociclib or placebo; see Figure 1 for an overview.

RNA was extracted from pre-NACT biopsies and post-surgical residual tumor tissue of patients from the Penelope^{B} study and quantified using the HTG EdgeSeq Oncology Biomarker Panel (HTG Molecular Diagnostics Inc.) resulting in a so-called count value for each of the 2559 genes in the assay. Quality control measures were performed as described by the manufacturer. For 906 patients HTG measurements were successfully performed and passed said quality controls. Normalized expression values were calculated from the count values using the CPM (counts per million) method and application of a lower bound of 3.

Biomarker analysis was performed in 629 pre-NACT and 782 post-NACT tumor samples including 540 paired samples using the HTG EdgeSeq Oncology Biomarker Panel targeting 2549 genes (HTG Molecular Diagnostics Inc.), with assessment of the absolute assignment of breast cancer intrinsic molecular subtype (AIMS).

Within the 540 paired samples 335 genes were found to be differentially expressed between the pre-NACT and the post-NACT sample. Unsupervised hierarchical clustering of the 1080 pre-NACT and post-NACT samples and said 335 genes resulted in 9 sample clusters named AC-1, AC-2, AC-3, AC-4, AC-5, AC-6, AC-7, AC-8, and AC-9 (AC = adaptive cluster). Most pre-NACT samples (516 of 540) were found in clusters AC-1, AC-2, AC-3, AC-4, and AC-5, which were then examined for prognostication of patient outcome (Fig. 2).

Surprisingly said clusters correlated strongly with the AIMS subtypes which are known to be prognostic. Patients were classified by the combination of pre-NACT and post-NACT AIMS and these classes were grouped by the risk of disease progression. The following table shows the percentage of AIMS subtype combinations for each cluster (pre-NACT sample):

**Table 9: Percentage of AIMS subtype combinations for each cluster (pre-NACT sample)**

| **Pre-NACT AIMS** | **Post-NACT AIMS** | **AC-1** | **AC-2** | **AC-3** | **AC-4** | **AC-5** |
|---|---|---|---|---|---|---|
| LumB | LumA | 45.14% | 12.06% | 48.36% | 18.56% | 0.00% |
| LumA | LumA | 24.31% | 75.89% | 13.93% | 67.01% | 0.00% |
| any | LumB, HER2E, BasalL | 15.28% | 2.13% | 28.69% | 6.19% | 91.67% |
| any | NormL | 13.89% | 8.51% | 7.38% | 6.19% | 8.33% |
| any other combination | | 1.39% | 1.42% | 1.64% | 2.06% | 0.00% |

| predominant AIMS combination | | switch from LumB to LumA | constantly LumA | switch from LumB to LumA | constantly LumA | High-risk post-NACT |
|---|---|---|---|---|---|---|
| number of patients / pre-NACT samples | | 144 | 141 | 122 | 97 | 12 |

Surprisingly classification of patients by clusters prognosticated patient outcome (iDFS) better than pre-NACT AIMS, see Figure 2. Therefore, said clusters are a superior biomarker for patients fulfilling the inclusion and exclusion criteria above.

Two large groups of tumors with excellent prognosis (adaptive cluster AC-1 and AC-2), as well as two groups with a poor prognosis (AC-3 and AC-4) were identified. The worst prognosis was observed in a small group of tumors with a pre-NACT Basal/HER2E AIMS subtype (AC-5).

AC-1 and AC-3 are enriched for tumors with an AIMS subtype change from LumB to LumA during NACT, with improved iDFS for AC-1. AC-2 and AC-4 are enriched for tumors with a constant LumA AIMS subtype before and after NACT, with improved iDFS for AC-2. The low-risk adaptive subtypes AC-1 and AC-2 cover a total of 52.7% of patients with a combined event rate of 2.1%. In contrast, the best pretherapeutic conventional AIMS-subtype, pre-NACT LumA, covers 51.7% of patients, but still has an event rate of 15.4%. This suggests that adaptive subtypes are superior to classical AIMS subtypes for prediction of survival after NACT in luminal BC.

Table 1.1 lists the 335 differentially expressed genes in pre-NACT and post-NACT samples of the PENELOPE^{B} study.

**Table 1.1: 335 differentially expressed markers**

| | | | | | | | |
|---|---|---|---|---|---|---|---|
| ABCA3 | ABCD1 | ACKR1 | ADAMTS1 | ADIPOQ | ADIPOR1 | ADM | AKR1C3 |
| AKT3 | ALDH1A1 | ALDH1A3 | ANGPTL1 | ANLN | ANXA1 | ANXA3 | APOC2 |
| AQP1 | ASPM | BAG2 | BMI1 | BOC | BRCA1 | BTG2 | BUB1 |
| C3 | CACNG4 | CAV1 | CAV2 | CCL14 | CCL2 | CCNA2 | CCNB1 |
| CCNE1 | CCT5 | CD209 | CD34 | CDC14B | CDC20 | CDC6 | CDK5R1 |
| CDK9 | CDKN1A | CDKN1C | CDKN2C | CDKN3 | CEBPD | CELSR2 | CENPF |
| CITED2 | CLCA2 | CNTFR | COL11A1 | COL1A2 | COL4A3 | COL6A6 | CREB5 |
| CSF2 | CCN2 | CXCL10 | CXCL16 | CXCL2 | CXCL3 | CXCL9 | CXCR1 |
| CXCR4 | CYP1A1 | CYP1B1 | CCN1 | DAB2 | DES | DESI1 | DKK2 |
| DLC1 | DLGAP5 | DNAJC14 | DNAJC22 | DUSP1 | DUSP8 | DVL1 | E2F1 |
| EDN1 | EGFR | EGR1 | EGR3 | EMP1 | ETS2 | EXO1 | EZH2 |
| F3 | FABP4 | OTULINL | FANCA | FAS | FAT4 | FBXW7 | FCGR1A |
| FGF14 | FGF2 | FGF7 | FGFBP1 | VEGFD | TENM3 | FLNC | FLRT2 |
| FN1 | FOS | FOXA1 | FOXC1 | FOXO1 | GADD45A | GADD45B | GADD45G |
| GAPDH | GAS1 | GATA3 | GFRA1 | GGH | GLUL | GNGT2 | GPI |
| ADGRG6 | GPR160 | GPSM2 | GRIN2A | GSN | GSTP1 | GTSE1 | GZMB |
| H2AX | HBEGF | HERC3 | HES1 | HIF3A | H3C10 | HJURP | HMGB3 |
| HMGCS2 | HSPA14 | HSPB6 | ID4 | IGF1 | IGF2 | IGFBP6 | IKBKG |
| IL18 | IL20 | IL33 | IL4 | IL6 | IRF7 | ISG15 | JMJD1C |
| JUN | JUNB | JUND | KDM4B | KIF2C | KIT | KLF4 | KRT14 |
| KRT17 | KRT19 | KRT5 | LEPR | LIF | LIFR | LIG4 | LPL |
| LRP2 | LTB | LYVE1 | MADD | MAFF | MAGEL2 | MAML2 | MAOB |
| MAP1B | MAPK10 | MAZ | MEIS1 | MIF | MKI67 | MMP11 | MMP2 |
| MMP9 | MST1 | MT1A | MT1X | MTDH | MYB | MYBL1 | MYBL2 |
| MYCN | NAT1 | NCAPD3 | NF2 | NME1 | NOL3 | NOLC1 | CCN3 |
| NPC1 | NR1H3 | NR4A1 | NR4A3 | NRAS | NRIP1 | NSD1 | NTH L1 |
| NUF2 | NUP62 | NUSAP1 | OAS1 | OASL | OCLN | OGG1 | ORC6 |
| PABPC1 | PCNA | PCOLCE | PCSK6 | PDIA4 | PDPK1 | PDZK1 | PER1 |
| PFKL | PGR | PIAS4 | PIK3R2 | PIK3R3 | PIM2 | PITRM1 | PKM |
| PKMYT1 | PLAU | PLCG1 | PLK1 | PLK4 | PMAIP1 | PMS2P3 | POLD1 |
| POLD2 | POLR2D | PLPP1 | PLPP3 | PPID | PPP2R2C | PTPA | PRC1 |
| PRDX6 | PRKCZ | PRL | PTGS2 | PTN | RAB25 | RAD21 | RASD1 |
| RASGRF2 | RASSF7 | RBL1 | RELN | RET | RGS2 | RHOU | RIPK2 |
| RPS4X | RRM2 | RUNX1T1 | SAP30 | SELE | SERPINA3 | SERPINE1 | SERTAD1 |
| SFN | SFRP1 | SFRP4 | SGK1 | SLC16A3 | SLC19A3 | SLC27A4 | SLC2A3 |
| SLIT2 | SMAD9 | SOCS3 | SOX17 | SPC25 | SPHK1 | SPP1 | SPRY1 |
| SPRY2 | SRC | SREBF1 | STAT1 | STEAP4 | STK11 | STK36 | STMN1 |
| SUSD3 | SYCP2 | TAP1 | TBP | TCF4 | TCF7L1 | TEK | TESC |
| TFF1 | TGFB3 | TGFBR2 | THBS2 | THBS4 | TIPARP | TK1 | TMEM132A |
| TMPRSS2 | TNC | TNFAIP3 | TNFRSF1B | TNN | TNXB | TOP2A | TPO |
| TPX2 | TRAF2 | TRIB1 | TSC22D3 | TSPAN7 | TUBB3 | TWIST1 | TWIST2 |
| TXNIP | TYMS | UBE2T | VCAM1 | WNT2 | XBP1 | ZFP36L1 | |

### Example 2

Many genes from example 1 (Table 2) correlate to each other. These correlations reveal involvement in common biological mechanisms. The following Table 3 contains these genes from Table 2 correlating highly to each other.

**Table 3: 184 highly correlating markers**

| | | | | | | | |
|---|---|---|---|---|---|---|---|
| ACKR1 | ADIPOQ | ALDH1A1 | ANGPTL1 | ANLN | ANXA1 | AQP1 | ASPM |
| BOC | BRCA1 | BUB1 | CAV1 | CAV2 | CCL14 | CCL2 | CCNA2 |
| CCNB1 | CCNE1 | CD209 | CDC14B | CDC20 | CDC6 | CDK5R1 | CDKN1C |
| CDKN2C | CDKN3 | CENPF | CLCA2 | CNTFR | COL11A1 | COL1A2 | COL4A3 |
| CREB5 | CSF2 | CCN2 | CXCL10 | CXCL16 | CXCL2 | CXCL3 | CXCL9 |
| CXCR1 | CYP1A1 | CCN1 | DAB2 | DKK2 | DLC1 | DLGAP5 | DNAJC14 |
| DUSP8 | E2F1 | EDN1 | EGFR | ETS2 | EXO1 | EZH2 | FANCA |
| FAS | FAT4 | FBXW7 | FGF14 | FGF2 | FGF7 | FGFBP1 | TENM3 |
| FLNC | FLRT2 | FN1 | FOXO1 | GAS1 | GNGT2 | GPSM2 | GRIN2A |
| GSN | GTSE1 | GZMB | H2AX | HERC3 | HES1 | H3C10 | HJURP |
| HSPB6 | ID4 | IGF1 | IGF2 | IGFBP6 | IKBKG | IL18 | IL33 |
| IL4 | IL6 | IRF7 | ISG15 | KIF2C | LEPR | LIF | LIG4 |
| LPL | LTB | LYVE1 | MADD | MAGEL2 | MEIS1 | MKI67 | MMP11 |
| MMP2 | MTDH | MYBL1 | MYBL2 | MYCN | CCN3 | NR1H3 | NR4A3 |
| NRAS | NSD1 | NUF2 | NUP62 | NUSAP1 | OAS1 | OASL | OGG1 |
| ORC6 | PCNA | PCOLCE | PFKL | PIM2 | PITRM1 | PKMYT1 | PLAU |
| PLK1 | PLK4 | POLD1 | POLR2D | PLPP1 | PLPP3 | PRC1 | PRDX6 |
| PRL | PTGS2 | PTN | RASD1 | RASGRF2 | RBL1 | RELN | RIPK2 |
| RPS4X | RRM2 | RUNX1T1 | SAP30 | SELE | SFRP1 | SFRP4 | SLC19A3 |
| SLIT2 | SMAD9 | SOCS3 | SOX17 | SPC25 | SPHK1 | SPRY1 | SPRY2 |
| STAT1 | STK36 | STMN1 | TAP1 | TBP | TCF4 | TEK | TESC |
| TGFB3 | TGFBR2 | THBS2 | TK1 | TNFAIP3 | TNFRSF1B | TNN | TNXB |
| TOP2A | TPX2 | TSPAN7 | TWIST2 | TYMS | UBE2T | VCAM1 | WNT2 |

### Example 3

To reduce costs, one may reduce the number of genes that need to be measured. This and the following examples illustrate usage of subsets from Table 2.

One way to select genes is to restrict genes to those being prognostic for iDFS in the biopsy samples by univariable Cox regression analysis.

**Table 4: 206 prognostic markers**

| | | | | | | | |
|---|---|---|---|---|---|---|---|
| ABCA3 | ACKR1 | ADAMTS1 | ADIPOQ | ADIPOR1 | ANLN | ANXA1 | APOC2 |
| AQP1 | ASPM | BMI1 | BRCA1 | BTG2 | BUB1 | C3 | CACNG4 |
| CCL14 | CCNA2 | CCNB1 | CCNE1 | CCT5 | CD209 | CDC6 | CDK5R1 |
| CDK9 | CDKN1A | CDKN1C | CDKN2C | CEBPD | CENPF | CITED2 | CCN2 |
| CXCL16 | CXCR1 | CXCR4 | CYP1B1 | DAB2 | DESI1 | DKK2 | DLC1 |
| DLGAP5 | DNAJC14 | DUSP1 | DUSP8 | DVL1 | EGR1 | EGR3 | EXO1 |
| OTULINL | FANCA | FCGR1A | FGF7 | FLNC | FOXA1 | FOXC1 | GAS1 |
| GATA3 | GFRA1 | GGH | GLUL | GNGT2 | GPI | GPR160 | GPSM2 |
| GSN | GSTP1 | GTSE1 | H2AX | HBEGF | HERC3 | HES1 | H3C10 |
| HJURP | HMGB3 | HMGCS2 | HSPA14 | IGF1 | IGF2 | IGFBP6 | IKBKG |
| IL18 | JMJD1C | JUNB | KDM4B | KIF2C | LIF | LIG4 | LTB |
| LYVE1 | MADD | MAFF | MAML2 | MAP1B | MAPK10 | MAZ | MKI67 |
| MMP2 | MST1 | MTDH | MYB | MYCN | NAT1 | NCAPD3 | NF2 |
| NME1 | NOL3 | NOLC1 | CCN3 | NR1H3 | NR4A1 | NR4A3 | NRAS |
| NRIP1 | NSD1 | NTH L1 | NUF2 | NUP62 | OAS1 | OASL | OCLN |
| OGG1 | ORC6 | PCSK6 | PDPK1 | PDZK1 | PER1 | PFKL | PGR |
| PIAS4 | PIK3R2 | PIK3R3 | PIM2 | PITRM1 | PKM | PKMYT1 | PLAU |
| PLCG1 | PLK4 | PMAIP1 | PMS2P3 | POLD1 | POLR2D | PLPP1 | PLPP3 |
| PPID | PPP2R2C | PTPA | PRC1 | PRDX6 | PRKCZ | PTGS2 | RASSF7 |
| RGS2 | RIPK2 | RPS4X | RRM2 | RUNX1T1 | SAP30 | SELE | SERPINA3 |
| SERTAD1 | SFN | SFRP1 | SFRP4 | SGK1 | SLC27A4 | SLC2A3 | SMAD9 |
| SOCS3 | SPHK1 | SPP1 | SPRY1 | SRC | SREBF1 | STAT1 | STEAP4 |
| STK11 | STK36 | STMN1 | SUSD3 | TAP1 | TBP | TCF4 | TCF7L1 |
| TEK | TESC | TGFB3 | TGFBR2 | THBS2 | TIPARP | TK1 | TMEM132A |
| TMPRSS2 | TNFAIP3 | TNFRSF1B | TNN | TNXB | TOP2A | TPX2 | TRAF2 |
| TUBB3 | TXNIP | TYMS | VCAM1 | WNT2 | XBP1 | | |

### Example 4

To further reduce the number of genes one may group clusters by outcome (Figure 2): Patients in clusters AC-1 and AC-2 have good outcome while patients in cluster AC-3, AC-4, and AC-5 have bad outcome. Only a subset of genes in Table 4 are needed to determine whether a patient is classified into cluster AC-1 or AC-2 on one hand or cluster AC-3, AC-4, or AC-5 in the other hand.

**Table 5: 91 markers from Table 4 distinguishing AC-1 and AC-2 vs AC-3, AC-4, and AC-5**

| | | | | | | | |
|---|---|---|---|---|---|---|---|
| ADIPOR1 | APOC2 | BMI1 | CDK5R1 | CDKN2C | CXCL16 | CXCR4 | DESI1 |
| DNAJC14 | DUSP8 | DVL1 | OTULINL | GGH | GLUL | GNGT2 | GPI |
| GPR160 | GPSM2 | GSN | GSTP1 | H2AX | HERC3 | HES1 | H3C10 |
| HMGB3 | HMGCS2 | IKBKG | IL18 | JMJD1C | LIG4 | MADD | MAFF |
| MKI67 | MST1 | MTDH | MYB | MYCN | NCAPD3 | NF2 | NME1 |
| NOL3 | NR1H3 | NR4A1 | NRAS | NRIP1 | NSD1 | NUF2 | NUP62 |
| OCLN | OGG1 | PFKL | PIAS4 | PIK3R2 | PIM2 | PITRM1 | PKM |
| PKMYT1 | PLAU | PLCG1 | PLK4 | PMAIP1 | POLR2D | PLPP1 | PLPP3 |
| PPID | PRC1 | PRDX6 | PRKCZ | RGS2 | RIPK2 | RPS4X | SAP30 |
| SFN | SOCS3 | SPHK1 | SRC | STK11 | STK36 | TAP1 | TBP |
| TCF4 | TCF7L1 | TEK | TESC | TGFB3 | TGFBR2 | TIPARP | TMEM132A |
| TMPRSS2 | TPX2 | TXNIP | | | | | |

### Example 5

The mathematical clustering algorithm in example 1 is a non-monotonic function with respect to gene expressions as inputs and patient risk / outcome estimation as output variables. With the restriction to two risk outcome groups introduced in example 4 the clustering becomes a monotonic function in each gene: We can now distinguish between good prognosis genes and bad prognosis genes. For a good prognosis gene the higher the expression the better the outcome for the patient, i.e., a higher expression indicates a lower risk of an iDFS event while a lower expression indicates a higher risk of an iDFS event. Reduction to good prognosis genes only allows further reduction of costs and potential insight into biological mechanisms.

**Table 6: 66 good prognosis markers from Table 5**

| | | | | | | | |
|---|---|---|---|---|---|---|---|
| ADIPOR1 | BMI1 | CDK5R1 | CDKN2C | DESI1 | DNAJC14 | DUSP8 | DVL1 |
| GGH | GLUL | GNGT2 | GPI | GPR160 | GPSM2 | HMGB3 | IKBKG |
| IL18 | LIG4 | MADD | MAFF | MST1 | MTDH | MYB | MYCN |
| NCAPD3 | NF2 | NME1 | NOL3 | NR1H3 | NR4A1 | NRAS | NRIP1 |
| NSD1 | NUF2 | NUP62 | OCLN | OGG1 | PFKL | PIAS4 | PIK3R2 |
| PIM2 | PITRM1 | PKM | PKMYT1 | PLAU | PLCG1 | PLK4 | PMAIP1 |
| POLR2D | PLPP1 | PLPP3 | PPID | PRC1 | PRDX6 | PRKCZ | RGS2 |
| RIPK2 | RPS4X | SAP30 | SFN | SPHK1 | SRC | STK11 | STK36 |
| TAP1 | TBP | | | | | | |

### Example 6

Further restriction of genes may be performed based on their ontology: chromosomal location, biological function, pathway, or some other common properties. Table 6 is enriched for DNA repair genes. The enrichment becomes even stronger if only genes correlating to each other are considered: Tables 3 and 6 have 36 gene in common; 24 of them are DNA repair genes.

**Table 7.1: 24 correlating DNA repair markers**

| | | | | | | | |
|---|---|---|---|---|---|---|---|
| CDK5R1 | DNAJC14 | DUSP8 | GNGT2 | IL18 | LIG4 | MADD | MTDH |
| NR1H3 | NRAS | NSD1 | NUP62 | OGG1 | PFKL | PIM2 | PITRM1 |
| PLK4 | POLR2D | PRDX6 | RIPK2 | RPS4X | SAP30 | STK36 | TBP |

### Example 7

Even further reduction of the number of genes may be achieved by variable selection algorithms based on regression models for patient outcome. As an example, backward selection based on multivariable Cox regression models for iDFS (invasive disease-free survival) adjusted for important clinical variables was used to select genes from Table 7.1. The following table shows the Cox regression results for the finally selected genes (all continuous variables, first eight rows) and the adjusting clinical variables (all binary, rows 9 to 15). A score predicting the risk of iDFS for an individual patient can be calculated as a linear combination of all 15 variables where the linear coefficients is shown in column "coefficient estimate"; the higher said score the higher the likelihood of an iDFS event for the patient.

**Table 10: 8 markers in final cox regression model from backward selection**

| Variable | Coefficient estimate | Hazard ratio (95% confidence interval) | p-value |
|---|---|---|---|
| PIM2 | -0.29 | 0.75 (0.59 - 0.95) | 0.0153 |
| STK36 | -0.29 | 0.75 (0.52 - 1.07) | 0.1090 |
| OGG1 | -0.83 | 0.44 (0.29 - 0.67) | 0.0001 |
| MTDH | -0.37 | 0.69 (0.52 - 0.91) | 0.0085 |
| PITRM1 | -0.56 | 0.57 (0.40 - 0.81) | 0.0016 |
| TBP | -0.49 | 0.61 (0.45 - 0.83) | 0.0014 |
| NUP62 | 0.94 | 2.55 (1.62 - 4.02) | <0.0001 |
| PLK4 | 0.53 | 1.69 (1.22 - 2.34) | 0.0015 |
| Age: >50 vs <=50 years | 0.06 | 1.06 (0.75 - 1.52) | 0.7319 |
| Region: Asian vs non-Asian | -0.56 | 0.57 (0.22 - 1.46) | 0.2394 |
| cT3-4 vs cT1-2 | -0.20 | 0.82 (0.55 - 1.21) | 0.3213 |
| ypT3-4 vs ypT0-2 | 0.70 | 2.01 (1.30 - 3.11) | 0.0018 |
| cN+ vs cN0 | -0.10 | 0.91 (0.46 - 1.80) | 0.7816 |
| ypN+ vs ypN0 | -0.26 | 0.77 (0.41 - 1.46) | 0.4290 |
| G3 vs G1-2 (pathological grading) | 0.14 | 1.15 (0.77 - 1.70) | 0.4955 |

All genes are good prognosis genes; therefore, one might expect all gene coefficients to be negative (the higher the expression the lower the likelihood of an iDFS event). Surprisingly, this is not true for genes NUP62 and PLK4 which indicates a more complex (e.g., non-monotonic) structure of the interaction between gene expressions and outcome.

### Example 8

While example 5 suggests using good prognosis genes only one can alternatively use bad prognosis genes only.

**Table 8: 25 bad prognosis markers from Table 5**

| | | | | | | | |
|---|---|---|---|---|---|---|---|
| APOC2 | CXCL16 | CXCR4 | OTULINL | GSN | GSTP1 | H2AX | HERC3 |
| HES1 | H3C10 | HMGCS2 | JMJD1C | MKI67 | SOCS3 | TCF4 | TCF7L1 |
| TEK | TESC | TGFB3 | TGFBR2 | TIPARP | TMEM132A | TMPRSS2 | TPX2 |
| TXNIP | | | | | | | |

### Example 9

A similar but alternative method to example 7 is to create a predictive linear model without clinical variables. Using the same genes coefficients are determined from a multivariable Cox regression model without clinical variables.

**Table 11: Linear predictor from multivariable Cox regression mode in Table 8.**

| Variable / gene | Coefficient estimate | Hazard ratio (95% confidence interval) | p-value |
|---|---|---|---|
| PIM2 | -0.23 | 0.80 (0.64 - 0.99) | 0.0435 |
| STK36 | -0.43 | 0.65 (0.47 - 0.90) | 0.0102 |
| OGG1 | -0.78 | 0.46 (0.31 - 0.69) | 0.0001 |
| MTDH | -0.39 | 0.68 (0.53 - 0.87) | 0.0023 |
| PITRM1 | -0.45 | 0.64 (0.46 - 0.88) | 0.0061 |
| TBP | -0.50 | 0.61 (0.45 - 0.82) | 0.0009 |
| NUP62 | 0.86 | 2.37 (1.50 - 3.76) | 0.0002 |
| PLK4 | 0.49 | 1.64 (1.21 - 2.21) | 0.0014 |

| | | | |
|---|---|---|---|
| This model shows a concordance of 0.827. | | | |

Coefficients in this model do not differ significantly from the coefficients in example 7.

### Example 10

Another alternative to examples 7 and 9 is to determine the coefficient of a gene from a univariable Cox regression model. While multivariable regression models become less robust and coefficient become larger confidence intervals with increasing numbers of genes, univariable regression models are robust regardless of the number of genes. Application of said method on Table 9 yields.

**Table 12: Linear predictor from univariable Cox regression models in Table 9**

| Variable / gene | Coefficient estimate | Hazard ratio (95% confidence interval) | p-value |
|---|---|---|---|
| APOC2 | 0.34 | 1.41 (1.19 - 1.67) | <.0001 |
| CXCL16 | 0.94 | 2.56 (2.09 - 3.14) | <.0001 |
| CXCR4 | 0.71 | 2.04 (1.64 - 2.54) | <.0001 |
| OTULINL | 0.37 | 1.44 (1.22 - 1.71) | <.0001 |
| GSN | 0.82 | 2.26 (1.85 - 2.78) | <.0001 |
| GSTP1 | 0.41 | 1.51 (1.29 - 1.76) | <.0001 |
| H2AX | 0.84 | 2.31 (1.83 - 2.90) | <.0001 |
| HERC3 | 0.94 | 2.56 (2.09 - 3.13) | <.0001 |
| HES1 | 0.79 | 2.21 (1.90 - 2.57) | <.0001 |
| H3C10 | 0.69 | 1.99 (1.65 - 2.39) | <.0001 |
| HMGCS2 | 0.11 | 1.11 (1.01 - 1.23) | 0.0389 |
| JMJD1C | 1.10 | 3.00 (1.97 - 4.56) | <.0001 |
| MKI67 | 0.74 | 2.11 (1.69 - 2.62) | <.0001 |
| SOCS3 | 0.65 | 1.92 (1.62 - 2.29) | <.0001 |
| TCF4 | 0.48 | 1.62 (1.18 - 2.21) | 0.0026 |
| TCF7L1 | 0.30 | 1.35 (1.15 - 1.59) | 0.0003 |
| TEK | 0.47 | 1.60 (1.33 - 1.92) | <.0001 |
| TESC | 0.29 | 1.34 (1.15 - 1.56) | 0.0002 |
| TGFB3 | 0.27 | 1.31 (1.05 - 1.65) | 0.0179 |
| TGFBR2 | 0.86 | 2.36 (1.80 - 3.09) | <.0001 |
| TIPARP | 0.47 | 1.60 (1.26 - 2.03) | 0.0001 |
| TMEM132A | 0.42 | 1.52 (1.27 - 1.82) | <.0001 |
| TMPRSS2 | 0.27 | 1.31 (1.17 - 1.46) | <.0001 |
| TPX2 | 0.56 | 1.76 (1.42 - 2.17) | <.0001 |
| TXNIP | 0.63 | 1.87 (1.51 - 2.31) | <.0001 |

Genes are bad prognosis genes; therefore, all coefficients are positive. The higher the score (linear combination of gene expressions using coefficients from the table above), the higher the likelihood of an iDFS event. Figure 3 shows the outcome of patients after application of the median score value as cutoff (i.e., a patient is assigned "low score" if the score is below the median score and "high score" otherwise).

### Example 11

After selection of good prognosis genes only (or bad prognosis genes only as an alternative with alternative prediction score sign), one can create a linear predictor without using any endpoint such as iDFS. One method is to calculate the sum of the z-transformed gene expression. The mean and standard deviation used for the z-transformation can be calculated from the training data, i.e., the PENELOPE-B cohort. The following gene list shows the parameters for such a predictor based on Table 7.

**Table 13: Linear predictor from z-transformation on Table 7**

| Variable / gene | mean | standard deviation |
|---|---|---|
| CDK5R1 | 6.19 | 0.71 |
| DNAJC14 | 7.43 | 0.41 |
| DUSP8 | 6.63 | 0.76 |
| GNGT2 | 4.45 | 1.05 |
| IL18 | 6.59 | 0.86 |
| LIG4 | 6.60 | 0.70 |
| MADD | 7.69 | 0.51 |
| MTDH | 6.03 | 0.96 |
| NR1H3 | 5.90 | 0.81 |
| NRAS | 8.26 | 0.52 |
| NSD1 | 8.18 | 0.47 |
| NUP62 | 9.05 | 0.46 |
| OGG1 | 7.09 | 0.62 |
| PFKL | 9.02 | 0.74 |
| PIM2 | 6.81 | 1.08 |
| PITRM1 | 6.76 | 0.74 |
| PLK4 | 7.00 | 0.77 |
| POLR2D | 7.68 | 0.55 |
| PRDX6 | 9.02 | 0.73 |
| RIPK2 | 5.57 | 1.14 |
| RPS4X | 8.87 | 0.87 |
| SAP30 | 6.44 | 0.69 |
| STK36 | 6.93 | 0.64 |
| TBP | 4.92 | 1.32 |

Genes are good prognosis genes: The higher the score (sum of z-transformed gene expressions using z-transform parameters from the table above), the lower the likelihood of an iDFS event.

Figure 4 shows the outcome of patients with negative and positive score.

### Example 12

To predict the outcome (e.g., the likelihood of an iDFS event) of a new, future patient based on example 1 a mathematical algorithm can be constructed comprising of the following steps:
1. measure the expression of the genes in Table 1.1 (same technical platform) for the new patient,
2. add the new patient to the training patient set (PENELOPE-B) and perform an unsupervised clustering of the combined patient set ("prediction clusters") as described in example 1,
3. determine the set of training (PENELOPE-B) patients in the same "prediction" cluster as the new patient,
4. within the training patients in said same cluster calculate the frequency of "training" clusters AC-1 through AC-5 these patients were assigned to in example 1,
5. assign the most frequent "training" cluster (AC-1, ..., AC-5) to the new patient, and assign the outcome prediction for the new patient based on the "training" cluster risk from figure 2.

Simulation studies showed that this algorithm is robust, i.e., in the last step the most frequent "training" cluster in the "prediction" of the new patient is the one assigned to at least 80% of the training patients.

### Example 13

There are many alternative mathematical methods to predict outcome for a new patient, including non-linear models. Examples are:
- regression trees where each tree node uses the expression of one gene from Table 2,
- random forests including several regression trees,
- neural networks with gene expressions as input variables,
- nearest neighbor and shrunken centroid classifiers,
- support vector machines.

These methods can be combined with several methods for gene selection ("feature or variable selection").

### Example 14

To evaluate the predictive capacity of small gene subsets (3, 5, 10, 20 or 30 genes) in classifying samples into molecular subtypes (AC-1 to AC-5), a supervised nearest centroid classifier was implemented. Gene expression data was used as input and samples were annotated by subtype labels (AC-1 to AC-5). Classification was based on bootstrap approach with 100 iterations, where each iteration randomly selected 3, 5, 10, 20 or 30 genes from the 335 genes (i.e., the markers as shown in Table 1.1). Bootstrapping involves repeatedly sampling subsets of genes with replacement to create multiple training sets, enabling assessment of classifier robustness and variability across different gene combinations. This bootstrapping technique enhances the reliability of the classifier and at the same time reducing the overfitting. For each selected subset, centroids representing the mean expression profile of each subtype were computed. Samples were then classified by assigning them to the nearest centroid using Euclidean distance. Classification performance was assessed using overall accuracy, confusion matrices and performance metrics (sensitivity, specificity, and F1 score).

The gene sets were ranked by accuracy, and the most predictive subsets were analyzed for recurring gene patterns. For the most predictive subsets, a confusion matrix is provided (see

Tables 15, 17, 19, 21, and 23). Each column of the confusion matrices represents the instances in the actual adaptive cluster (AC) (= adaptive breast cancer subtype) while each row represents the instances in the predicted AC. The actual numbers of the ACs are: AC-1: 137, AC-2: 142, AC-3: 132, AC-4: 117, and AC-5: 13.

To assign new data points to clusters, a distance-based approach was used that evaluated how close each point is to predefined centroids. The method relies on calculating squared differences to determine proximity, ensuring each point is matched with the most appropriate cluster.

### A) 3 Genes

Figure 5, Table 14 show that randomly selected sets of 3 genes, wherein the genes are selected from Table 1.1, can predict the adaptive breast cancer subtype clusters using a centroid approach. For each gene set, 100 iterations were performed, and the accuracy results are presented in a line plot (Figure 5A). List of the 20 genes that are present in 1 or 2 gene sets (Figure 5B). The top 20 gene sets are shown with gene symbols (Table 14). For the top 5 sets, confusion matrix along with other performance metrics (accuracy, specificity, sensitivity and F1) are shown (Table 15).

**Table 14: 20 randomly selected sets of 3 genes for prediction of AC clusters**

| | **Iteration** | **Accuracy** | **Genes** |
|---|---|---|---|
| Set 1 | 4 | 0,639 | TESC,BUB1,TWIST2 |
| Set 2 | 99 | 0,619 | CEBPD,PRC1,HERC3 |
| Set 3 | 75 | 0,593 | NSD1,MTDH,TWIST2 |
| Set 4 | 92 | 0,591 | HJURP,RGS2,KLF4 |
| Set 5 | 32 | 0,561 | PRDX6,PTGS2,KIF2C |
| Set 6 | 70 | 0,533 | CDKN1C,NSD1,EMP1 |
| Set 7 | 48 | 0,524 | RGS2,CD209,DUSP8 |
| Set 8 | 79 | 0,519 | CDC20,GATA3,SAP30 |
| Set 9 | 83 | 0,515 | FN1,NR1H3,EZH2 |
| Set 10 | 97 | 0,513 | GATA3,EGFR,FOXC1 |
| Set 11 | 10 | 0,511 | TBP,TNFAIP3,HMGB3 |
| Set 12 | 2 | 0,509 | DLGAP5,PIK3R2,IL20 |
| Set 13 | 27 | 0,509 | PPP2R4,CXCL16,SPHK1 |
| Set 14 | 98 | 0,509 | NR1H3,DLC1,IL33 |
| Set 15 | 96 | 0,507 | TK1,HSPA14,NOLC1 |
| Set 16 | 17 | 0,506 | CCNE1,FLNC,PIK3R2 |
| Set 17 | 66 | 0,504 | EGR3,PFKL,CXCL2 |
| Set 18 | 13 | 0,498 | PGR,OGG1,PPP2R2C |
| Set 19 | 22 | 0,494 | HBEGF,EGFR,TUBB3 |
| Set 20 | 42 | 0,493 | CYP1A1,TMPRSS2,JMJD1C |

**Table 15: Top 5 sets of 3 genes for prediction of AC clusters**

| **Set 1** | | **AC-1** | **AC-2** | **AC-3** | **AC-4** | **AC-5** | | **Sensitivity** | **Specificity** | **F1** |
|---|---|---|---|---|---|---|---|---|---|---|
| | **AC-1** | 77 | 28 | 11 | 5 | 4 | **AC-1** | 0,562 | 0,881 | 0,588 |
| | **AC-2** | 21 | 98 | 0 | 10 | 0 | **AC-2** | 0,690 | 0,922 | 0,723 |
| | **AC-3** | 12 | 2 | 78 | 17 | 2 | **AC-3** | 0,595 | 0,919 | 0,645 |
| | **AC-4** | 3 | 13 | 15 | 85 | 0 | **AC-4** | 0,726 | 0,927 | 0,730 |
| | **AC-5** | 24 | 1 | 27 | 0 | 7 | **AC-5** | 0,538 | 0,901 | 0,194 |
| | | | | | | | | | | |

| **Set 2** | | **AC-1** | **AC-2** | **AC-3** | **AC-4** | **AC-5** | | **Sensitivity** | **Specificity** | **F1** |
|---|---|---|---|---|---|---|---|---|---|---|
| | **AC-1** | 93 | 36 | 11 | 1 | 1 | **AC-1** | 0,679 | 0,878 | 0,667 |
| | **AC-2** | 24 | 86 | 3 | 1 | 0 | **AC-2** | 0,606 | 0,930 | 0,672 |
| | **AC-3** | 7 | 3 | 58 | 20 | 4 | **AC-3** | 0,443 | 0,917 | 0,520 |
| | **AC-4** | 0 | 4 | 18 | 91 | 2 | **AC-4** | 0,778 | 0,943 | 0,784 |
| | **AC-5** | 13 | 13 | 41 | 4 | 6 | **AC-5** | 0,462 | 0,865 | 0,133 |
| | | | | | | | | | | |

| **Set 3** | | **AC-1** | **AC-2** | **AC-3** | **AC-4** | **AC-5** | | **Sensitivity** | **Specificity** | **F1** |
|---|---|---|---|---|---|---|---|---|---|---|
| | **AC-1** | 100 | 32 | 22 | 2 | 1 | **AC-1** | 0,730 | 0,859 | 0,680 |
| | **AC-2** | 27 | 86 | 4 | 11 | 0 | **AC-2** | 0,606 | 0,894 | 0,637 |
| | **AC-3** | 7 | 16 | 55 | 19 | 2 | **AC-3** | 0,420 | 0,892 | 0,478 |
| | **AC-4** | 0 | 5 | 29 | 71 | 2 | **AC-4** | 0,607 | 0,915 | 0,634 |
| | **AC-5** | 3 | 3 | 21 | 14 | 8 | **AC-5** | 0,615 | 0,922 | 0,258 |
| | | | | | | | | | | |

| **Set 4** | | **AC-1** | **AC-2** | **AC-3** | **AC-4** | **AC-5** | | **Sensitivity** | **Specificity** | **F1** |
|---|---|---|---|---|---|---|---|---|---|---|
| | **AC-1** | 79 | 21 | 17 | 22 | 0 | **AC-1** | 0,577 | 0,851 | 0,572 |
| | **AC-2** | 13 | 88 | 3 | 21 | 0 | **AC-2** | 0,620 | 0,907 | 0,659 |
| | **AC-3** | 9 | 2 | 84 | 13 | 3 | **AC-3** | 0,641 | 0,934 | 0,694 |
| | **AC-4** | 17 | 28 | 15 | 58 | 0 | **AC-4** | 0,496 | 0,858 | 0,494 |
| | **AC-5** | 19 | 3 | 12 | 3 | 10 | **AC-5** | 0,769 | 0,930 | 0,333 |
| | | | | | | | | | | |

| **Set 5** | | **AC-1** | **AC-2** | **AC-3** | **AC-4** | **AC-5** | | **Sensitivity** | **Specificity** | **F1** |
|---|---|---|---|---|---|---|---|---|---|---|
| | **AC-1** | 89 | 32 | 14 | 0 | 3 | **AC-1** | 0,650 | 0,878 | 0,647 |
| | **AC-2** | 32 | 93 | 8 | 8 | 0 | **AC-2** | 0,655 | 0,879 | 0,657 |
| | **AC-3** | 6 | 7 | 37 | 29 | 1 | **AC-3** | 0,282 | 0,895 | 0,351 |
| | **AC-4** | 0 | 8 | 29 | 76 | 1 | **AC-4** | 0,650 | 0,910 | 0,658 |
| | **AC-5** | 10 | 2 | 43 | 4 | 8 | **AC-5** | 0,615 | 0,888 | 0,200 |

### B) 5 Genes

Figure 6 and Table 16 show that randomly selected sets of 5 genes, wherein the genes are selected from Table 1.1, can predict the adaptive breast cancer subtype clusters using a centroid approach. For each gene set, 100 iterations were performed, and the accuracy results are presented in a line plot (Figure 6A). List of the 20 genes that are present in 1, 2 or 3 gene sets (Figure 6B). The top 20 gene sets are shown with gene symbols (Table 16). For the top 5 sets, confusion matrix along with other performance metrics (accuracy, specificity, sensitivity and F1) are shown (Table 17).

**Table 16: 20 randomly selected sets of 5 genes for prediction of AC clusters**

| | **Iteration** | **Accuracy** | **Genes** |
|---|---|---|---|
| Set 1 | 6 | 0,728 | BUB1,KIT,TBP,TNFAIP3,HMGB3 |
| Set 2 | 69 | 0,683 | HES1,CYP1B1,MT1A,COL1A2,GSN |
| Set 3 | 77 | 0,681 | E2F1,GADD45A,PABPC1,ANXA3,POLR2D |
| Set 4 | 60 | 0,669 | PRC1,HERC3,IRF7,CXCL2,BMI1 |
| Set 5 | 55 | 0,661 | TCF7L1,MADD,RAB25,HJURP,RGS2 |
| Set 6 | 98 | 0,644 | BMI1,NUP62,FLNC,POLR2D,NCAPD3 |
| Set 7 | 99 | 0,630 | POLD2,RPS4X,SGK1,GAS1,RUNX1T1 |
| Set 8 | 83 | 0,624 | NCAPD3,ALDH1A1,HJURP,IL33,TCF7L1 |
| Set 9 | 8 | 0,619 | ADAMTS1,PGR,OGG1,PPP2R2C,GAS1 |
| Set 10 | 58 | 0,619 | TK1,HSPA14,NOLC1,GATA3,EGFR |
| Set 11 | 76 | 0,619 | OGG1,COL4A3,CSF2,CAV1,PGR |
| Set 12 | 45 | 0,615 | STEAP4,IL4,NSD1,MTDH,TWIST2 |
| Set 13 | 54 | 0,615 | PFKL,NRIP1,SRC,FAS,STEAP4 |
| Set 14 | 79 | 0,615 | RASSF7,SAP30,SOCS3,PRL,DLGAP5 |
| Set 15 | 75 | 0,609 | WNT2,NR1H3,GADD45A,GNGT2,FOXA1 |
| Set 16 | 13 | 0,607 | PRDX6,TUBB3,GLUL,HBEGF,EGFR |
| Set 17 | 21 | 0,604 | ANXA1,GADD45B,RHOU,TBP,NTHL1 |
| Set 18 | 29 | 0,604 | HSPA14,RGS2,CD209,DUSP8,ANXA1 |
| Set 19 | 81 | 0,604 | SMAD9,BUB1,E2F1,APOC2,PPAP2B |
| Set 20 | 19 | 0,593 | TBP,CDKN1A,XBP1,PRDX6,PTGS2 |

**Table 17: Top 5 sets of 5 genes for prediction of AC clusters**

| **Set 1** | | **AC-1** | **AC-2** | **AC-3** | **AC-4** | **AC-5** | | **Sensitivity** | **Specificity** | **F1** |
|---|---|---|---|---|---|---|---|---|---|---|
| | **AC-1** | 106 | 30 | 10 | 0 | 1 | **AC-1** | 0,774 | 0,898 | 0,746 |
| | **AC-2** | 24 | 97 | 3 | 9 | 0 | **AC-2** | 0,683 | 0,910 | 0,705 |
| | **AC-3** | 5 | 3 | 93 | 20 | 2 | **AC-3** | 0,710 | 0,927 | 0,732 |
| | **AC-4** | 0 | 12 | 13 | 87 | 0 | **AC-4** | 0,744 | 0,941 | 0,760 |
| | **AC-5** | 2 | 0 | 12 | 1 | 10 | **AC-5** | 0,769 | 0,972 | 0,526 |
| | | | | | | | | | | |

| **Set 2** | | **AC-1** | **AC-2** | **AC-3** | **AC-4** | **AC-5** | | **Sensitivity** | **Specificity** | **F1** |
|---|---|---|---|---|---|---|---|---|---|---|
| | **AC-1** | 95 | 18 | 11 | 0 | 2 | **AC-1** | 0,693 | 0,923 | 0,722 |
| | **AC-2** | 28 | 104 | 7 | 4 | 0 | **AC-2** | 0,732 | 0,902 | 0,730 |
| | **AC-3** | 8 | 7 | 69 | 13 | 3 | **AC-3** | 0,527 | 0,924 | 0,597 |
| | **AC-4** | 0 | 8 | 24 | 96 | 3 | **AC-4** | 0,821 | 0,917 | 0,774 |
| | **AC-5** | 6 | 5 | 20 | 4 | 5 | **AC-5** | 0,385 | 0,934 | 0,189 |
| | | | | | | | | | | |

| **Set 3** | | **AC-1** | **AC-2** | **AC-3** | **AC-4** | **AC-5** | | **Sensitivity** | **Specificity** | **F1** |
|---|---|---|---|---|---|---|---|---|---|---|
| | **AC-1** | 107 | 23 | 16 | 0 | 0 | **AC-1** | 0,781 | 0,903 | 0,756 |
| | **AC-2** | 20 | 87 | 5 | 5 | 0 | **AC-2** | 0,613 | 0,925 | 0,672 |
| | **AC-3** | 7 | 11 | 78 | 18 | 1 | **AC-3** | 0,595 | 0,910 | 0,634 |
| | **AC-4** | 0 | 13 | 19 | 87 | 3 | **AC-4** | 0,744 | 0,917 | 0,728 |
| | **AC-5** | 3 | 8 | 13 | 7 | 9 | **AC-5** | 0,692 | 0,941 | 0,340 |
| | | | | | | | | | | |

| **Set 4** | | **AC-1** | **AC-2** | **AC-3** | **AC-4** | **AC-5** | | **Sensitivity** | **Specificity** | **F1** |
|---|---|---|---|---|---|---|---|---|---|---|
| | **AC-1** | 95 | 29 | 13 | 0 | 0 | **AC-1** | 0,693 | 0,896 | 0,693 |
| | **AC-2** | 27 | 92 | 4 | 2 | 1 | **AC-2** | 0,648 | 0,915 | 0,687 |
| | **AC-3** | 10 | 4 | 77 | 19 | 2 | **AC-3** | 0,588 | 0,914 | 0,634 |
| | **AC-4** | 0 | 7 | 13 | 88 | 1 | **AC-4** | 0,752 | 0,950 | 0,779 |
| | **AC-5** | 5 | 10 | 24 | 8 | 9 | **AC-5** | 0,692 | 0,911 | 0,261 |
| | | | | | | | | | | |

| **Set 5** | | **AC-1** | **AC-2** | **AC-3** | **AC-4** | **AC-5** | | **Sensitivity** | **Specificity** | **F1** |
|---|---|---|---|---|---|---|---|---|---|---|
| | **AC-1** | 89 | 24 | 21 | 8 | 0 | **AC-1** | 0,650 | 0,868 | 0,638 |
| | **AC-2** | 17 | 96 | 2 | 10 | 0 | **AC-2** | 0,676 | 0,927 | 0,719 |
| | **AC-3** | 14 | 2 | 80 | 18 | 0 | **AC-3** | 0,611 | 0,917 | 0,653 |
| | **AC-4** | 16 | 20 | 17 | 80 | 1 | **AC-4** | 0,684 | 0,872 | 0,637 |
| | **AC-5** | 1 | 0 | 11 | 1 | 12 | **AC-5** | 0,923 | 0,975 | 0,632 |

### C) 10 Genes

Figure 7, Table 18 show that randomly selected sets of 10 genes, wherein the genes are selected from Table 1.1, can predict the adaptive breast cancer subtype clusters using a centroid approach. For each gene set, 100 iterations were performed, and the accuracy results are presented in a line plot (Figure 7A). List of the 20 genes that are present in 4 or 5 gene sets (Figure 7B). The top 20 gene sets are shown with gene symbols (Table 18). For the top 5 sets, confusion matrix along with other performance metrics (accuracy, specificity, sensitivity and F1) are shown (Table 19).

**Table 18: 20 randomly selected sets of 10 genes for prediction of AC clusters**

| | **Iteration** | **Accuracy** | **Genes** |
|---|---|---|---|
| Set 1 | 90 | 0,815 | MKI67, GSN, DLC1, SRC, FOXO1, NUSAP1, KRT17, HSPB6, TPX2, CXCL2 |
| Set 2 | 10 | 0,798 | TBP, CDKN1A, XBP1, PRDX6, PTGS2, KIF2C, C3, MYBL1, CCNB1, PMAIP1 |
| Set 3 | 68 | 0,796 | POLR2D, IL18, E2F1, MEIS1, C3, NCAPD3, IGF2, CREB5, MADD, MMP11 |
| Set 4 | 38 | 0,785 | GNGT2, FOXA1, OGG1, COL4A3, CSF2, CAV1, PGR, E2F1, GADD45A, PABPC1 |
| Set 5 | 94 | 0,785 | XBP1, EGFR, HERC3, OASL, UBE2T, KRT17, ALDH1A3, ANLN, FOXO1, PKM |
| Set 6 | 67 | 0,781 | HJURP, C3, BUB1, RASD1, TRIB1, STEAP4, RPS4X, NRIP1, IL18, MST1 |
| Set 7 | 60 | 0,774 | PPP2R2C, JMJD1C, MADD, CCT5, GSN, SPC25, TFF1, ASPM, IRF7, JUNB |
| Set 8 | 69 | 0,759 | PMAIP1, CYP1B1, LEPR, STAT1, CDC6, MT1X, PRDX6, STK11, SPHK1, TCF4 |
| Set 9 | 30 | 0,754 | FOXC1, NR1H3, DLC1, IL33, CEBPD, PRC1, HERC3, IRF7, CXCL2, BMI1 |
| Set 10 | 50 | 0,754 | RUNX1T1, NSD1, TNXB, SELE, KRT5, MEIS1, GADD45G, CCNB1, MTDH, COL4A3 |
| Set 11 | 1 | 0,748 | CITED2, TRAF2, JUN, DLGAP5, PIK3R2, IL20, GPR160, ZFP36L1, GZMB, TESC |
| Set 12 | 91 | 0,741 | FAM105A, MT1X, NTHL1, CNTFR, EXO1, LPL, TIPARP, NUSAP1, PLK1, TESC |
| Set 13 | 73 | 0,739 | LIG4, ANXA1, CXCL2, DLGAP5, VCAM1, LRP2, ZFP36L1, DES, RPS4X, KLF4 |
| Set 14 | 54 | 0,735 | DAB2, SELE, RELN, ASPM, PPAP2A, MAPK10, GATA3, CCL2, IL4, THBS2 |
| Set 15 | 28 | 0,726 | TCF7L1, MADD, RAB25, HJURP, RGS2, KLF4, IGF1, CCL2, COL4A3, ABCD1 |
| Set 16 | 52 | 0,724 | MAP1B, NUP62, TNFRSF1B, GLUL, SFRP1, CDC6, NR4A3, PDPK1, KRT5, IL4 |
| Set 17 | 27 | 0,722 | SPRY1, TIPARP, SLIT2, PPID, NR4A1, PFKL, NRIP1, SRC, FAS, STEAP4 |
| Set 18 | 75 | 0,722 | KLF4, MEIS1, CD209, MTDH, CXCR4, RGS2, AQP1, CDKN1C, CCNE1, EGFR |
| Set 19 | 15 | 0,715 | HSPA14, RGS2, CD209, DUSP8, ANXA1, KDM4B, FGF14, PIK3R3, MKI67, GTSE1 |
| Set 20 | 29 | 0,715 | CAV2, GADD45B, OAS1, PLCG1, RRM2, TK1, HSPA14, NOLC1, GATA3, EGFR |

**Table 19: Top 5 sets of 10 genes for prediction of AC clusters**

| **Set 1** | | **AC-1** | **AC-2** | **AC-3** | **AC-4** | **AC-5** | | **Sensitivity** | **Specificity** | **F1** |
|---|---|---|---|---|---|---|---|---|---|---|
| | **AC-1** | 114 | 17 | 7 | 2 | 0 | **AC-1** | 0,832 | 0,935 | 0,823 |
| | **AC-2** | 13 | 108 | 3 | 6 | 0 | **AC-2** | 0,761 | 0,945 | 0,794 |
| | **AC-3** | 10 | 2 | 106 | 8 | 0 | **AC-3** | 0,809 | 0,951 | 0,825 |
| | **AC-4** | 0 | 13 | 9 | 99 | 0 | **AC-4** | 0,846 | 0,948 | 0,832 |
| | **AC-5** | 0 | 2 | 6 | 2 | 13 | **AC-5** | 1,000 | 0,981 | 0,722 |
| | | | | | | | | | | |

| **Set 2** | | **AC-1** | **AC-2** | **AC-3** | **AC-4** | **AC-5** | | **Sensitivity** | **Specificity** | **F1** |
|---|---|---|---|---|---|---|---|---|---|---|
| | **AC-1** | 113 | 18 | 6 | 0 | 0 | **AC-1** | 0,825 | 0,940 | 0,825 |
| | **AC-2** | 19 | 122 | 2 | 4 | 0 | **AC-2** | 0,859 | 0,937 | 0,844 |
| | **AC-3** | 3 | 0 | 97 | 23 | 1 | **AC-3** | 0,740 | 0,934 | 0,761 |
| | **AC-4** | 0 | 1 | 20 | 89 | 2 | **AC-4** | 0,761 | 0,946 | *0,777* |
| | **AC-5** | 2 | 1 | 6 | 1 | 10 | **AC-5** | 0,769 | 0,981 | 0,606 |
| | | | | | | | | | | |
| **Set 3** | | **AC-1** | **AC-2** | **AC-3** | **AC-4** | **AC-5** | | **Sensitivity** | **Specificity** | **F1** |
| | **AC-1** | 113 | 14 | 5 | 0 | 0 | **AC-1** | 0,825 | 0,953 | 0,840 |
| | **AC-2** | 19 | 120 | 1 | 2 | 0 | **AC-2** | 0,845 | 0,945 | 0,845 |
| | **AC-3** | 2 | 0 | 91 | 16 | 1 | **AC-3** | 0,695 | 0,954 | 0,755 |
| | **AC-4** | 0 | 4 | 14 | 96 | 2 | **AC-4** | 0,821 | 0,953 | 0,824 |
| | **AC-5** | 3 | 4 | 20 | 3 | 10 | **AC-5** | 0,769 | 0,943 | 0,377 |
| | | | | | | | | | | |
| **Set 4** | | **AC-1** | **AC-2** | **AC-3** | **AC-4** | **AC-5** | | **Sensitivity** | **Specificity** | **F1** |
| | **AC-1** | 116 | 26 | 12 | 0 | 1 | **AC-1** | 0,847 | 0,903 | 0,795 |
| | **AC-2** | 18 | 110 | 0 | 7 | 0 | **AC-2** | 0,775 | 0,937 | 0,794 |
| | **AC-3** | 3 | 3 | 98 | 21 | 0 | **AC-3** | 0,748 | 0,934 | 0,766 |
| | **AC-4** | 0 | 3 | 21 | 89 | 1 | **AC-4** | 0,761 | 0,941 | 0,771 |
| | **AC-5** | 0 | 0 | 0 | 0 | 11 | **AC-5** | 0,846 | 1,000 | 0,917 |
| | | | | | | | | | | |
| **Set 5** | | **AC-1** | **AC-2** | **AC-3** | **AC-4** | **AC-5** | | **Sensitivity** | **Specificity** | **F1** |
| | **AC-1** | 112 | 16 | 15 | 0 | 1 | **AC-1** | 0,818 | 0,921 | 0, 797 |
| | **AC-2** | 18 | 118 | 1 | 5 | 0 | **AC-2** | 0,831 | 0,940 | 0,831 |
| | **AC-3** | 6 | 5 | 94 | 21 | 1 | **AC-3** | 0,718 | 0,919 | 0,729 |
| | **AC-4** | 1 | 2 | 19 | 90 | 1 | **AC-4** | 0,769 | 0,946 | 0,783 |
| | **AC-5** | 0 | 1 | 2 | 1 | 10 | **AC-5** | 0,769 | 0,992 | 0,741 |

### D) 20 Genes

Figure 8 and Table 220 show that randomly selected sets of 20 genes, wherein the genes are selected from Table 1.1, can predict the adaptive breast cancer subtype clusters a using centroid approach. For each gene set, 100 iterations were performed, and the accuracy results are presented in a line plot (Figure 8A). List of the 20 genes that are present in 3, 4 or 5 gene sets (Figure 8B). The top 20 gene sets are shown with gene symbols (Table 20). For the top 5 sets, confusion matrix along with other performance metrics (accuracy, specificity, sensitivity and F1) are shown (Table 21).

**Table 20: 20 randomly selected sets of 20 genes for prediction of AC clusters**

| | **Iteration** | **Accuracy** | **Genes** |
|---|---|---|---|
| Set 1 | 95 | 0,893 | TESC, EGFR, PITRM1, PLK1, CXCL9, CNTFR, EGR1, SLC27A4, MAGEL2, FAT4, RRM2, IL6, COL4A3, C3, SOCS3, POLR2D, RASSF7, NSD1, RGS2, STK11 |
| Set 2 | 15 | 0,863 | PLCG1, RRM2, TK1, HSPA14, NOLC1, GATA3, EGFR, FOXC1, NR1H3, DLC1, IL33, CEBPD, PRC1, HERC3, IRF7, CXCL2, BMI1, LTB, AQP1, TXNIP |
| Set 3 | 87 | 0,859 | KRT5, FAM105A, ADAMTS1, SPHK1, PIK3R3, RET, RHOU, NOLC1, CCT5, BUB1, GADD45G, CD34, IGF2, ANLN, PCOLCE, PPAP2B, DAB2, TBP, SYCP2, IL6 |
| Set 4 | 60 | 0,857 | TUBB3, TFF1, SERPINE1, SPRY2, ABCA3, CCNB1, LYVE1, PFKL, HERC3, RELN, CXCL10, GAPDH, CNTFR, SLC2A3, TK1, TEK, ADIPOR1, TGFB3, AKR1C3, AKT3 |
| Set 5 | 81 | 0,856 | ANXA3, TPX2, HMGCS2, COL1A2, SPRY1, PLCG1, PCNA, ANXA1, LRP2, TBP, DKK2, TNFAIP3, NR4A1, TCF4, LYVE1, HBEGF, FAM105A, BUB1, PTGS2, SERTAD1 |
| Set 6 | 25 | 0,854 | FLNC, POLR2D, NCAPD3, POLD2, RPS4X, SGK1, GAS1, RUNX1T1, NSD1, TNXB, SELE, KRT5, MEIS1, GADD45G, CCNB1, MTDH, COL4A3, H2AFX, ADM, NR4A1 |
| Set 7 | 27 | 0,854 | FGF14, MMP2, CEBPD, PRDX6, XBP1, FGF2, TOP2A, DAB2, SELE, RELN, ASPM, PPAP2A, MAPK10, GATA3, CCL2, IL4, THBS2, KRT19, SLC19A3, MEIS1 |
| Set 8 | 52 | 0,848 | FOXA1, THBS4, GTSE1, TNFRSF1B, PLCG1, TAP1, MYBL2, FOXO1, AQP1, COL6A6, GADD45A, PRDX6, FANCA, IRF7, PRL, MYBL1, DAB2, ORC6, NR4A3, SOCS3 |
| Set 9 | 57 | 0,846 | GSN, ANGPTL1, MAFF, RASSF7, MT1X, TESC, DNAJC22, GADD45G, POLR2D, TGFB3, VCAM1, MTDH, TWIST1, NR1H3, CCNA2, NUF2, ADAMTS1, DNAJC14, STMN1, DLGAP5 |
| Set 10 | 69 | 0,846 | PPAP2A, XBP1, PCSK6, TCF4, OAS1, GTSE1, EXO1, DES, KRT19, IGF2, TNN, CTGF, HMGB3, KLF4, TPX2, GAS1, HERC3, SMAD9, KRT14, MTDH |
| Set 11 | 14 | 0,841 | PPID, NR4A1, PFKL, NRIP1, SRC, FAS, STEAP4, TCF7L1, MADD, RAB25, HJURP, RGS2, KLF4, IGF1, CCL2, COL4A3, ABCD1, CAV2, GADD45B, OAS1 |
| Set 12 | 47 | 0,841 | GNGT2, CACNG4, TNC, PRKCZ, SOX17, EGFR, HERC3, OASL, KRT17, ALDH1A3, ANLN, FOXO1, PKM, CD34, ANXA1, DAB2, MMP9, EGR3, PPAP2B, OGG1 |
| Set 13 | 56 | 0,839 | FOXA1, PIK3R3, FAM105A, KRT5, TUBB3, CAV2, NR4A1, CEBPD, LIF, STK36, HES1, CTGF, ID4, TYMS, PLK4, CXCL9, PRKCZ, GADD45G, CD34, HBEGF |
| Set 14 | 5 | 0,837 | GADD45G, DKK2, SPHK1, ABCA3, IGF1, NRIP1, TMEM132A, TBP, CDKN1A, TWIST1, PRDX6, PTGS2, KIF2C, C3, MYBL1, CCNB1, PMAIP1, ANXA1, GADD45B, RHOU |
| Set 15 | 34 | 0,837 | TRIB1, STEAP4, RPS4X, NRIP1, IL18, MST1, POLR2D, UBE2T, E2F1, MEIS1, C3, NCAPD3, IGF2, CREB5, MADD, MMP11, PMAIP1, CYP1B1, LEPR, STAT1 |
| Set 16 | 55 | 0,837 | TESC, ALDH1A3, PLAU, THBS2, MAPK10, IGFBP6, POLD1, EGR1, EXO1, GNGT2, C3, ACKR1, SFN, SLC19A3, PLK4, CAV1, KLF4, PPAP2A, TNFAIP3, NPC1 |
| Set 17 | 58 | 0,837 | E2F1, NOLC1, CD209, PTN, PPAP2A, SLC19A3, CXCL2, PCNA, SPHK1, STK11, NRAS, CYR61, MAML2, AKR1C3, PIAS4, GNGT2, PRC1, HSPA14, CDC6, SFRP1 |
| Set 18 | 100 | 0,837 | STK36, DUSP8, IL18, JUND, SLC16A3, PPAP2A, NSD1, SAP30, PDZK1, FOXA1, RHOU, CAV2, MYBL2, TGFB3, DNAJC14, PKMYT1, EGR3, FBXW7, KRT14, COL1A2 |
| Set 19 | 37 | 0,835 | DLGAP5, LRP2, LIG4, DES, RPS4X, KLF4, FABP4, MYB, SLIT2, STK11, TNFAIP3, EGR3, ALDH1A3, POLR2D, MADD, HES1, TYMS, MEIS1, CD209, MTDH |
| Set 20 | 46 | 0,835 | LPL, TIPARP, NUSAP1, PLK1, TESC, SRC, NSD1, DKK2, ADIPOR1, CDKN1C, PDPK1, MEIS1, FIGF, RASD1, LIF, PCOLCE, SOX17, MAP1B, IL18, CXCL3 |

**Table 21: Top 5 sets of 20 genes for prediction of AC clusters**

| **Set 1** | | **AC-1** | **AC-2** | **AC-3** | **AC-4** | **AC-5** | | **Sensitivity** | **Specificity** | **F1** |
|---|---|---|---|---|---|---|---|---|---|---|
| | **AC-1** | 129 | 10 | 5 | 0 | 1 | **AC-1** | 0,942 | 0,960 | 0,915 |
| | **AC-2** | 8 | 129 | 1 | 0 | 0 | **AC-2** | 0,908 | 0,977 | 0,921 |
| | **AC-3** | 0 | 1 | 110 | 13 | 1 | **AC-3** | 0,840 | 0,963 | 0,859 |
| | **AC-4** | 0 | 1 | 13 | 103 | 0 | **AC-4** | 0,880 | 0,967 | 0,880 |
| | **AC-5** | 0 | 1 | 2 | 1 | 11 | **AC-5** | 0,846 | 0,992 | 0,786 |
| | | | | | | | | | | |

| **Set 2** | | **AC-1** | **AC-2** | **AC-3** | **AC-4** | **AC-5** | | **Sensitivity** | **Specificity** | **F1** |
|---|---|---|---|---|---|---|---|---|---|---|
| | **AC-1** | 122 | 20 | 7 | 0 | 0 | **AC-1** | 0,891 | 0,933 | 0,853 |
| | **AC-2** | 13 | 117 | 0 | 1 | 0 | **AC-2** | 0,824 | 0,965 | 0,857 |
| | **AC-3** | 2 | 0 | 109 | 11 | 0 | **AC-3** | 0,832 | 0,968 | 0,862 |
| | **AC-4** | 0 | 5 | 15 | 105 | 0 | **AC-4** | 0,897 | 0,953 | 0,868 |
| | **AC-5** | 0 | 0 | 0 | 0 | 13 | **AC-5** | 1,000 | 1,000 | 1,000 |
| | | | | | | | | | | |

| **Set 3** | | **AC-1** | **AC-2** | **AC-3** | **AC-4** | **AC-5** | | **Sensitivity** | **Specificity** | **F1** |
|---|---|---|---|---|---|---|---|---|---|---|
| | **AC-1** | 119 | 18 | 4 | 0 | 2 | **AC-1** | 0,869 | 0,940 | 0,850 |
| | **AC-2** | 13 | 118 | 0 | 1 | 0 | **AC-2** | 0,831 | 0,965 | 0,861 |
| | **AC-3** | 4 | 3 | 115 | 14 | 0 | **AC-3** | 0,878 | 0,949 | 0,861 |
| | **AC-4** | 0 | 2 | 7 | 101 | 0 | **AC-4** | 0,863 | 0,979 | 0,890 |
| | **AC-5** | 1 | 1 | 5 | 1 | 11 | **AC-5** | 0,846 | 0,985 | 0,688 |
| | | | | | | | | | | |

| **Set 4** | | **AC-1** | **AC-2** | **AC-3** | **AC-4** | **AC-5** | | **Sensitivity** | **Specificity** | **F1** |
|---|---|---|---|---|---|---|---|---|---|---|
| | **AC-1** | 117 | 20 | 3 | 0 | 0 | **AC-1** | 0,854 | 0,943 | 0,845 |
| | **AC-2** | 15 | 116 | 0 | 1 | 0 | **AC-2** | 0,817 | 0,960 | 0,847 |
| | **AC-3** | 3 | 0 | 114 | 13 | 0 | **AC-3** | 0,870 | 0,961 | 0,874 |
| | **AC-4** | 1 | 2 | 9 | 103 | 0 | **AC-4** | 0,880 | 0,972 | 0,888 |
| | **AC-5** | 1 | 4 | 5 | 0 | 13 | **AC-5** | 1,000 | 0,981 | 0,722 |
| | | | | | | | | | | |

| **Set 5** | | **AC-1** | **AC-2** | **AC-3** | **AC-4** | **AC-5** | | **Sensitivity** | **Specificity** | **F1** |
|---|---|---|---|---|---|---|---|---|---|---|
| | **AC-1** | 123 | 16 | 8 | 0 | 1 | **AC-1** | 0,898 | 0,938 | 0,863 |
| | **AC-2** | 13 | 120 | 0 | 2 | 0 | **AC-2** | 0,845 | 0,962 | 0,866 |
| | **AC-3** | 1 | 3 | 108 | 15 | 1 | **AC-3** | 0,824 | 0,951 | 0,834 |
| | **AC-4** | 0 | 3 | 12 | 100 | 0 | **AC-4** | 0,855 | 0,965 | 0,862 |
| | **AC-5** | 0 | 0 | 3 | 0 | 11 | **AC-5** | 0,846 | 0,994 | 0,815 |

### E) 30 Genes

Figure 9 and Table 22 show that randomly selected sets of 30 genes, wherein the genes are selected from Table 1.1, can predict the adaptive breast cancer subtype clusters using a centroid approach. For each gene set, 100 iterations were performed, and the accuracy results are presented in a line plot (Figure 9A). List of the 20 genes that are present in 4, 5, or 6 gene sets (Figure 9B). The top 20 gene sets are shown with gene symbols (Table 22). For the top 5 sets, confusion matrix along with other performance metrics (accuracy, specificity, sensitivity and F1) are shown (Table 23).

**Table 22: 20 randomly selected sets of 30 genes for prediction of AC clusters**

| | **Iteration** | **Accuracy** | **Genes** |
|---|---|---|---|
| Set 1 | 98 | 0, 906 | TXNIP, ANXA3, CITED2, PPID, CAV2, STK11, CXCR1, GPR160, NR4A3, CCNA2, GSN, STEAP4, ABCD1, PFKL, MAPK10, SFRP1, FGF14, SREBF1, TCF4, CCNB1, ANXA1, STAT1, SLC27A4, TPX2, PIK3R3, TYMS, PLAU, FGFBP1, TRIB1, PIK3R2 |
| Set 2 | 22 | 0, 900 | RELN, NPC1, TCF7L1, FAM105A, SPHK1, PCOLCE, TAP1, AQP1, BMI1, DNAJC22, EZH2, KIT, OCLN, MAZ, GADD45G, KRT17, FOS, HJURP, C3, BUB1, RASD1, STEAP4, RPS4X, NRIP1, IL18, MST1, POLR2D, TK1, E2F1, MEIS1 |
| Set 3 | 56 | 0, 889 | MAML2, TNXB, CDKN1A, MTDH, PLAU, OAS1, FAS, PKMYT1, NOL3, CYR61, PCNA, TIPARP, MYBL2, CCNE1, CACNG4, UBE2T, CCL14, HES1, CXCL10, COL1A2, CYP1A1, CDKN2C, NR4A3, GLUL, PDPK1, MADD, GATA3, SLIT2, CXCR1, HSPB6 |
| Set 4 | 61 | 0, 889 | STK11, EXO1, CDC14B, TRAF2, ID4, ANXA1, PLK4, NF2, SLC16A3, TCF7L1, SERTAD1, NOV, ZFP36L1, MAPK10, CXCL3, EGFR, TNFAIP3, NME1, GTSE1, MYBL1, CCNE1, MAZ, F3, CCL2, HIST1H3H, IGF2, RUNX1T1, CXCR1, MEIS1, AKT3 |
| Set 5 | 39 | 0, 883 | AKR1C3, PIAS4, GNGT2, TPX2, TYMS, PRC1, HSPA14, CDC6, SFRP1, IL4, ID4, TUBB3, SPC25, TOP2A, GAS1, ACKR1, TESC, TSPAN7, STAT1, PDZK1, RUNX1T1, FABP4, CXCL9, FLJ10474, CXCR4, CDKN1A, MYB, ANLN, TFF1, SERPINE1 |
| Set 6 | 94 | 0, 881 | HES1, FOS, FLRT2, TMEM132A, DLC1, IGF1, EXO1, COL1A2, GATA3, KDM4B, ANLN, IL33, NSD1, CENPF, TUBB3, SUSD3, EGFR, CXCL3, IRF7, MT1X, DUSP1, GGH, MADD, SFRP4, SMAD9, GAS1, NOLC1, RASSF7, MAPK10, TXNIP |
| Set 7 | 16 | 0, 880 | TNN, FLNC, HSPA14, NOV, PDZK1, MAFF, EGR3, MMP11, GPI, MAOB, AKR1C3, FN1, TUBB3, DNAJC14, IGF1, XBP1, ASPM, HIF3A, CCNA2, TMPRSS2, BMI1, NUP62, TPO, POLR2D, NCAPD3, POLD2, RPS4X, SGK1, GAS1, RUNX1T1 |
| Set 8 | 23 | 0, 880 | C3, NCAPD3, IGF2, CREB5, MADD, MMP11, PMAIP1, CYP1B1, LEPR, STAT1, CDC6, MT1X, PRDX6, STK11, SPHK1, TCF4, TPO, ANGPTL1, GADD45B, HMGCS2, EZH2, XBP1, HERC3, JUNB, RASGRF2, PLK4, PER1, DVL1, TESC, FAM105A |
| Set 9 | 30 | 0, 880 | ISG15, PRL, ABCD1, GZMB, PRC1, PLAU, MKI67, GSN, DLC1, SRC, FOXO1, NUSAP1, KRT17, HSPB6, TPX2, CXCL2, FAM105A, MT1X, NTHL1, CNTFR, EXO1, LPL, TIPARP, TSC22D3, PLK1, TESC, TUBB3, NSD1, DKK2, ADIPOR1 |
| Set 10 | 38 | 0, 880 | RASSF7, MT1X, TESC, DNAJC22, GADD45G, POLR2D, TGFB3, ZFP36L1, MTDH, TYMS, NR1H3, CCNA2, NUF2, ADAMTS1, |
| | | | DNAJC14, STMN1, DLGAP5, E2F1, NOLC1, CD209, PTN, PPAP2A, SLC19A3, CXCL2, PCNA, SPHK1, STK11, NRAS, CYR61, MAML2 |
| Set 11 | 40 | 0, 880 | SPRY2, ABCA3, CCNB1, LYVE1, PFKL, HERC3, RELN, CXCL10, GAPDH, CNTFR, SLC2A3, TK1, TEK, ADIPOR1, TGFB3, AKR1C3, AKT3, PITRM1, CD34, TNFAIP3, RIPK2, SPHK1, NSD1, PDPK1, GTSE1, POLD1, CXCR4, CTGF, CENPF, NR4A1 |
| Set 12 | 13 | 0, 878 | GNGT2, FOXA1, OGG1, COL4A3, CSF2, CAV1, PGR, E2F1, GADD45A, PABPC1, ANXA3, POLR2D, BTG2, TNXB, RUNX1T1, IGF1, CXCL3, RASSF7, SAP30, SOCS3, PRL, DLGAP5, GPSM2, FGFBP1, NOL3, FAM105A, OAS1, SMAD9, BUB1, TWIST2 |
| Set 13 | 71 | 0, 878 | SUSD3, FABP4, PRDX6, JMJD1C, GAPDH, EGFR, EGR3, RAB25, STAT1, PDZK1, CAV1, MT1A, GADD45B, SLC16A3, KDM4B, PABPC1, TYMS, CDKN1C, CCNB1, MYBL1, ALDH1A1, SFN, PITRM1, GNGT2, HES1, MADD, LIF, MAFF, TRAF2, GADD45A |
| Set 14 | 47 | 0, 876 | TRAF2, GZMB, NOL3, FN1, TPO, GATA3, SRC, LIF, ID4, ADIPOR1, PPAP2B, TMEM132A, SOCS3, CCT5, CITED2, GADD45B, TOP2A, NCAPD3, AQP1, LEPR, IL20, NUF2, FOXC1, NAT1, MYBL2, PRC1, PIM2, CD209, DNAJC14, WNT2 |
| Set 15 | 62 | 0,874 | CSF2, NME1, KRT5, TNC, CXCR1, ABCA3, FN1, MEIS1, KDM4B, LIG4, RGS2, COL1A2, NR4A3, TGFBR2, THBS4, NUF2, MYCN, NOV, MADD, RUNX1T1, ID4, GFRA1, TESC, EGFR, PITRM1, PLK1, CXCL9, CNTFR, EGR1, SLC27A4 |
| Set 16 | 7 | 0, 872 | MIF, POLD1, SPC25, PTN, FAS, TNC, EGR3, PFKL, CXCL2, FLJ10474, LYVE1, IRF7, MKI67, TK1, COL6A6, COL4A3, TNFAIP3, MAFF, CDKN1C, NSD1, EMP1, JUN, POLD2, CITED2, HSPB6, KDM4B, SFRP4, TPX2, PGR, PRL |
| Set 17 | 10 | 0, 872 | CAV2, GADD45B, OAS1, PLCG1, RRM2, TK1, HSPA14, NOLC1, GATA3, EGFR, FOXC1, NR1H3, DLC1, IL33, CEBPD, PRC1, HERC3, IRF7, CXCL2, BMI1, LTB, AQP1, TUBB3, NUSAP1, MAGEL2, SFRP1, FAT4, MAPK10, MAZ, DLGAP5 |
| Set 18 | 77 | 0, 872 | TBP, ADIPOQ, GSN, HERC3, GADD45A, PLCG1, FGF7, PPP2R4, IGF2, EGR1, CCNE1, SPC25, LIG4, MT1A, EGR3, TOP2A, EMP1, XBP1, IRF7, SFRP4, CACNG4, SPRY2, RASGRF2, TNFAIP3, MKI67, MAFF, ABCA3, MEIS1, HMGB3, IL33 |
| Set 19 | 84 | 0, 872 | CITED2, HMGB3, LTB, CDC14B, JMJD1C, OCLN, OASL, COL1A2, IGF2, NPC1, MAFF, ANLN, EGR1, OAS1, MT1A, COL4A3, STK11, ID4, HES1, MYBL2, FGFBP1, TFF1, AKT3, NUSAP1, FN1, PGR, IL4, XBP1, CCNB1, SELE |
| Set 20 | 91 | 0, 872 | PTGS2, MADD, THBS2, CXCL10, SLC16A3, IL20, IL33, TNXB, CXCR1, BMI1, CDC14B, CDC20, PRL, PLK4, SFRP4, RHOU, RPS4X, PIK3R2, KRT17, TNFAIP3, DUSP1, HIF3A, SPHK1, CXCL3, NR4A3, DVL1, JUNB, ASPM, CCL14, ANGPTL1 |

**Table 23: Top 5 sets of 30 genes for prediction of AC clusters**

| **Set 1** | | **AC-1** | **AC-2** | **AC-3** | **AC-4** | **AC-5** | | **Sensitivity** | **Specificity** | **F1** |
|---|---|---|---|---|---|---|---|---|---|---|
| | **AC-1** | 128 | 11 | 4 | 1 | 0 | **AC-1** | 0,934 | 0,960 | 0,911 |
| | **AC-2** | 9 | 129 | 0 | 0 | 1 | **AC-2** | 0,908 | 0,975 | 0,918 |
| | **AC-3** | 0 | 0 | 116 | 12 | 0 | **AC-3** | 0,885 | 0,971 | 0,896 |
| | **AC-4** | 0 | 2 | 10 | 104 | 0 | **AC-4** | 0,889 | 0,972 | 0,893 |
| | **AC-5** | 0 | 0 | 1 | 0 | 12 | **AC-5** | 0,923 | 0,998 | 0,923 |
| | | | | | | | | | | |

| **Set 2** | | **AC-1** | **AC-2** | **AC-3** | **AC-4** | **AC-5** | | **Sensitivity** | **Specificity** | **F1** |
|---|---|---|---|---|---|---|---|---|---|---|
| | **AC-1** | 127 | 13 | 2 | 0 | 0 | **AC-1** | 0,927 | 0,963 | 0,910 |
| | **AC-2** | 9 | 123 | 0 | 1 | 0 | **AC-2** | 0,866 | 0,975 | 0,895 |
| | **AC-3** | 0 | 1 | 116 | 9 | 0 | **AC-3** | 0,885 | 0,976 | 0,903 |
| | **AC-4** | 0 | 5 | 12 | 107 | 0 | **AC-4** | 0,915 | 0,960 | 0,888 |
| | **AC-5** | 1 | 0 | 1 | 0 | 13 | **AC-5** | 1,000 | 0,996 | 0,929 |
| | | | | | | | | | | |

| **Set 3** | | **AC-1** | **AC-2** | **AC-3** | **AC-4** | **AC-5** | | **Sensitivity** | **Specificity** | **F1** |
|---|---|---|---|---|---|---|---|---|---|---|
| | **AC-1** | 130 | 14 | 3 | 0 | 0 | **AC-1** | 0,949 | 0,958 | 0,915 |
| | **AC-2** | 7 | 122 | 1 | 2 | 0 | **AC-2** | 0,859 | 0,975 | 0,891 |
| | **AC-3** | 0 | 3 | 114 | 14 | 0 | **AC-3** | 0,870 | 0,958 | 0,870 |
| | **AC-4** | 0 | 3 | 13 | 101 | 0 | **AC-4** | 0,863 | 0,962 | 0,863 |
| | **AC-5** | 0 | 0 | 0 | 0 | 13 | **AC-5** | 1,000 | 1,000 | 1,000 |
| | | | | | | | | | | |

| **Set 4** | | **AC-1** | **AC-2** | **AC-3** | **AC-4** | **AC-5** | | **Sensitivity** | **Specificity** | **F1** |
|---|---|---|---|---|---|---|---|---|---|---|
| | **AC-1** | 128 | 8 | 7 | 0 | 0 | **AC-1** | 0,934 | 0,963 | 0,914 |
| | **AC-2** | 6 | 131 | 2 | 0 | 0 | **AC-2** | 0,923 | 0,980 | 0,932 |
| | **AC-3** | 3 | 2 | 110 | 18 | 1 | **AC-3** | 0,840 | 0,941 | 0,830 |
| | **AC-4** | 0 | 1 | 11 | 99 | 0 | **AC-4** | 0,846 | 0,972 | 0,868 |
| | **AC-5** | 0 | 0 | 1 | 0 | 12 | **AC-5** | 0,923 | 0,998 | 0,923 |
| | | | | | | | | | | |

| **Set 5** | | **AC-1** | **AC-2** | **AC-3** | **AC-4** | **AC-5** | | **Sensitivity** | **Specificity** | **F1** |
|---|---|---|---|---|---|---|---|---|---|---|
| | **AC-1** | 122 | 18 | 3 | 0 | 0 | **AC-1** | 0,891 | 0,948 | 0,871 |
| | **AC-2** | 10 | 121 | 0 | 1 | 0 | **AC-2** | 0,852 | 0,972 | 0,883 |
| | **AC-3** | 4 | 0 | 121 | 16 | 0 | **AC-3** | 0,924 | 0,951 | 0,890 |
| | **AC-4** | 1 | 3 | 7 | 100 | 0 | **AC-4** | 0,855 | 0,974 | 0,877 |
| | **AC-5** | 0 | 0 | 0 | 0 | 13 | **AC-5** | 1,000 | 1,000 | 1,000 |

### F) Predictive value of the Adaptive Clusters

As explained in Example 1, the adaptive breast cancer subtypes AC-1 to AC-5 correlated strongly with the AIMS subtypes, which are known to be prognostic, wherein AC-1 and AC-3 are enriched for tumors with an AIMS subtype change from LumB to LumA during NACT, with improved iDFS for AC-1, AC-2 and AC-4 are enriched for tumors with a constant LumA AIMS subtype before and after NACT, with improved iDFS for AC-2, and AC-5 is enriched for tumors with a pre-NACT Basal/HER2E AIMS subtype with bad prognosis (see Table 9 in Example 1). Classification of patients by the adaptive subtypes (AC-1 to AC-5) prognosticated patient outcome (iDFS) better than pre-NACT AIMS (see Figure 2 and Example 1). Taken together, the experimental data of this disclosure shows that the response or resistance to and/or benefit from a systemic treatment in a subject suffering from a hormone receptor-positive (HR+) and human epidermal growth factor receptor 2-negative (HER2-) breast cancer can be predicted by clustering a subject into an adaptive breast cancer subtype, because said adaptive subtypes are superior to classical AIMS subtypes for prediction of survival after NACT in luminal BC.

## Claims

1. A method for predicting a response or resistance to and/or benefit from a systemic treatment in a subject suffering from a hormone receptor-positive (HR+) and human epidermal growth factor receptor 2-negative (HER2-) breast cancer, optionally in an early-stage, comprising the steps of:
(a) determining in a sample obtained from said subject the expression levels of three or more markers selected from Table 1.1;
(b) optionally normalizing the determined expression levels of said three or more markers;
(c) mathematically combining the determined expression levels of the three or more markers selected from Table 1.1 to generate a prediction, preferably a predictive score; and
(d) predicting the response or resistance to and/or benefit from said systemic treatment in said subject based on the prediction, preferably based on the predictive score;
wherein the expression levels of the three or more markers are indicative for predicting the response or resistance to and/or benefit from the systemic treatment in said subject.

2. The method of claim 1, wherein the systemic treatment is neoadjuvant or adjuvant treatment, preferably wherein the systemic treatment is neoadjuvant treatment, preferably wherein the systemic treatment is chemotherapy, most preferably wherein the systemic treatment is neoadjuvant chemotherapy, optionally comprising a taxane-containing agent for at least six weeks.

3. The method of claim 1 or 2, wherein the method further comprises the determination of one or more clinical parameters selected from the group consisting of pathological grading of the tumor, clinical tumor size (cT), clinical nodal status (cN), proliferation (in particular Ki-67), immune system markers (in particular lymphocytes, preferably tumor infiltrating lymphocytes (TILs)), estrogen receptor status, progesterone receptor status, and variables derived from them (clinical and pathological stage, CPS-EG).

4. The method of any one of claims 1 to 3, wherein the mathematical combination of the determined expression levels of said three or more markers selected from Table 1.1 to generate a prediction, optionally a mathematical combination of the determined expression levels of said three or more markers and one or more additional markers, one or more clinical parameters and/or one or more non-clinical parameters, is performed using a statistical and/or mathematical model.

5. The method of any one of claims 1 to 4, wherein the response or resistance to the systemic treatment is measured by the long-term development of the disease, in particular development of invasive relapses, distant metastases, disease-related survival, and/or overall survival.

6. The method of any one of claims 1 to 5, wherein the expression levels of three or more markers selected from any one of Tables 3-8 are determined, preferably wherein the expression levels of three or more markers selected from Table 4 are determined, preferably Table 5, more preferably Table 6 or Table 8, most preferably Table 7.1.

7. The method of claim 6, wherein the expression levels of three or more markers selected from Table 6 or 7.1 are determined and the high expression levels of three or more markers selected from Table 6 or 7.1 in a tumor sample are indicative for a good response of the subject to the neoadjuvant chemotherapy and/or the patient is predicted to benefit from the neoadjuvant treatment, optionally wherein the three or more markers are DNA repair genes or wherein the expression levels of three or more markers selected from Table 8 are determined and high expression levels of a plurality of markers selected from Table 8 in a tumor sample are indicative for a bad response of the subject to the treatment and/or the patient is predicted to not benefit from the treatment.

8. The method of any one of claims 1 to 7, wherein said subject has a low chance to reach a pathological complete remission (pCR) after neoadjuvant treatment.

9. The method of any one of claims 1 to 8, wherein at least five markers are selected from Table 1.1, preferably at least ten markers from Table 1.1, more preferably at least twenty markers from Table 1.1, more preferably at least fifty markers from Table 1.1, most preferably all markers from Table 1.1 are selected.

10. The method of any one of claims 1 to 9, wherein the tumor sample is tumor tissue sample, preferably wherein the sample is a primary tumor tissue sample, more preferably a core biopsy sample, more preferably a core biopsy sample from a primary tumor before any systemic treatment, most preferably a core biopsy sample from a primary tumor before neoadjuvant treatment.

11. The method of any one of claims 1 to 10, wherein the expression levels are determined at gene level, preferably mRNA level, in a hybridization-based method, a PCR based method, a microarray-based method, a quantitative transcriptomic analysis, a sequencing and/or next generation sequencing (NGS) method.

12. Neoadjuvant chemotherapy for use in the treatment of a HR+ and HER2- breast cancer, wherein the neoadjuvant chemotherapy is to be administered to a subject who has been predicted to respond to and/or benefit from said treatment or for whom said treatment has been determined to have a positive outcome according to the method of any one of claims 1 to 11, optionally wherein the neoadjuvant chemotherapy comprises a taxane-containing agent and the taxane-containing agent is any one of paclitaxel, docetaxel, abraxane, cabazitaxel, and albumin-bound paclitaxel (nab-paclitaxel).

13. A method for therapy guidance, wherein a decision about systemic treatment, preferably neoadjuvant chemotherapy, is based on a prediction of response or resistance to and/or benefit from said systemic treatment, preferably neoadjuvant chemotherapy, wherein the prediction is made according to the method of any one of claims 1 to 11,
a) wherein if the subject is predicted to respond to and/or benefit from said systemic treatment, preferably neoadjuvant chemotherapy, the patient is treated with said systemic treatment; or
b) wherein if the subject is predicted not to respond to, to be resistant to and/or not to benefit from said systemic treatment, preferably neoadjuvant chemotherapy, the subject is treated with a different systemic treatment or with said systemic treatment and an additional systemic treatment.

14. A method for stratifying the risk of subjects suffering from HER+ and HER2- breast cancer, wherein the method comprises predicting the response or resistance to and/or benefit from systemic treatment, preferably neoadjuvant chemotherapy, according to the method of any one of claims 1 to 11.

15. Use of a prognostic assay for predicting the response or resistance to and/or benefit from a systemic treatment, preferably neoadjuvant chemotherapy, in a subject suffering from a HR+ and HER2- breast cancer, wherein the prognostic assay comprises a RNA-based assay, optionally wherein the prognostic assay is one or more of NGS-based RNA analysis, PCR-based RNA analysis, sequencing-based RNA analysis, quantitative transcriptome analysis, quantitative RNA expression analysis, RNA-hybridization-based technologies including spatial RNA analysis or microarrays including Affymetrix.
